# EUROPÄISCHE PATENTSCHRIFT

(11) **EP 3 325 472 B1**
(45) Veröffentlichungstag und Bekanntmachung des Hinweises auf die Patenterteilung: **17.04.2019**
(21) Anmeldenummer: 16739483.2
(22) Anmeldetag: 18.07.2016
(51) Int. Cl.: C07D 405/12, A01N 43/12, C07D 409/12, A01P 21/00

(54) **SUBSTITUIERTE FURANO-/THIENOCYCLOALKYLAMINO-2-PYRIMIDINDERIVATE UND DEREN VERWENDUNG ZUR BEKÄMPFUNG UNERWÜNSCHTEN PFLANZENWACHSTUMS**
SUBSTITUTED FURANO/THIENOCYCLOALKYLAMINO -2- PYRIMIDINE DERIVATIVES AND USE OF SAME FOR COMBATING UNDESIRED PLANT GROWTH
DERIVES DE 2-PYRIMIDINE /THIENO-CYCLO-ALKYL-AMINO/FURANO ET LEUR UTILISATION POUR LUTTER CONTRE LA CROISSANCE DE PLANTES INDESIRABLES

(30) Priorität: 24.07.2015 EP 15178186
(43) Veröffentlichungstag der Anmeldung: 30.05.2018
(73) Patentinhaber: Bayer CropScience AG, 40789 Monheim am Rhein (DE)
(72) Erfinder: JAKOBI, Harald, 60598 Frankfurt (DE); MINN, Klemens, 65795 Hattersheim (DE); BUSCATO ARSEQUELL, Estella, 60486 Frankfurt am Main (DE); DIETRICH, Hansjörg, 65835 Liederbach (DE); GATZWEILER, Elmar, 61231 Bad Nauheim (DE); ROSINGER, Christopher Hugh, 65719 Hofheim (DE); SCHMUTZLER, Dirk, 65795 Hattersheim (DE)
(74) Vertreter: BIP Patents
(86) Internationale Anmeldenummer: PCT/EP2016/067035
(87) Internationale Veröffentlichungsnummer: WO 2017/016914

(56) Entgegenhaltungen:
- EP-A1- 2 210 879
- WO-A1-2013/144187
- MASATOSHI TAMARU ET AL: "Studies of the New Herbicide KIH-6127. Part II. Synthesis and Herbicidal Activity of 6-Acyl Pyrimidin-2-yl Salicylates and Analogues against Barnyard Grass", PESTICIDE SCIENCE, Bd. 47, Nr. 4, 1. August 1996 (1996-08-01) , Seiten 327-335, XP055214456, ISSN: 0031-613X, DOI: 10.1002/(SICI)1096-9063(199608)47:4<327::A ID-PS432>3.0.CO;2-U

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen sowie im Ziergartenbereich und zur generellen Bekämpfung von Unkräutern und Ungräsern in Umweltbereichen, in denen Pflanzenwuchs störend ist.

Insbesondere betrifft die Erfindung substituierte Furano-/Thienocycloalkylamino-2-Pyrimidinderivate, Verfahren zu deren Herstellung sowie deren Verwendung zur Bekämpfung von Schadpflanzen.

Die erfindungsgemäßen Verbindungen der Formel (I) weisen in der 2-Positon des Pyrimidins einen in der alpha Position zum Aromaten über ein Amin gebundenen teilhydrierten bicyclischen Substituenten auf, wobei das Pyrimidin in der 4-Position, 5-Position und 6-Position ebenfalls substituiert sein kann und benachbarte Substituenten einen Ring bilden können.

Aus dem Stand der Technik ist die herbizide Wirkung von Diaminopyrimidinen und auch von Monoaminopyrimidinen bereits bekannt.

Monaminopyrimidinderivate mit herbizider Wirkung, nämlich 5-Amino-Pyrimidinderivate, sind beispielsweise aus WO 2013/144187 A1 bekannt, während 2,4-Diaminopyrimidine und deren Anwendung im Bereich des Pflanzenschutzes beispielsweise durch EP 0523533 A1, WO 2010/076009 und WO 2010/076010 vorbeschrieben sind.

2,4-Diaminopyrimidine mit bicyclischem Rest, welche am verbrückten und am benachbarten Kohlenstoffatom eine (1R, 2S)-Konfiguration aufweisen und sich zugleich durch eine herbizide Wirksamkeit auszeichnen, sind aus US 2010/0167934 A1 bekannt.

Andererseits sind herbizid wirksame substituierte Thienocycloalk(en)ylamino-1,3,5-triazine bekannt und beispielsweise in den Druckschriften WO 2003/070710 A1, JP 2002020383 (auch Furane) und DE 19921883 beschreiben.

Die Anwendung der bekannten Pyrimidin- und Triazinderivate als selektive Herbizide zur Schadpflanzenbekämpfung oder als Pflanzenwachstumsregulatoren in verschiedenen Nutzpflanzenkulturen erfordert jedoch häufig eine hohe Kosten verursachende Aufwandmenge oder führt zu unerwünschten Schädigungen der Nutzpflanzen. Zudem ist die Anwendung der Wirkstoffe in vielen Fällen wegen verhältnismäßig hoher Herstellkosten unwirtschaftlich.

Es ist deshalb erstrebenswert, alternative chemische Wirkstoffe auf Basis von Furano-/Thienocycloalkylamino-2-Pyrimidinderivate bereitzustellen, die als Herbizide oder Pflanzenwachstumsregulatoren eingesetzt werden können und mit welchen bestimmte Vorteile im Vergleich zu aus dem Stand der Technik bekannten Systemen verbunden sind.

Die Aufgabe der vorliegenden Erfindung besteht daher in der Bereitstellung alternativer Furano-/Thienocycloalkylamino-2-Pyrimidin-Derivate, welche als Herbizide oder Pflanzenwachstumsregulatoren, mit einer zufriedenstellenden herbiziden Wirkung und einem breiten Wirkspektrum gegenüber Schadpflanzen und/oder mit einer hohen Selektivität in Nutzpflanzenkulturen, eingesetzt werden können.

Außerdem sollen die alternativen Furano/Thienocycloalkylamino-2-Pyrimidin-Derivate im Vergleich zu den aus dem Stand der Technik bekannten Pyrimidinderivaten ein besseres Eigenschaftsprofil, insbesondere eine bessere herbizide Wirkung gegen Schadpflanzen, ein breiteres Spektrum gegenüber Schadpflanzen und/oder eine höhere Selektivität in Nutzpflanzenkulturen zeigen.

Gelöst wird die Aufgabe durch speziell substituierte Furano/Thienocycloalkylamino-2-Pyrimidin-Derivate der Formel (I) oder einem agrochemisch verträglichen Salz davon, welche vorteilhaft als Herbizide und auch als Pflanzenwachstumsregulatoren eingesetzt werden können.

Gegenstand der vorliegenden Erfindung sind daher Verbindungen der allgemeinen Formel (I) 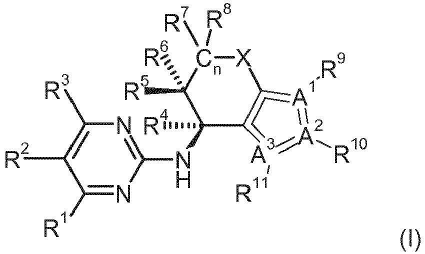 und deren agrochemisch verträglichen Salze, wobei
- A¹, A² und A³: jeweils unabhängig voneinander ausgewählt sind aus O, S, CR⁹, CR¹⁰ oder CR¹¹, wobei genau ein Atom aus A¹, A² und A³ O oder S ist;
- R⁹, R¹⁰ und R¹¹: jeweils unabhängig voneinander ausgewählt sind aus der Gruppe, bestehend aus Wasserstoff, Halogen, Cyano, C(O)OH, C(O)NH₂, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkenyl, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl und Nitro;
- R¹ und R²: jeweils unabhängig voneinander, ausgewählt sind aus der Gruppe, bestehend aus
- -: Halogen, Hydroxy, Nitro, Amino, Cyano, C(O)NH₂;
- -: (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl; - (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- -: (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
- -: (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl;
- -: Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- -: (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können;
- -: (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
- -: Aminocarbonyl-(C₁-C₆)-alkyl, Di-(C₁-C₆)-Alkylaminocarbonyl-(C₁-C₆)-alkyl;
- -: N-((C₁-C₆)-Haloalkanoyl)-amino-carbonyl, Mono-((C₆-C₁₄)-aryl)-amino-carbonyl, Di-((C₆-C₁₄)-aryl)-amino-carbonyl;
- -: (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy;
- -: (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycar-bonyloxy;
- -: (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cyclo-alkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbo-nyloxy, (C₃-C₈)-Cycloalkenyl-(C₁-C₆)-haloalkoxycarbonyloxy;
- -: Hydroxy-(C₁-C₆)-alkyl, Hydroxy-(C₁-C₆)-alkoxy Cyano-(C₁-C₆)-alkoxy, Cyano-(C₁-C₆)-alkyl; und
- -: (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Al-kylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio und (C₃-C₆)-Alkinylthio;
- R³: ausgewählt ist aus der Gruppe, bestehend aus
- -: Wasserstoff, Halogen, Hydroxy, Nitro, Amino, Cyano, C(O)OH, C(O)NH₂;
- -: (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
- -: (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- -: (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
- -: (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl;
- -: Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- -: (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können;
- -: (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
- -: Aminocarbonyl-(C₁-C₆)-alkyl, Di-(C₁-C₆)-Alkylaminocarbonyl-(C₁-C₆)-alkyl;
- -: N-((C₁-C₆)-Haloalkanoyl)-amino-carbonyl, Mono-((C₆-C₁₄)-aryl)-amino-carbonyl, Di-((C₆-C₁₄)-aryl)-amino-carbonyl;
- -: (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy;
- -: (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycar-bonyloxy;
- -: (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cyclo-alkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbo-nyloxy, (C₃-C₈)-Cycloalkenyl-(C₁-C₆)-haloalkoxycarbonyloxy;
- -: Hydroxy-(C₁-C₆)-alkyl, Hydroxy-(C₁-C₆)-alkoxy Cyano-(C₁-C₆)-alkoxy, Cyano-(C₁-C₆)-alkyl; und
- -: (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Al-kylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio und (C₃-C₆)-Alkinylthio, oder
- R¹ mit R²: über eine Bindung miteinander verbunden sein kann, so dass sich ein 5-bis 7-gliedriger teilhydrierter Carbocyclus oder Heterocyclus mit mindestens einem Heteroatom ausgewählt aus N, O, S und P ergibt, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl und (C₁-C₆)-Haloalkyl substituiert ist, und
- R³: wie oben definiert ist, bevorzugt jedoch Wasserstoff oder Amino ist;
- R⁴: ausgewählt ist aus der Gruppe bestehend aus Wasserstoff, (C₁-C₆)-Alkyl, (C₁-C₆)-Alkoxy, (C₁-C₆)-Alkoxy-(C₁-C₆)-Alkyl, (C₁-C₆)-Alkoxycarbonyl und Aminocarbonyl;
- R⁵ und R⁶: jeweils unabhängig voneinander ausgewählt sind aus der Gruppe, bestehend aus Wasserstoff, Hydroxy, (C₁-C₆)-Alkyl, (C₁-C₆)-Alkylphenyl, (C₁-C₆)-Haloalkyl, (C₁-C₆)-Alkoxy und (C₁-C₆)-Halogenalkoxy; oder die Reste R⁵ und R⁶ zusammen mit dem Kohlenstoffatom, an welchem sie gebunden sind, einen 3- bis 7-gliedrigen Ring bilden;
- R⁷ und R⁸: jeweils unabhängig voneinander ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl; oder die Reste R⁷ und R⁸ zusammen eine (C₁-C₇)-Alkylengruppe bilden, die ein oder mehrere Sauerstoff- und/oder Schwefelatome enthalten können, wobei die (C₁-C₇)-Alkylengruppe durch Halogen einfach oder mehrfach substituiert sein kann und die jeweilgen Halogensubstituenten gleich oder verschieden sein können;
- X: für eine Bindung (falls n=1 oder 2 ist) steht, oder ausgewählt ist aus der Gruppe bestehend aus O, S, CH₂, C=O, NH, CR¹²R¹³, und NR¹⁴, CH₂O und CH₂S, wobei bei den beiden letztgenannten Gruppen das Kohlenstoffatom an den aromatischen Teil und das Heteroatom O oder S an den teilhydrierten Teil des Amins gebunden ist;
- R¹² und R¹³: jeweils unabhängig voneinander, ausgewählt sind aus der Gruppe, bestehend aus Wasserstoff, (C₁-C₆)-Alkyl und (C₁-C₆)-Haloalkyl;
- R¹⁴: ausgewählt ist aus der Gruppe bestehend aus Wasserstoff, (C₁-C₆)-Alkyl und (C₁-C₆)-Haloalkyl; und
- n: die Laufzahl 0, 1 oder 2 ist.

Die erfindungsgemäßen Furano-/Thienocycloalkylamino-2-Pyrimidinderivate der Formel (I) unterscheiden sich von den aus den Dokumenten EP 0523533 A1, WO 2010/076009 und WO 2010/076010 bekannten Herbiziden mit 2,4-Diaminopyrimidin-Struktur durch die speziellen Furano-/Thienocycloalkylamino Substituenten in der 2-Position des Pyrimidins.

Die Verbindungen der Formel (I) zeichnen sich, neben einem guten Wirksamkeitsprofil und einer guten Kulturpflanzenverträglichkeit, durch ihre kostengünstige Herstellung aus, da die erfindungsgemäßen Substanzen aus preiswerten und gut zugänglichen Vorstufen durch kostengünstige Prozesse hergestellt werden können. Daher kann auf die Verwendung teurer und schwer zugänglicher Zwischenprodukte verzichtet werden.

Verbindungen der Formel (I) zeichnen sich besonders durch eine gute Kulturpflanzenverträglichkeit in Soja aus.

Im Folgenden werden, jeweils für die einzelnen Substituenten, bevorzugte, besonders bevorzugte und ganz besonders bevorzugte Bedeutungen beschrieben. Die übrigen Substituenten der allgemeinen Formel (I), welche nachfolgend nicht genannt werden, weisen die oben genannte Bedeutung auf. Das gilt auch für die Laufzahl n, d.h. die Laufzahl n ist in den nachfolgenden Ausführungsformen 0, 1 oder 2.

Eine erste Ausführungsform der vorliegenden Erfindung umfasst Verbindungen der allgemeinen Formel (I), in welchen
- A¹, A² und A³: bevorzugt jeweils unabhängig voneinander ausgewählt sind aus S, CR⁹, CR¹⁰ oder CR¹¹, wobei genau ein Atom aus A¹, A² und A³ S ist.

In einer zweiten Ausführungsform der vorliegenden Erfindung umfasst Verbindungen der allgemeinen Formel (I), in welchen
R⁹, R¹⁰ und R¹¹ bevorzugt jeweils unabhängig voneinander ausgewählt sind aus der Gruppe, bestehend aus Wasserstoff Halogen und (C₁-C₆)-Alkyl, und
R⁹, R¹⁰ und R¹¹ besonders bevorzugt jeweils unabhängig voneinander ausgewählt sind aus der Gruppe bestehend aus Wasserstoff, Chlor und (C₁-C₃)-Alkyl; und in welchem
R⁹, R¹⁰ und R¹¹ noch mehr bevorzugt jeweils unabhängig voneinander ausgewählt sind aus der Gruppe bestehend aus Wasserstoff und Methyl, und in welchem
am meisten bevorzugt genau ein Rest R⁹, R¹⁰ oder R¹¹ Methyl ist und die anderen beiden Reste Wasserstoff sind.

In einer dritten Ausführungsform der vorliegenden Erfindung umfasst Verbindungen der allgemeinen Formel (I), in welchem
- A¹, A² und A³: bevorzugt jeweils unabhängig voneinander, ausgewählt sind aus O, S, CR⁹, CR¹⁰ oder CR¹¹, wobei genau ein Atom aus A¹, A² und A³ O oder S ist, und
- R⁹, R¹⁰ und R¹¹: jeweils unabhängig voneinander, ausgewählt sind aus der Gruppe, bestehend aus Wasserstoff, Chlor und (C₁-C₃)-Alkyl; und
- A¹, A² und A³: besonders bevorzugt jeweils unabhängig voneinander, ausgewählt sind aus S, CR⁹, CR¹⁰ oder CR¹¹, wobei genau ein Atom aus A¹, A² und A³ S ist, und
- R⁹, R¹⁰, und R¹¹: jeweils unabhängig voneinander ausgewählt sind aus der Gruppe bestehend aus Wasserstoff und (C₁-C₃)-Alkyl; und
- A¹, A² und A³: ganz besonders bevorzugt jeweils unabhängig voneinander, ausgewählt sind aus S, CR⁹, CR¹⁰ oder CR¹¹, wobei genau ein Atom aus A¹, A² und A³ S ist, und
- R⁹, R¹⁰, und R¹¹: jeweils unabhängig voneinander ausgewählt sind aus Wasserstoff und Methyl, und am meisten bevorzugt genau ein Rest R⁹, R¹⁰ oder R¹¹ Methyl ist und die anderen beiden Reste Wasserstoff sind.

Eine vierte Ausführungsform der vorliegenden Erfindungen umfasst Verbindungen der allgemeinen Formel (I), in welchen
- R¹ und R²: bevorzugt jeweils unabhängig voneinander, ausgewählt sind aus der Gruppe, bestehend aus Halogen, Hydroxy, Nitro, Amino, Cyano, (C₁-C₆)-Alkyl, (C₁-C₆)-Halogenalkyl, (C₁-C₆)-Alkylcarbonyl, (C₁-C₆)-Alkylsulfonyl, (C₁-C₆)-Alkylthio, (C₁-C₆)-Alkylsulfinyl und (C₁-C₆)-Haloalkylsulfonyl; und
- R³: bevorzugt jeweils unabhängig voneinander, ausgewählt ist aus der Gruppe, bestehend aus Wasserstoff, Halogen, Hydroxy, Nitro, Amino, Cyano, (C₁-C₆)-Alkyl, (C₁-C₆)-Halogenalkyl, (C₁-C₆)-Alkylcarbonyl, (C₁-C₆)-Alkylsulfonyl, (C₁-C₆)-Alkylthio, (C₁-C₆)-Alkylsulfinyl und (C₁-C₆)-Haloalkylsulfonyl; und
- R¹ und R²: mehr bevorzugt jeweils unabhängig voneinander, ausgewählt sind aus der Gruppe bestehend aus Amino, Trifluoromethyl, (C₁-C₃)-Alkyl, (C₁-C₆)-Alkylsulfonyl; und in welchem
- R³: mehr bevorzugt jeweils unabhängig voneinander, ausgewählt ist aus der Gruppe bestehend aus Wasserstoff, Amino, Trifluoromethyl, (C₁-C₃)-Alkyl, (C₁-C₆)-Alkylsulfonyl; und in welchem
- R¹ und R²: besonders bevorzugt jeweils unabhängig voneinander ausgewählt sind aus der Gruppe bestehend aus Amino, Trifluoromethyl, Methyl und Methylsulfonyl, und in welchem
- R³: besonders bevorzugt jeweils unabhängig voneinander ausgewählt sind aus der Gruppe bestehend aus Wasserstoff, Amino, Trifluoromethyl, Methyl und Methylsulfonyl, und in welchem
- R¹, R² und R³: am meisten bevorzugt jeweils verschieden voneinander sind und R¹ = Amino, R² = Trifluoromethyl oder Methylsulfonyl, und R³ = Wasserstoff oder Methyl; oder
- R¹ mit R²: über eine Bindung miteinander verbunden sind, so dass sich bevorzugt ein 5- oder 6-gliedriger teilhydrierter Carbocyclus oder Heterocyclus mit mindestens einem Heteroatom ausgewählt aus N, O, S und P bildet, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl, und C₁-C₆)-Haloalkyl substituiert ist, und
- R³: ausgewählt ist aus der Gruppe bestehend aus Wasserstoff, Amino, Methyl und Trifluoromethyl; oder
- R¹ mit R²: über eine Bindung miteinander verbunden sind, so dass sich besonders bevorzugt ein Cyclohexylring bildet, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl, und C₁-C₆)-Haloalkyl substituiert ist, und ganz bevorzugt mit einer Oxogruppe substituiert ist, und
- R³: Wasserstoff, Amino oder Methyl; oder
- R¹ mit R²: über eine Bindung miteinander verbunden sind, so dass sich besonders bevorzugt ein 6-gliedriger teilhydrierter Heterocyclus mit einem Schwefelatom bildet, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl, und C₁-C₆)-Haloalkyl substituiert ist, und ganz bevorzugt das Schwefelatom mit zwei Oxogruppen substituiert ist, und
- R³: Wasserstoff, Amino oder Methyl ist.

Eine fünfte Ausführungsform der vorliegenden Erfindungen umfasst Verbindungen der allgemeinen Formel (I), in welchen
- R⁴: besonders bevorzugt ausgewählt ist aus der Gruppe bestehend aus Wasserstoff, CH₃, CH₂CH₂OCH₃, COOCH₃ und CONH₂; und
- R⁴: ganz besonders bevorzugt ausgewählt ist aus der Gruppe bestehend aus Wasserstoff, CH₃, COOCH₃; und in welchen
- R⁴: am meisten bevorzugt Wasserstoff ist.

Eine sechste Ausführungsform der vorliegenden Erfindungen umfasst Verbindungen der allgemeinen Formel (I), in welchen
- R⁵ und R⁶: jeweils unabhängig voneinander bevorzugt ausgewählt sind aus der Gruppe bestehend aus Wasserstoff, Hydroxy, (C₁-C₆)-Alkyl, (C₁-C₆)-Alkylphenyl und (C₁-C₆)-Alkoxy; und
- R⁵ und R⁶: jeweils unabhängig voneinander besonders bevorzugt ausgewählt sind aus der Gruppe bestehend aus Wasserstoff, (C₁-C₆)-Alkyl und (C₁-C₆)-Alkoxy;
- R⁵ und R⁶: jeweils unabhängig voneinander ganz besonders bevorzugt ausgewählt sind aus der Gruppe bestehend aus Wasserstoff, (C₁-C₃)-Alkyl, und (C₁-C₆)-Alkoxy; und in welchen
- R⁵ und R⁶: jeweils unabhängig voneinander am meisten bevorzugt Wasserstoff oder Methyl sind.

In der siebte Ausführungsform ist speziell bevorzugt, wenn mindestens einer der Reste R⁵ bzw. R⁶ Wasserstoff ist. D.h. wenn jeweils mindestens einer der Reste R⁵ bzw. R⁶ Wasserstoff ist und der andere Rest R⁵ bzw. R⁶ ungleich Wasserstoff, insbesondere (C₁-C₆)-Alkyl, bevorzugt CH₃ ist.

Eine achte Ausführungsform der vorliegenden Erfindungen umfasst Verbindungen der allgemeinen Formel (I), in welchem
- R⁷ und R⁸: jeweils unabhängig voneinander bevorzugt ausgewählt sind aus der Gruppe bestehend aus Wasserstoff, (C₁-C₆)-Alkyl und (C₆-C₁₄)-Aryl; und
- R⁷ und R⁸: jeweils unabhängig voneinander besonders bevorzugt ausgewählt sind aus der Gruppe bestehend aus Wasserstoff, Methyl und Phenyl; und in welchen
- R⁷ und R⁸: ganz besonders bevorzugt jeweils Wasserstoff oder Methyl sind.

Eine neunte Ausführungsform der vorliegenden Erfindungen umfasst Verbindungen der allgemeinen Formel (I), in welchen
- X: besonders bevorzugt für eine Bindung (falls n=1 oder 2 ist) steht oder ausgewählt ist aus der Gruppe bestehend aus O, S, CH₂, C=O, NH, CHCH₃, NCH₃, C(CH₃)₂, OCH₂ und SCH₂, wobei bei den beiden letztgenannten das Kohlenstoffatom an den aromatischen Teil und das Heteroatom O oder S an den teilhydrierten Teil des Amins gebunden ist; und
- X: ganz besonders bevorzugt für eine Bindung (falls n=1 oder 2 ist) steht, oder CH₂ oder O ist.

Eine zehnte Ausführungsform der vorliegenden Erfindungen umfasst Verbindungen der allgemeinen Formel (I), in welchen die Laufzahl n bevorzugt 1 oder 2 beträgt. In einer ganz besonders bevorzugten Ausführungsform (1) beträgt die Laufzahl n = 2 und X steht für eine Bindung oder (2) beträgt die Laufzahl n = 1 und X steht für CH₂, (3) oder beträgt die Laufzahl n = 1 und X steht für eine Bindung, so dass sich in allen drei Fällen (1) bis (3) jeweils ein 5- oder 6-gliedriger Ring ausbildet.

Im Rahmen der vorliegenden Erfindung ist es möglich, die einzelnen bevorzugten, besonders bevorzugten und ganz besonders bevorzugten Bedeutungen für die Substituenten R¹ bis R¹⁴, A und X beliebig miteinander zu kombinieren, wobei die Laufzahl n 0, 1 oder 2 ist, bevorzugt 1 oder 2.

Das heißt, dass Verbindungen der allgemeinen Formel (I) von der vorliegenden Erfindung umfasst sind, in welchen beispielsweise der Substituent R¹ eine bevorzugte Bedeutung aufweist und die Substituenten R² bis R¹⁴ die allgemeine Bedeutung aufweisen oder aber der Substituent R² eine bevorzugte Bedeutung aufweist, der Substituent R³ eine besonders bevorzugte, bzw. eine ganz besonders bevorzugte, Bedeutung aufweist und die übrigen Substituenten eine allgemeine Bedeutung aufweisen.

Drei dieser Kombinationen der oben für die Substituenten R¹ bis R¹⁴, A und X gegebenen Definitionen werden nachfolgend beispielhaft erläutert und jeweils als weitere Ausführungsformen offenbart:
- Kombination der oben für die Substituenten R¹ bis R¹¹, A und X jeweils als besonders bevorzugt bezeichneten Definitionen (elfte Ausführungsform),
- Kombination der oben für die Substituenten R¹ bis R¹¹, A und X jeweils als ganz besonders bevorzugt bezeichneten Definitionen (zwölfte Ausführungsform), und
- Kombination der oben für den Substituenten R¹ bis R11, A und X als ganz besonders bevorzugt bezeichneten Definition (dreizehnte Ausführungsform)

Die vorgenannten auf den Kombinationen der Substituenten basierenden weiteren Ausführungsformen werden nachfolgend aus Gründen der Klarheit explizit offenbart:
Eine elfte Ausführungsform der vorliegenden Erfindung umfasst Verbindungen der allgemeinen Formel (I), in welchen
- R¹, R² und R³: jeweils unabhängig voneinander ausgewählt sind aus der Gruppe, bestehend aus Wasserstoff, Amino, Trifluoromethyl, (C₁-C₆)-Alkyl, (C₁-C₆)-Alkylsulfonyl; oder
- R¹ mit R²: über eine Bindung miteinander verbunden sind, so dass sich ein 5- oder 6-gliedriger teilhydrierter Carbocyclus oder Heterocyclus mit mindestens einem Heteroatom ausgewählt aus N, O, S und P bildet, der optional mit einem oder mehreren Substituten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl und (C₁-C₆)-Haloalkyl substituiert ist, und
- R³: ausgewählt ist aus Wasserstoff, Methyl, Methylsulfonyl, Amino, und Trifluoromethyl;
- R⁴: ausgewählt ist aus der Gruppe bestehend aus Wasserstoff, CH₃, CH₂CH₂OCH₃, COOCH₃ und CONH₂;
- R⁵ und R⁶: jeweils unabhängig voneinander ausgewählt sind aus der Gruppe bestehend aus Wasserstoff, (C₁-C₃)-Alkyl und (C₁-C₆)-Alkoxy;
- R⁷ und R⁸: jeweils unabhängig voneinander ausgewählt sind aus der Gruppe, bestehend aus Wasserstoff, (C₁-C₆)-Alkyl und (C₆-C₁₄)-Aryl;
- A¹, A² und A³: jeweils unabhängig voneinander ausgewählt sind aus S, CR⁹, CR¹⁰, CR¹¹, wobei genau ein Atom aus A¹, A² und A³ S ist;
- R⁹, R¹⁰ und R¹¹: jeweils unabhängig voneinander ausgewählt sind aus der Gruppe bestehend aus Wasserstoff, Halogen und (C₁-C₆)-Alkyl;
- X: für eine Bindung (falls n=1 oder 2 ist) steht, oder ausgewählt ist aus der Gruppe bestehend aus O, S, CH₂, C=O, NH, CHCH₃ und NCH₃, C(CH₃)₂, OCH₂ und SCH₂, wobei bei den beiden letztgenannten das Kohlenstoffatom an den aromatischen Teil und das Heteroatom O oder S an den teilhydrierten Teil des Amins gebunden ist; und
- n: die Laufzahl 1 oder 2 ist.

Eine zwölfte Ausführungsform der vorliegenden Erfindung umfasst Verbindungen der allgemeinen Formel (I), in welchen
- R¹, R² und R³: jeweils unabhängig voneinander ausgewählt sind aus der Gruppe, bestehend aus Wasserstoff, Amino, Trifluoromethyl, Methyl und (C₁-C₃)-Alkylsulfonyl; oder
- R¹ mit R²: über eine Bindung miteinander verbunden sind, so dass sich ein Cyclohexylring bildet, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere Halogenatome substituiertes Phenyl, (C₁-C₆)-Alkyl, (C₃-C₆)-Cycloalkyl, und (C₁-C₃)-Haloalkyl, insbesondere Trifluoromethyl substituiert ist, und
- R³: Wasserstoff, Methyl, Methylsulfonyl oder Amino ist; oder
- R¹ mit R²: über eine Bindung miteinander verbunden sind, so dass sich ein 6-gliedriger teilhydrierter Heterocyclus mit einem Schwefelatom bildet, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere Halogenatome substituiertes Phenyl, (C₁-C₆)-Alkyl, (C₃-C₆)-Cycloalkyl, und (C₁-C₃)-Haloalkyl substituiert ist,und
- R³: Wasserstoff, Methyl, Methylsulfonyl oder Amino ist;
- R⁴: ausgewählt ist aus der Gruppe bestehend aus Wasserstoff, CH₃ und COOCH₃;
- R⁵ und R⁶: jeweils unabhängig voneinander ausgewählt sind aus der Gruppe bestehend aus Wasserstoff, (C₁-C₆)-Alkyl und (C₁-C₆)-Alkoxy;
- R⁷ und R⁸: jeweils unabhängig voneinander ausgewählt sind aus der Gruppe bestehend aus Wasserstoff, Methyl und Phenyl;
- A¹, A² und A³: jeweils unabhängig voneinander ausgewählt sind aus S, CR⁹, CR¹⁰, und CR¹¹, wobei genau ein Atom aus A¹, A² und A³ S ist;
- R⁹, R¹⁰ und R¹¹: jeweils unabhängig voneinander, ausgewählt sind aus der Gruppe, bestehend aus Wasserstoff, Chlor und (C₁-C₃)-Alkyl;
- X: für eine Bindung (falls n=1 oder 2 ist) steht, oder CH₂ oder O ist; und
- n: die Laufzahl 1 oder 2 ist.

Eine dreizehnte Ausführungsform der vorliegenden Erfindung umfasst Verbindungen der allgemeinen Formel (I), in welchen
- R¹, R² und R³: jeweils verschieden voneinander sind und ausgewählt sind aus der Gruppe bestehend aus Wasserstoff, Amino, Trifluoromethyl, Methyl und Methylsulfonyl; oder
- R¹ mit R²: über eine Bindung miteinander verbunden sind, so dass sich ein Cyclohexylring bildet, der optional mit =O substituiert ist, und
- R³: Wasserstoff, Methyl oder Amino ist; oder
- R¹ mit R²: über eine Bindung miteinander verbunden sind, so dass sich 6-gliedriger teilhydrierter Hetercyclus mit einem Schwefelatom bildet, der mit 2 Oxogruppen substituiert ist, und
- R³: Wasserstoff, Methyl oder Amino ist;
- R⁴: Wasserstoff ist;
- R⁵ und R⁶: jeweils unabhängig voneinander Wasserstoff und Methyl sind;
- R⁷ und R⁸: jeweils unabhängig voneinander Wasserstoff und Methyl sind;
- A¹, A² und A³: jeweils unabhängig voneinander ausgewählt sind aus S, CR⁹, CR¹⁰ und CR¹¹, wobei genau ein Atom aus A¹, A² und A³ S ist;
- R⁹, R¹⁰, und R¹¹: jeweils unabhängig voneinander Wasserstoff oder Methyl sind; und bevorzugt, dass genau ein Rest R⁹, R¹⁰ oder R¹¹ Methyl ist und die anderen beiden Reste Wasserstoff sind.
- X: für eine Bindung (falls n=1 oder 2 ist) steht, oder CH₂ oder O ist; und
- n: die Laufzahl 1 oder 2 ist.

Im Rahmen der vorliegenden Erfindung sind von der Verbindung der allgemeinen Formel (I) auch Verbindungen umfasst, die durch a) Protonierung, b) Alkylierung oder c) Oxidation an einem Stickstoffatom quaterniert sind. Insbesondere sind diesbezüglich die entsprechenden N-Oxide zu nennen.

Die Verbindungen der Formel (I) können Salze bilden. Salzbildung kann durch Einwirkung einer Base auf solche Verbindungen der Formel (I) erfolgen, die ein acides Wasserstoffatom tragen. Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R"']⁺, worin R bis R'" jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Im Folgenden werden die Verbindungen der Formel (I) und ihre Salze auch kurz als erfindungsgemäß verwendete oder erfindungsgemäße "Verbindungen (I)" bezeichnet.

In der allgemeinen Formel (I) und allen übrigen Formeln in der vorliegenden Erfindung können die Reste Alkyl, Alkoxy, Haloalkyl, Haloalkoxy, Alkylamino, Alkylthio, Haloalkylthio, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 6 Kohlenstoffatomen, insbesondere 1 bis 4 Kohlenstoffatomen, bzw. bei ungesättigten Gruppen mit 2 bis 6 Kohlenstoffatomen, insbesondere 2 bis 4 Kohlenstoffatomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexy und 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Vinyl, Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tri-cyclischer Form vorkommen.

Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl.

Alkylen-Gruppen sind bei diesen Resten die niederen Kohlenstoffgerüste, z. B. mit 1 bis 10 C-Atomen, insbesondere 1 bis 6 C-Atomen oder bevorzugt 2 bis 4 C-Atomen, sowie die entsprechenden ungesättigten und/oder substituierten Reste im Kohlenstoffgerüst, die jeweils geradkettig oder verzweigt sein können. Beispiele sind Methylen, Ethylen, n- und i- Propylen und n-, s-, i-, t-Butylen.

Hydroxyalkylgruppen sind bei diesen Resten die niederen Kohlenstoffgerüste, z. B. mit 1 bis 6 C-Atomen, insbesondere 1 bis 4 C-Atomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder Iod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CF₂Cl, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Aryl bedeutet ein mono-, bi- oder polycyclisches aromatisches System, beispielsweise Phenyl oder Naphthyl, vorzugsweise Phenyl.

Ein Heterocyclus bedeutet ein Carbocyclus, in dem mindestens ein C-Atom durch ein Heteroatom ersetzt ist, vorzugsweise durch ein Heteroatom aus der Gruppe N, O, S, und P.

Der heterocyclische Ring enthält vorzugsweise 5 bis 7 Ringatome, insbesondere 5 bis 6, und ein oder mehrere, vorzugsweise 1 bis 2, insbesondere 1 Heteroatome im heterocyclischen Ring, vorzugsweise aus der Gruppe N, O, und S, besonders bevorzugt O oder S und insbesondere bevorzugt S.

Die Heterocyclen sind optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl und (C₁-C₆)-Haloalkyl substituiert.
Vor allem aus den Gründen der höheren herbiziden Wirkung, besseren Selektivität und/oder besseren Herstellbarkeit sind erfindungsgemäße Verbindungen der allgemeinen Formel (I) oder deren agrochemischen Salze oder quartären N-Derivate von besonderem Interesse, worin einzelne Reste eine der bereits genannten oder im Folgenden genannten bevorzugten Bedeutungen haben, oder insbesondere solche, worin eine oder mehrere der bereits genannten oder im folgenden genannten bevorzugten Bedeutungen kombiniert auftreten.

Die oben angeführten allgemeinen oder in Vorzugsbereichen angeführten Restedefinitionen gelten sowohl für die Endprodukte der allgemeinen Formel (I) als auch entsprechend für die die jeweils zur Herstellung benötigten Ausgangs- und Zwischenprodukte. Diese Restedefinitionen können untereinander, als auch zwischen den angegebenen bevorzugten Bereichen vertauscht werden.

Sofern Tautomere möglich sind, sind mit der beschriebenen Form alle möglichen tautomere Strukturen umfasst. Wie nachfolgend dargestellt, sind wenn zum Beispiel für R¹ und / oder R³ = Hydroxy beschrieben ist, die möglichen Keto-Tautomeren ebenfalls erfasst, anloges gilt für Amino und mögliche Imino-Tautomere.

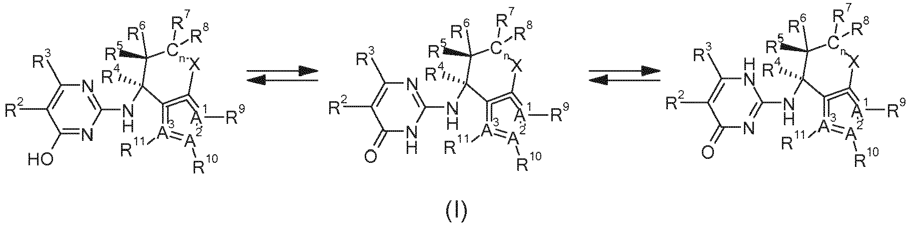

Die vorliegenden Verbindungen der allgemeinen Formel (I) weisen an der Bindungstelle zum Aminopyrimidin ein chirales Kohlenstoffatom auf, welches in der unten dargestellten Struktur durch die Kennzeichnung (*) verdeutlicht ist:

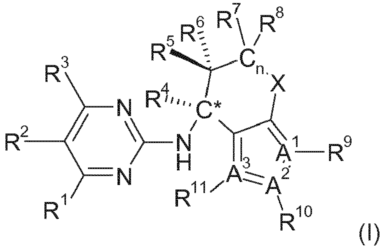

Gemäß den Regeln nach Cahn, Ingold und Prelog (CIP-Regeln) kann dieses Kohlenstoffatom sowohl eine (R)- als auch eine (S)-Konfiguration aufweisen.

Von der vorliegenden Erfindung werden Verbindungen der allgemeinen Formel (I) sowohl mit (S)- als auch mit (R)-Konfiguration erfasst, d.h., dass die vorliegende Erfindung die Verbindungen der allgemeinen Formel (I) erfasst, in welchen das betreffende Kohlenstoffatom
(1) eine (R)-Konfiguration; oder
(2) eine (S)-Konfiguration
aufweist.

Darüber hinaus werden im Rahmen der vorliegenden Erfindung auch
(3) beliebige Mischungen von Verbindungen der allgemeinen Formel (I), welche eine (R)-Konfiguration (Verbindungen der allgemeinen Formel (I-(R)) aufweisen, mit Verbindungen der allgemeinen Formel (I), welche eine (S)-Konfiguration (Verbindungen der allgemeinen Formel (I-S)) aufweisen,
erfasst, wobei eine racemische Mischung der Verbindungen der allgemeinen Formel (I) mit (R)- und (S)-Konfiguration von der vorliegenden Erfindung ebenfalls umfasst ist.

Allerdings sind im Rahmen der vorliegenden Erfindung insbesondere Verbindungen der allgemeinen Formel (I) mit (R)-Konfiguration mit einer Selektivität von 60 bis 100%, vorzugsweise 80 bis 100%, insbesondere 90 bis 100%, ganz besonders 95 bis 100%, bevorzugt, wobei die jeweilige (R)-Verbindung mit einer Enantioselektivität von jeweils mehr als 50% ee, vorzugsweise 60 bis 100% ee, insbesondere 80 bis 100% ee, ganz besonders 90 bis 100% ee, meist bevorzugt 95 bis 100% ee, bezogen auf den Gesamtgehalt an betreffender (R)-Verbindung vorliegt.

Daher betrifft die vorliegende Erfindung insbesondere Verbindungen der allgemeinen Formel (I*), in welchen die stereochemische Konfiguration an dem mit (*) gekennzeichnet Kohlenstoffatom mit einer stereochemischen Reinheit von 60 bis 100 % (R), vorzugsweise 80 bis 100 % (R), insbesondere 90 bis 100 % (R), ganz besonders 95 bis 100 % (R), vorliegt.

Unter Berücksichtigung der Regel nach Cahn, Ingold und Prelog kann es an dem mit (*) gekennzeichneten Kohlenstoffatom auch zu einer Situation kommen, in welcher aufgrund der Priorität der jeweiligen Substituenten die (S)-Konfiguration am mit (*) gekennzeichneten Kohlenstoffatom bevorzugt ist. Dieses ist beispielweise dann der Fall, wenn die Reste R⁴ und/oder R⁵ einem C₁-C₆-Alkoxyrest entsprechen.

Daher sind im Rahmen der vorliegenden Erfindung insbesondere Verbindungen der allgemeinen Formel (I) bevorzugt, die in ihrer räumlichen Anordnung den jenigen Verbindungen der allgemeinen Formel (I) mit R⁴ und R⁵ =Wasserstoff mit (R)-Konfiguration entsprechen, mit einer Selektivität von 60 bis 100 %, vorzugsweise 80 bis 100 %, insbesondere 90 bis 100 %, ganz besonders 95 bis 100 %, bevorzugt, wobei die jeweilige (R)-analoge-Verbindung mit einer Enantioselektivität von jeweils mehr als 50 % ee, vorzugsweise 60 bis 100 % ee, insbesondere 80 bis 100 % ee, ganz besonders 90 bis 100 % ee, meist bevorzugt 95 bis 100 % ee, bezogen auf den Gesamtgehalt an betreffender (R)-analogen-Verbindung, vorliegt. Daher betrifft die vorliegende Erfindung insbesondere Verbindungen der allgemeinen Formel (I), in welchen die stereochemische Konfiguration an dem mit (*) gekennzeichnet Kohlenstoffatom mit einer stereochemischen Reinheit von 60 bis 100 % (R, bzw. analog-R), vorzugsweise 80 bis 100 % (R, bzw. analog-R), insbesondere 90 bis 100 % (R, bzw. analog-R), ganz besonders 95 bis 100 % (R, bzw. analog-R), vorliegt.

Insbesondere können die erfindungsgemäßen Verbindungen der allgemeinen Formel (I) noch weitere Chiralitätszentren an den mit (**) und (***) gekennzeichneten Kohlenstoffatomen aufweisen:

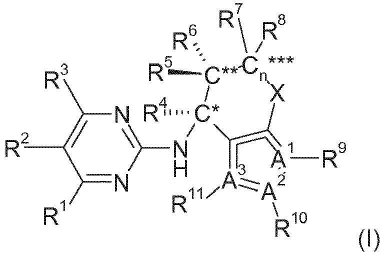

Im Rahmen der vorliegenden Erfindung sind beliebige stereochemische Konfigurationen an den mit (*), (**) und (***) gekennzeichneten Kohlenstoffatomen möglich:

| Konfiguration Kohlenstoffatom (*) | Konfiguration Kohlenstoffatom (**) | Konfiguration Kohlenstoffatom (***) |
|---|---|---|
| R | R | R |
| R | R | S |
| R | S | R |
| S | R | R |
| R | S | S |
| S | R | S |
| S | S | R |
| S | S | S |

Darüber hinaus können, je nach Wahl der jeweiligen Reste, weitere Stereoelemente in den erfindungsgemäßen Verbindungen der allgemeinen Formel (I) vorliegen.

Sind beispielsweise eine oder mehrere Alkenylgruppen vorhanden, so können Diastereomere (Z- und E-Isomere) auftreten.

Sind beispielsweise ein oder mehrere asymmetrische Kohlenstoffatome vorhanden, so können Enantiomere und Diastereomere auftreten.

Entsprechende Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfaßt werden, jedoch nicht mit ihrer spezifischen Stereoform angegeben sind und deren Gemische.

Die Kombinationsmöglichkeiten der verschiedenen Substituenten der allgemeinen Formel (I) sind so zu verstehen, dass die allgemeinen Grundsätze des Aufbaus chemischer Verbindungen zu beachten sind, d.h. die Formel (I) nicht Verbindungen umfasst, von denen der Fachmann weiß, dass sie chemisch nicht möglich sind.

Im Folgenden werden in tabellarischer Form Beispiele der Verbindungen der allgemeinen Formel (I) wiedergegeben.

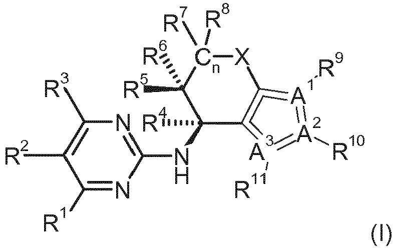

In den nachfolgenden Tabellen 1 und 2 werden die in Formel (I) allgemein definierten Substituenten spezifiziert. Dabei steht:
- "StNR⁴" für stereochemische Anordnung am Kohlenstoffatom, an das NH und R⁴ gebunden sind,
   "StR⁵R⁶" und "StR⁷R⁸" steht analog für die Kohlenstoffatome an die die jeweiligen Substituienten gebunden sind,
- die Bindung der Substituenten ist jeweils links,
- sofern für X zwei Bindestellen angegeben sind, bindet die linke Bindung an den Aromaten und die rechte Bindung an den hydrierten Teil des bicyclischen Amins,
- ein Bindestrich "-" für eine direkte Bindung, und
- falls n=0 ist enthält die Tabelle in dem entsprechenden Feld für R⁶ und R⁷ keinen Eintrag.

**Tabelle 1**

| Nr. | R¹ | R² | R³ | R⁴ | St N R⁴ | R⁵ | R⁶ | St R⁵ R⁶ | R⁷ | R⁸ | n | St R⁷ R⁸ | A¹-R⁹ | A²-R¹⁰ | A³-R¹¹ | X |
|---|---|---|---|---|---|---|---|---|---|---|---|---|---|---|---|---|
| 1. | -NH₂ | -CF₃ | -H | -H | Rac | -H | -H | | -H | -H | 2 | | S | C-Cl | C-Cl | - |
| 2. | -NH₂ | -CF₃ | -H | -H | Rac | -H | -H | | -H | -H | 2 | | C-CH₃ | S | C-CH₃ | - |
| 3. | -NH₂ | -SO₂-CH₃ | -H | -H | Rac | -H | -H | | -H | -H | 2 | | C-CH₃ | S | C-H | - |
| 4. | -NH₂ | -SO₂-CH₃ | -H | -H | Rac | -H | -H | | -H | -H | 1 | | S | C-CH₃ | C-H | - |
| 5. | T1 | -CF₃ | -H | -H | Rac | -H | -H | | -H | -H | 2 | | S | C-CH₃ | C-H | - |
| 6. | -NH₂ | -SO₂-CH₃ | -H | -H | Rac | -H | -H | | -H | -H | 2 | | C-CH₃ | S | C-CH₃ | - |
| 7. | -NH₂ | -CF₃ | -H | -H | Rac | -H | -H | | -H | -H | 2 | | O | C-H | C-H | - |
| 8. | -(CH₂)₃C(=O)- | | -H | -H | Rac | -H | -H | | -H | -H | 2 | | C-H | C-CH₃ | S | - |
| 9. | -(CH₂)₃C(=O)- | | -H | -H | Rac | -H | -H | | -H | -H | 2 | | S | C-CH₃ | C-H | - |
| 10. | -OCH₃ | -CF₃ | -H | -H | Rac | -H | -H | | -H | -H | 2 | | S | C-CH₃ | C-H | - |
| 11. | -NH₂ | -CF₃ | -H | -H | Rac | -H | -H | | -H | -H | 2 | | S | C-CH₃ | C-H | - |
| 12. | -NH₂ | -F | -H | -H | Rac | -H | -H | | -H | -H | 2 | | S | C-CH₃ | C-H | - |
| 13. | -NH₂ | -SO₂-CH₃ | -H | -H | Rac | -H | -H | | -H | -H | 2 | | O | C-H | C-H | - |
| 14. | -NH₂ | -CClF₂ | -H | -H | Rac | -H | -H | | -H | -H | 2 | | S | C-CH₃ | C-H | - |
| 15. | -NH₂ | -SO₂-CH₃ | -H | -H | Rac | -H | -H | | -CH₃ | - CH₃ | 1 | | O | C-CH₃ | C-H | CH₂ |
| 16. | -NH₂ | -SO₂-CH₃ | -Cl | -H | Rac | -H | -H | | -H | -H | 2 | | S | C-CH₃ | C-H | - |
| 17. | -NH₂ | -SO₂-CH₃ | -H | -H | Rac | -CH₃ | -H | rac | -H | -H | 1 | | S | C-CH₃ | C-H | - |
| 18. | -NH₂ | -SO₂-CH₃ | -H | -H | Rac | -H | -H | | -H | -H | 1 | | C-H | C-CH₃ | S | - |
| 19. | -NH₂ | -SO₂-CH₃ | -H | -H | Rac | -H | -H | | -H | -H | 2 | | C-H | C-CH₃ | S | - |
| 20. | -NH₂ | -SO₂-CH₃ | -H | -H | Rac | -H | -H | | -H | -H | 2 | | S | C-CH₃ | C-CH₃ | - |
| 21. | -NH₂ | -CF₃ | -H | -H | Rac | -H | -H | | -CH₃ | - CH₃ | 1 | | O | C-CH₃ | C-H | CH₂ |
| 22. | -NH₂ | -CF₃ | -H | -H | Rac | -H | -H | | -H | -H | 2 | | S | C-Cl | C-H | - |
| 23. | -NH₂ | -SO₂-CH₃ | -H | -H | Rac | -H | -H | | -H | -H | 2 | | S | C-H | C-H | - |
| 24. | -NH₂ | -SO₂-CH₃ | -H | -H | R | -H | -H | | -H | -H | 2 | | S | C-H | C-H | - |

| Nr. | R¹ | R² | R³ | R⁴ | St N R⁴ | R⁵ | R⁶ | St R⁵ R⁶ | R⁷ | R⁸ | n | St R⁷ R⁸ | A¹-R⁹ | A²-R¹⁰ | A³- R¹¹ | X |
|---|---|---|---|---|---|---|---|---|---|---|---|---|---|---|---|---|
| 25. | -NH₂ | -SO₂-CH₃ | -H | -H | Rac | -H | -H | | -H | -H | 2 | | O | C-CH₃ | C-CH₃ | - |
| 26. | -NH₂ | -CF₂H | -H | -H | Rac | -H | -H | | -H | -H | 2 | | S | C-CH₃ | C-H | - |
| 27. | -NH₂ | -S(=O),(=NH)-Ph | -H | -H | Rac | -H | -H | | -H | -H | 2 | | S | C-H | C-H | - |
| 28. | -NH₂ | -CF₃ | -H | -H | Rac | -H | -H | | -H | -H | 2 | | O | C-CH₃ | C-H | - |
| 29. | -NH₂ | -CF₃ | -H | -H | Rac | -H | -H | | -H | -H | 1 | | C-H | C-H | S | - |
| 30. | -NH₂ | -C(=O)CClF₂ | -H | -H | Rac | -H | -H | | -H | -H | 2 | | S | C-CH₃ | C-H | - |
| 31. | -NH₂ | -CF₃ | -H | -H | Rac | -H | -H | | -H | -H | 2 | | S | C-CH₃ | C-CH₃ | - |
| 32. | -NH₂ | -NO₂ | -H | -H | Rac | -H | -H | | -H | -H | 1 | | C-H | C-H | S | - |
| 33. | -NH₂ | -SO₂-CH₃ | -H | -H | Rac | -H | -H | | -H | -H | 1 | | C-H | C-H | S | - |
| 34. | -NH₂ | -CF₃ | -H | -H | Rac | -H | -H | | -H | -H | 2 | | S | C-C(CH₃)₃ | C-H | - |
| 35. | -NH₂ | -SO-CH₃ | -CF₃ | -H | Rac | -H | -H | | -H | -H | 2 | | S | C-CH₃ | C-H | - |
| 36. | -NH₂ | -SO₂-Cyclopropyl | -H | -H | Rac | -H | -H | | -H | -H | 2 | | S | C-H | C-H | - |
| 37. | -NH₂ | -SO₂-(CH₂)₂-CH₃ | -H | -H | Rac | -H | -H | | -H | -H | 2 | | S | C-H | C-H | - |
| 38. | -NH₂ | -SO₂-NH-CH₂-(4-F-Ph) | -H | -H | Rac | -H | -H | | -H | -H | 1 | | S | C-CH₃ | C-H | - |
| 39. | -NH₂ | -SO-CH₃ | -Cl | -H | Rac | -H | -H | | -H | -H | 2 | | S | C-CH₃ | C-H | - |
| 40. | -NH₂ | -NO₂ | -H | -H | Rac | -H | -H | | -CH₃ | CH₃ | 1 | | O | C-CH₃ | C-H | CH₂ |
| 41. | -NH₂ | -CF₃ | -H | -H | Rac | -H | -H | | -H | -H | 2 | | S | C-CF₃ | C-H | - |
| 42. | -NH₂ | -SO₂-CH₃ | -H | -H | Rac | -H | -H | | -H | -H | 2 | | S | C-Cl | C-H | - |
| 43. | -NH₂ | -CF₃ | -H | -H | Rac | -H | -H | | -H | -H | 2 | | S | C-CH₃ | C-H | - |
| 44. | -(CH₂)₃-S(=O)₂- | | -H | -H | R | -H | -H | | -H | -H | 2 | | S | C-CH₃ | C-H | - |
| 45. | -NH₂ | -SO₂-CH₃ | -H | -H | Rac | -H | -H | | | | | | S | C-H | C-H | C(=O) |
| 46. | -NH₂ | -CF₃ | -H | -H | R | -H | -H | | -H | -H | 2 | | S | C-H | C-H | - |
| 47. | -NH₂ | -CF₃ | -H | -H | R | -H | -H | | -H | -H | 2 | | S | C-CH₃ | C-H | - |
| 48. | -NH₂ | -CF₃ | -H | -H | Rac | -H | -H | | -H | -H | 1 | | C-H | C-Cl | S | - |
| 49. | -NH₂ | -CN | -H | -H | Rac | -H | -H | | -H | -H | 1 | | C-H | C-Cl | S | - |
| 50. | -NH₂ | -SO₂-CH₃ | -H | -H | Rac | -H | -H | | -H | -H | 1 | | C-H | C-Cl | S | - |
| 51. | -NH₂ | -CF₃ | -H | -H | Rac | -CH₃ | - CH₃ | | -H | -H | 2 | | S | C-CH₃ | C-H | - |
| 52. | -NH₂ | -CN | -H | -H | Rac | -H | -H | | -H | -H | 2 | | S | C-CH₃ | C-H | - |
| 53. | -NH₂ | -SO₂-CH₃ | -H | -H | Rac | -H | -H | | -H | -H | 2 | | C-H | C-Cl | S | - |
| 54. | -(CH₂)₃-C(=O)- | | -H | -H | Rac | -H | -H | | -H | -H | 2 | | O | C-H | C-H | - |
| 55. | -NH₂ | -CF₃ | -H | -H | Rac | -H | -H | | -H | -H | 2 | | C-H | C-CH₃ | s | - |
| 56. | -NH₂ | -CN | -H | -H | Rac | -H | -H | | -H | -H | 2 | | C-CH₃ | S | C-CH₃ | - |
| 57. | -NH₂ | -CN | -H | -H | Rac | -H | -H | | -H | -H | 2 | | C-CH₃ | S | C-H | - |
| 58. | -NH₂ | -SO₂-CH₃ | -H | -H | Rac | -H | -H | | -H | -H | 2 | | C-CH₃ | C-CH₃ | S | - |
| 59. | -NH₂ | -SO₂-CH₃ | -H | -H | Rac | -H | -H | | -H | -H | 2 | | S | C-CH₃ | C-H | - |
| 60. | -CH₃ | -C(=O)-CH₃ | -H | -H | Rac | -H | -H | | -H | -H | 2 | | O | C-H | C-H | - |
| 61. | -NH₂ | -CF₃ | -H | -H | Rac | -H | -H | | -H | -H | 2 | | S | C-CH₃ | C-H | - |
| 62. | -NH₂ | -SO₂-CH₃ | -H | -H | Rac | -H | -H | | -H | -H | 2 | | O | C-CH₃ | C-H | - |
| 63. | -NH₂ | -SO₂-CH-(CH₃)₂ | -H | -H | Rac | -H | -H | | -H | -H | 1 | | S | C-CH₃ | C-H | - |
| 64. | -NH₂ | -CN | -CH₃ | -H | Rac | -H | -H | | -H | -H | 2 | | C-CH₃ | S | C-CH₃ | - |
| 65. | -NH₂ | -CF₃ | -H | -H | Rac | -H | -H | | -H | -H | 2 | | C-H | O | C-H | - |
| 66. | -NH₂ | -SO₂-(CH₂)₃-OCH₃ | -H | -H | Rac | -H | -H | | -H | -H | 2 | | S | C-H | C-H | - |
| 67. | -NH₂ | -CF₃ | -H | -H | Rac | -COOCH₃ | -H | | -H | -H | 2 | | S | C-CH₃ | C-H | - |
| 68. | -(CH₂)₃-S(=O)₂- | | -H | -H | Rac | -H | -H | | -H | -H | 2 | | C-CH₃ | S | C-CH₃ | - |
| 69. | -NH₂ | -SO₂-CH₃ | -H | -H | Rac | -H | -H | | -H | -H | 2 | | C-H | C-H | S | - |
| 70. | -NH₂ | -CF₃ | -H | -H | Rac | -H | -H | | -H | -H | 2 | | S | C-H | C-H | - |
| 71. | -CH₃ | -C(=O)-CH₃ | -H | -H | R | -H | -H | | -H | -H | 2 | | S | C-H | C-H | - |
| 72. | -NH₂ | -C(=O)-CF₃ | -H | -H | Rac | -H | -H | | -H | -H | 2 | | S | C-CH₃ | C-H | - |
| 73. | -(CH₂)₃-S(=O)₂- | | -H | -H | R | -H | -H | | -H | -H | 2 | | S | C-H | C-H | - |
| 74. | -NH₂ | -CN | -CH₃ | -H | Rac | -H | -H | | -H | -H | 2 | | S | C-CH₃ | C-H | - |
| 75. | T2 | -CF₃ | -H | -H | Rac | -H | -H | | -H | -H | 2 | | S | C-CH₃ | C-H | - |
| 76. | -NH₂ | -SO₂-C(CH₃)₃ | -H | -H | Rac | -H | -H | | -H | -H | 2 | | S | C-CH₃ | C-H | - |
| 77. | -NH₂ | -SO₂-C(CH₃)₃ | -H | -H | Rac | -H | -H | | -H | -H | 1 | | S | C-CH₃ | C-H | - |
| 78. | -NH₂ | -CF₃ | -H | -H | Rac | -H | -H | | -H | -H | 2 | | S | C-CH₂-CH₃ | C-H | - |
| 79. | T3 | -CF₃ | -H | -H | Rac | -H | -H | | -H | -H | 2 | | S | C-CH₂-CH₃ | C-H | - |
| 80. | -CH₃ | -S-CF₃ | -H | -H | Rac | -H | -H | | -H | -H | 2 | | S | C-CH₃ | C-H | - |
| 81. | T4 | -CF₃ | -H | -H | Rac | -H | -H | | -H | -H | 2 | | C-H | C-CH₃ | S | - |
| 82. | T5 | -CF₃ | -H | -H | R | -H | -H | | -H | -H | 2 | | S | C-CH₃ | C-H | - |
| 83. | -NH₂ | -CN | -CH₂-CH₃ | -H | Rac | -H | -H | | -H | -H | 2 | | S | C-CH₃ | C-H | - |
| 84. | -NH₂ | -NO₂ | -H | -H | Rac | -H | -H | | -H | -H | 2 | | C-CH₃ | S | C-CH₃ | - |
| 85. | -NH₂ | -CF₂-CH₃ | -H | -H | Rac | -H | -H | | -H | -H | 2 | | S | C-CH₃ | C-H | - |
| 86. | -NHCOCH₃ | -CF₃ | -H | -H | Rac | -H | -H | | -H | -H | 2 | | S | C-CH₃ | C-H | - |
| 87. | -NHCOC(CH₃)₃ | -CF₃ | -H | -H | Rac | -H | -H | | -H | -H | 2 | | S | C-CH₃ | C-H | - |
| 88. | -NHCOCF₃ | -CF₃ | -H | -H | Rac | -H | -H | | -H | -H | 2 | | S | C-CH₃ | C-H | - |
| 89. | -NHCOPh | -CF₃ | -H | -H | Rac | -H | -H | | -H | -H | 2 | | S | C-CH₃ | C-H | - |
| 90. | -NHCO-(3-MeO-Ph) | -CF₃ | -H | -H | Rac | -H | -H | | -H | -H | 2 | | S | C-CH₃ | C-H | - |
| 91. | -NHCO-(3,5-di-Cl-Ph) | -CF₃ | -H | -H | Rac | -H | -H | | -H | -H | 2 | | S | C-CH₃ | C-H | - |
| 92. | -NHCOCH₂CH₂OCH₃ | -CF₃ | -H | -H | Rac | -H | -H | | -H | -H | 2 | | S | C-CH₃ | C-H | - |
| 93. | -NHCOCH₂OCH₃ | -CF₃ | -H | -H | Rac | -H | -H | | -H | -H | 2 | | S | C-CH₃ | C-H | - |
| 94. | -NHCO-(3-CF₃-Ph) | -CF₃ | -H | -H | Rac | -H | -H | | -H | -H | 2 | | S | C-CH₃ | C-H | - |
| 95. | -NHCO-(3,5-diCH₃-Ph) | -CF₃ | -H | -H | Rac | -H | -H | | -H | -H | 2 | | S | C-CH₃ | C-H | - |
| 96. | -N=CH-N(CH₃)₂ | -CF₃ | -H | -H | Rac | -H | -H | | -H | -H | 2 | | S | C-CH₃ | C-H | - |
| 97. | -N=CCH₃-N(CH₃)₂ | -CF₃ | -H | -H | Rac | -H | -H | | -H | -H | 2 | | S | C-CH₃ | C-H | - |
| 98. | T6 | -CF₃ | -H | -H | Rac | -H | -H | | -H | -H | 2 | | S | C-CH₃ | C-H | - |
| 99. | T7 | -CF₃ | -H | -H | Rac | -H | -H | | -H | -H | 2 | | S | C-CH₃ | C-H | - |
| 100. | -NHOH | -CF₃ | -H | -H | Rac | -H | -H | | -H | -H | 2 | | S | C-CH₃ | C-H | - |
| 101. | -OH | -CF₃ | -H | -H | Rac | -H | -H | | -H | -H | 2 | | S | C-CH₃ | C-H | - |
| 102. | -NH-NH₂ | -CF₃ | -H | -H | Rac | -H | -H | | -H | -H | 2 | | S | C-CH₃ | C-H | - |
| 103. | -NHOCH₃ | -CF₃ | -H | -H | Rac | -H | -H | | -H | -H | 2 | | S | C-CH₃ | C-H | - |
| 104. | -NHOCH₂CH₃ | -CF₃ | -H | -H | Rac | -H | -H | | -H | -H | 2 | | S | C-CH₃ | C-H | - |
| 105. | -NH-NHCOCH₃ | -CF₃ | -H | -H | Rac | -H | -H | | -H | -H | 2 | | S | C-CH₃ | C-H | - |
| 106. | -triazol-1yl- | -CF₃ | -H | -H | Rac | -H | -H | | -H | -H | 2 | | S | C-CH₃ | C-H | - |
| 107. | -O-(4-F-Ph) | -CF₃ | -H | -H | Rac | -H | -H | | -H | -H | 2 | | S | C-CH₃ | C-H | - |
| 108. | -NH-O-Ph | -CF₃ | -H | -H | Rac | -H | -H | | -H | -H | 2 | | S | C-CH₃ | C-H | - |
| 109. | -O-(3,5-diCH₃-Ph) | -CF₃ | -H | -H | Rac | -H | -H | | -H | -H | 2 | | S | C-CH₃ | C-H | - |
| 110. | -O-(4-CH₃-O-Ph) | -CF₃ | -H | -H | Rac | -H | -H | | -H | -H | 2 | | S | C-CH₃ | C-H | - |
| 111. | -OCH₂(4-F-Ph) | -CF₃ | -H | -H | Rac | -H | -H | | -H | -H | 2 | | S | C-CH₃ | C-H | - |
| 112. | -OCO(4-F-Ph) | -CF₃ | -H | -H | Rac | -H | -H | | -H | -H | 2 | | S | C-CH₃ | C-H | - |
| 113. | -NHCO(4-F-Ph) | -CF₃ | -H | -H | Rac | -H | -H | | -H | -H | 2 | | S | C-CH₃ | C-H | - |
| 114. | -COOCH₃ | -Cl | -H | -H | R | -H | -H | | -H | -H | 2 | | S | C-H | C-H | - |
| 115. | -CF₃ | -CH₃ | -H | -H | R | -H | -H | | -H | -H | 2 | | S | C-H | C-H | - |
| 116. | -CH₃ | -CH₃ | -H | -H | R | -H | -H | | -H | -H | 2 | | S | C-H | C-H | - |
| 117. | -(CH₂)₄- | | -H | -H | R | -H | -H | | -H | -H | 2 | | S | C-H | C-H | - |
| 118. | -(CH₂)₄- | | -CF₃ | -H | R | -H | -H | | -H | -H | 2 | | S | C-H | C-H | - |
| 119. | -(CH₂)₃C(=O)- | | -H | -H | R | -H | -H | | -H | -H | 2 | | S | C-H | C-H | - |
| 120. | -(CH₂)₃C(<Z>=NOH)- | | -H | -H | R | -H | -H | | -H | -H | 2 | | S | C-H | C-H | - |
| 121. | -(CH₂)₃C(<E>=NOH)- | | -H | -H | R | -H | -H | | -H | -H | 2 | | S | C-H | C-H | - |
| 122. | -(CH₂)₃C(<Z>=NOCH₃)- | | -H | -H | R | -H | -H | | -H | -H | 2 | | S | C-H | C-H | - |
| 123. | -(CH₂)₃C(<Z>=NOH)- | | -NH₂ | -H | R | -H | -H | | -H | -H | 2 | | S | C-H | C-H | - |
| 124. | -(CH₂)₃C(=O)- | | -NH₂ | -H | R | -H | -H | | -H | -H | 2 | | S | C-H | C-H | - |
| 125. | -NH-(CH₂)₂C(=O)- | | -H | -H | R | -H | -H | | -H | -H | 2 | | S | C-H | C-H | - |
| 126. | -NH-CH=CH-C(=O)- | | -H | -H | R | -H | -H | | -H | -H | 2 | | S | C-H | C-H | - |
| 127. | -NCH₃-(CH₂)₂C(=O)- | | -H | -H | R | -H | -H | | -H | -H | 2 | | S | C-H | C-H | - |
| 128. | -N(4-F-Ph)-CH=CH-C(=O)- | | -H | -H | R | -H | -H | | -H | -H | 2 | | S | C-H | C-H | - |
| 129. | -NCH₃-C(-COOCH₃)=CH-C(=O)- | | -H | -H | R | -H | -H | | -H | -H | 2 | | S | C-H | C-H | - |
| 130. | -NH-C(-COOCH₃)=CH-C(=O)- | | -H | -H | R | -H | -H | | -H | -H | 2 | | S | C-H | C-H | - |
| 131. | -N(CH₃)₂ | -F | -H | -H | R | -H | -H | | -H | -H | 2 | | S | C-H | C-H | - |
| 132. | -N(CH₃)-(CH₂)₃- | | -H | -H | R | -H | -H | | -H | -H | 2 | | S | C-H | C-H | - |
| 133. | -NH₂ | -F | -H | -H | R | -H | -H | | -H | -H | 2 | | S | C-H | C-H | - |
| 134. | -NH₂ | -CH₃ | -H | -H | R | -H | -H | | -H | -H | 2 | | S | C-H | C-H | - |
| 135. | -NH₂ | -CH₃ | -CH₃ | -H | R | -H | -H | | -H | -H | 2 | | S | C-H | C-H | - |
| 136. | -NH₂ | -(CH₂)₄- | | -H | R | -H | -H | | -H | -H | 2 | | S | C-H | C-H | - |
| 137. | -CH₂-O-CH₂-C(=O)- | | -H | -H | R | -H | -H | | -H | -H | 2 | | S | C-H | C-H | - |
| 138. | -CH₂-O-CH₂-C(=O)- | | -H | -H | R | -H | -H | | -H | -H | 2 | | S | C-CH₃ | C-H | - |
| 139. | -CH₂-CH(CH₃)-CH₂-C(=O)- | | -H | -H | R | -H | -H | | -H | -H | 2 | | S | C-H | C-H | - |
| 140. | -CH₂-CH(Ph)-CH₂-C(=O)- | | -H | -H | R | -H | -H | | -H | -H | 2 | | S | C-H | C-H | - |
| 141. | -CH₂-CH(4-F-Ph)-CH₂-C(=O)- | | -H | -H | R | -H | -H | | -H | -H | 2 | | S | C-CH₃ | C-H | - |
| 142. | -CH₂-CH(4-F-Ph)-CH₂-C(<E>=NOH)- | | -H | -H | R | -H | -H | | -H | -H | 2 | | S | C-CH₃ | C-H | - |
| 143. | -CH₂-CH(4-F-Ph)-CH₂-C(<Z>=NOCH₃)- | | -H | -H | R | -H | -H | | -H | -H | 2 | | S | C-H | C-H | - |
| 144. | -NH₂ | -CF₂-CF₃ | -H | -H | rac | -H | -H | | -H | -H | 2 | | S | C-CH₃ | C-H | - |
| 145. | -NH₂ | -C≡CH | -H | -H | rac | -H | -H | | -H | -H | 2 | | S | C-CH₃ | C-H | - |
| 146. | -NH₂ | -C≡CCH₃ | -H | -H | rac | -H | -H | | -H | -H | 2 | | S | C-CH₃ | C-H | - |
| 147. | -NH₂ | -C≡CPh | -H | -H | rac | -H | -H | | -H | -H | 2 | | S | C-CH₃ | C-H | - |
| 148. | -NH₂ | -C≡CCH₂-OH | -H | -H | rac | -H | -H | | -H | -H | 2 | | S | C-CH₃ | C-H | - |
| 149. | -NH₂ | -C(=O)-CH₂-CH₃ | -H | -H | rac | -H | -H | | -H | -H | 2 | | S | C-CH₃ | C-H | - |
| 150. | -NH₂ | -C(=O)-CH₃ | -H | -H | rac | -H | -H | | -H | -H | 2 | | S | C-CH₃ | C-H | - |
| 151. | -CH₂-CH₃ | -C(=O)-CH₂-CH₃ | -H | -H | rac | -H | -H | | -H | -H | 2 | | S | C-CH₃ | C-H | - |
| 152. | -N(CH₃)₂ | -CF₃ | -H | -H | rac | -H | -H | | -H | -H | 2 | | S | C-CH₃ | C-H | - |
| 153. | -NH₂ | -C(=O)-CH₂-O-CH₃ | -H | -H | R | -H | -H | | -H | -H | 2 | | S | C-CH₃ | C-H | - |
| 154. | -N(CH₃)₂ | -CF₃ | -H | -H | R | -H | -H | | -H | -H | 2 | | S | C-H | C-H | - |
| 155. | -N(CH₃)₂ | -CF₃ | -H | -H | rac | -H | -H | | -H | -H | 2 | | S | C-CH₃ | C-CH₃ | - |
| 156. | -N(CH₃)₂ | -C(=O)-CH₃ | -H | -H | R | -H | -H | | -H | -H | 2 | | S | C-H | C-H | - |
| 157. | -N(CH₃)₂ | -C(=O)-CH₃ | -H | -H | R | -H | -H | | -H | -H | 2 | | S | C-CH₃ | C-H | - |
| 158. | -CH₃ | -C(<E>=NOH)-CH₃ | -H | -H | R | -H | -H | | -H | -H | 2 | | S | C-H | C-H | - |
| 159. | -CH₃ | -C(<E>=NOCH₃)-CH₃ | -H | -H | R | -H | -H | | -H | -H | 2 | | S | C-H | C-H | - |
| 160. | -CH₃ | -C(<E>=NOPh)-CH₃ | -H | -H | R | -H | -H | | -H | -H | 2 | | S | C-H | C-H | - |
| 161. | -CH₃ | -CH(-OH)-CH₃ | -H | -H | R | -H | -H | | -H | -H | 2 | | S | C-H | C-H | - |
| 162. | -CH₃ | -CFH-CH₃ | -H | -H | R | -H | -H | | -H | -H | 2 | | S | C-H | C-H | - |
| 163. | -CH₃ | -CH(-OCH₂-Ph)-CH₃ | -H | -H | rac | -H | -H | | -H | -H | 2 | | S | C-H | C-H | - |
| 164. | -CH₃ | -CH(-OCH₂-4-F-Ph)-CH₃ | -H | -H | R | -H | -H | | -H | -H | 2 | | S | C-H | C-H | - |
| 165. | -CH₃ | -CH(-OCH₂-4-OCH₃-Ph)-CH₃ | -H | -H | R | -H | -H | | -H | -H | 2 | | S | C-H | C-H | - |
| 166. | -NH₂ | -CH(-OCH₂-4-OCH₃-Ph)-CH₃ | -H | -H | R | -H | -H | | -H | -H | 2 | | S | C-H | C-H | - |
| 167. | -NH₂ | -CH(-OH)-CH₃ | -H | -H | R | -H | -H | | -H | -H | 2 | | S | C-H | C-H | - |
| 168. | -NH₂ | -CH(-OH)-CH₃ | -H | -H | R | -H | -H | | -H | -H | 2 | | S | C-CH₃ | C-H | - |
| 169. | -NH₂ | -CFH-CH₃ | -H | -H | R | -H | -H | | -H | -H | 2 | | S | C-CH₃ | C-H | - |
| 170. | -NH₂ | -Cl | -H | -H | R | -H | -H | | -H | -H | 2 | | S | C-CH₃ | C-H | - |
| 171. | -NH₂ | -Cl | -H | -H | R | -H | -H | | -H | -H | 2 | | S | C-H | C-H | - |
| 172. | -NH₂ | -Br | -H | -H | R | -H | -H | | -H | -H | 2 | | S | C-CH₃ | C-H | - |
| 173. | -NH₂ | -I | -H | -H | R | -H | -H | | -H | -H | 2 | | S | C-CH₃ | C-H | - |
| 174. | -NH₂ | -COOCH₃ | -H | -H | R | -H | -H | | -H | -H | 2 | | S | C-CH₃ | C-H | - |

| Nr. | R¹ | R² | R³ | R⁴ | St N R⁴ | R⁵ | R⁶ | St R⁵ R⁶ | R⁷ | R⁸ | n | St R⁷ R⁸ | A¹-R⁹ | A²-R¹⁰ | A³-R¹¹ | X |
|---|---|---|---|---|---|---|---|---|---|---|---|---|---|---|---|---|
| 175. | CH₃ | -C(=O)-N(NCH₃)2 | -H | -H | R | -H | -H | | -H | -H | 2 | | S | C-CH₃ | C-H | - |
| 176. | -CH3 | -C(=O)-OCH₃ | -H | -H | R | -H | -H | | -H | -H | 2 | | S | C-H | C-H | - |
| 177. | -CH3 | -C(=O)-NH₂ | -H | -H | R | -H | -H | | -H | -H | 2 | | S | C-CH₃ | C-H | - |
| 178. | -NH₂ | -C(=O)-N-(CH₃)₂ | -H | -H | R | -H | -H | | -H | -H | 2 | | S | C-H | C-H | - |
| 179. | -NHCH₃ | -CF₃ | -H | -H | rac | -H | -H | | -H | -H | 2 | | O | C-H | C-H | - |
| 180. | CH₃ | -CF₃ | -H | -H | R | -H | -H | | -H | -H | 2 | | O | C-H | C-H | - |
| 181. | -CH₃ | -C(=O)-CH₃ | -H | -H | R | -H | -H | | -H | -H | 2 | | O | C-H | C-H | - |
| 182. | -CH₃ | -C(=O)-CH₃ | -H | -H | R | -H | -H | | -H | -H | 2 | | S | C-CH₃ | C-H | - |
| 183. | -CH₃ | -C(<E>=NOH)-CH₃ | -H | -H | R | -H | -H | | -H | -H | 2 | | S | C-CH₃ | C-H | - |
| 184. | -(CH₂)₂-C(<Z>=NOH)- | | -H | -H | R | -H | -H | | -H | -H | 2 | | S | C-CH₃ | C-H | - |
| 185. | -(CH₂)₂-C(=O)- | | -H | -H | R | -H | -H | | -H | -H | 2 | | S | C-CH₃ | C-H | - |
| 186. | -(CH₂)₂-C(=O)- | | -H | -H | R | -H | -H | | -H | -H | 2 | | S | C-H | C-H | - |
| 187. | -OCH₃ | -S-CF₃ | -H | -H | R | -H | -H | | -H | -H | 2 | | S | C-H | C-H | - |
| 188. | -CH₃ | -S-CF₃ | -H | -H | R | -H | -H | | -H | -H | 2 | | S | C-H | C-H | - |
| 189. | -NH₂ | -S-CF₃ | -H | -H | R | -H | -H | | -H | -H | 2 | | S | C-H | C-H | - |
| 190. | -(CH₂)-C(=O)-CH₂- | | -H | -H | R | -H | -H | | -H | -H | 2 | | S | C-H | C-H | - |
| 191. | -C(=O)-(CH₂)₃- | | -H | -H | R | -H | -H | | -H | -H | 2 | | S | C-H | C-H | - |
| 192. | -C(<E>=NOH)-(CH₂)₃- | | -H | -H | R | -H | -H | | -H | -H | 2 | | S | C-H | C-H | - |
| 193. | -NH₂ | -SO₂-CH₃ | -H | -H | R | -H | -H | | -H | -H | 1 | | S | C-H | C-H | O |
| 194. | -NH₂ | -SO₂-CH₃ | -H | -H | R | -H | -H | | -H | -H | 1 | | S | C-H | C-H | N-CH₃ |
| 195. | -NH₂ | -SO₂-CH₃ | -H | -H | R | -H | -H | | -H | -H | 1 | | S | C-H | C-H | N-Ph |
| 196. | -NH₂ | -SO₂-CH₃ | -H | -H | rac | -CH₃ | -H | rac | -H | -H | 2 | | S | C-H | C-H | - |
| 197. | -NH₂ | -Cl | -H | -H | rac | -CH₂-CH₃ | -H | rac | -H | -H | 2 | | S | C-H | C-H | - |
| 198. | -NH₂ | -CF₃ | -H | -H | rac | -spiro-Cyclopropyl | | | -H | -H | 2 | | S | C-H | C-H | - |
| 199. | -NH₂ | -SO₂-CH₃ | -NH₂ | -H | rac | -H | -H | | -H | -H | 2 | | S | C-CH₃ | C-H | - |
| 200. | -NH₂ | -SO₂-CH₃ | -NH₂ | -H | R | -H | -H | | -H | -H | 2 | | S | C-H | C-H | - |
| 201. | -NH₂ | -SO₂-CH₃ | -H | -CH₃ | rac | -H | -H | | -H | -H | 2 | | S | C-H | C-H | - |
| 202. | -NH₂ | -CF₃ | -H | -CH₃ | rac | -H | -H | | -H | -H | 2 | | S | C-H | C-H | - |
| 203. | -NH₂ | -SO₂-CH₃ | -H | -CN | rac | -H | -H | | -H | -H | 2 | | S | C-H | C-H | - |
| 204. | -NH₂ | -SO₂-CH₃ | -H | COOCH₃ | rac | -H | -H | | -H | -H | 2 | | S | C-H | C-H | - |
| 205. | -NH₂ | -SO₂-CH₃ | -H | | R | -CH₃ | -H | R | -H | -H | 2 | | S | C-H | C-H | - |
| 206. | -NH₂ | -CF₃ | -H | | R | -CH₃ | -H | S | -H | -H | 2 | | S | C-H | C-H | - |
| 207. | -NH₂ | -CF₃ | -H | -H | rac | -H | -H | | -CH₃ | -H | 1 | rac | S | C-CH₃ | C-H | CH₂ |
| 208. | -NH₂ | -CF₃ | -H | -H | rac | -H | -H | | -Ph | -H | 1 | rac | S | C-CH₃ | C-H | O |
| 209. | -NH₂ | -SO-CH₃ | -H | -H | rac | -H | -H | | -CH₃ | -H | 1 | R | S | C-CH₃ | C-H | CH₂ |
| 210. | -NH₂ | -SO-CH₃ | -H | -H | rac | -H | -H | | -CH₃ | -H | 1 | S | S | C-H | C-H | CH₂ |
| 211. | -CF₃ | -CN | -H | -H | R | -H | -H | | -H | -H | 2 | | S | C-H | C-H | - |
| 212. | -CF₃ | -CN | -H | -H | rac | -H | -H | | -H | -H | 2 | | S | C-CH₃ | C-H | - |
| 213. | -NH₂ | -CF₃ | -H | -H | rac | -H | -H | | -H | -H | 2 | | S | C-CH₃ | C-CH₃ | - |
| 214. | -T8 | -CF₃ | -H | -H | rac | -H | -H | | -H | -H | 2 | | S | C-CH₃ | C-CH₃ | - |
| 215. | -NH₂ | -CF₃ | -H | -H | rac | -H | -H | | -H | -H | 1 | | S | C-H | C-H | - |
| 216. | -CH₃ | -CF₃ | -H | -H | rac | -H | -H | | -H | -H | 2 | | S | C-CH₂CH₃ | C-H | - |
| 217. | -CH₃ | -CF₃ | -H | -H | rac | -H | -H | | -H | -H | 1 | | C-H | C-H | S | - |
| 218. | -CH₃ | -CF₃ | -H | -H | rac | -H | -H | | -H | -H | 2 | | C-H | C-H | S | - |
| 219. | -CH₃ | -CF₃ | -H | -H | rac | -H | -H | | -H | -H | 2 | | C-CH₃ | S | C-CH₃ | - |
| 220. | -CH₃ | -CF₃ | -H | -H | R | -H | -H | | -H | -H | 2 | | S | C-CH₃ | C-H | - |
| 221. | -CF₃ | -COOCH₃ | -H | -H | rac | -H | -H | | -H | -H | 2 | | S | C-CH₃ | C-H | - |
| 222. | -NH₂ | -Cl | -CF₃ | -H | rac | -H | -H | | -H | -H | 2 | | S | C-CH₃ | C-H | - |
| 223. | -CH₃ | -F | -H | -H | R | -H | -H | | -H | -H | 2 | | S | C-H | C-H | - |
| 224. | -OCH₃ | -F | -H | -H | rac | -H | -H | | -H | -H | 2 | | S | C-CH₃ | C-H | - |
| 225. | -OCH₃ | -Cl | -H | -H | R | -H | -H | | -H | -H | 2 | | S | C-H | C-H | - |
| 226. | -OCH₃ | -Cl | -H | -H | rac | -H | -H | | -H | -H | 2 | | S | C-CH₃ | C-H | - |
| 227. | -OCH₃ | -Br | -H | -H | R | -H | -H | | -H | -H | 2 | | S | C-H | C-H | - |
| 228. | -OCH₃ | -Br | -H | -H | rac | -H | -H | | -H | -H | 2 | | S | C-CH₃ | C-H | - |
| 229. | -OCH₃ | -C≡CSi(CH₃)₃ | -H | -H | R | -H | -H | | -H | -H | 2 | | S | C-H | C-H | - |
| 230. | --OCH₃ | -C≡CH | -H | -H | R | -H | -H | | -H | -H | 2 | | S | C-H | C-H | - |
| 231. | -OCH₃ | -C≡CCH₂CH₂CH₂CH₃ | -H | -H | R | -H | -H | | -H | -H | 2 | | S | C-H | C-H | - |
| 232. | -OCH₃ | -C≡CSi(CH₃)₃ | -H | -H | rac | -H | -H | | -H | -H | 2 | | S | C-CH₃ | C-H | - |
| 233. | -OCH₃ | -C≡CH | -H | -H | rac | -H | -H | | -H | -H | 2 | | S | C-CH₃ | C-H | - |
| 234. | -OCH₃ | -C≡CCH₂CH₂CH₂CH₃ | -H | -H | rac | -H | -H | | -H | -H | 2 | | S | C-CH₃ | C-H | - |
| 235. | -CH₃ | -C(=O)-OCH₂CH₃ | -H | -H | R | -H | -H | | -H | -H | 2 | | S | C-H | C-H | - |
| 236. | -CH₃ | -C(=O)N(CH₃)₂ OH | -H | -H | R | -H | -H | | -H | -H | 2 | | S | C-H | C-H | - |
| 237. | -CH₃ | -C(=O)-N(CH₃)CH₂-4-F-Ph | -H | -H | R | -H | -H | | -H | -H | 2 | | S | C-H | C-H | - |
| 238. | -CH₃ | -C(=O)-azepin | -H | -H | R | -H | -H | | -H | -H | 2 | | S | C-H | C-H | - |
| 239. | -CH₃ | -C(=O)-N(CH₃)CH₂-2-F-Ph | -H | -H | R | -H | -H | | -H | -H | 2 | | S | C-CH₃ | C-H | - |
| 240. | -CH₃ | -C(=O)-OCH₂CH₃ | -H | -H | rac | -H | -H | | -H | -H | 2 | | S | C-CH₃ | C-H | - |
| 241. | -CH₃ | -C(=O)NHCH₃ | -H | -H | rac | -H | -H | | -H | -H | 2 | | S | C-CH₃ | C-H | - |
| 242. | -CH₃ | -C(=O)-azepin | -H | -H | rac | -H | -H | | -H | -H | 2 | | S | C-CH₃ | C-H | - |
| 243. | -CH₃ | -C(=O)-N(CH₃)CH₂-4-F-Ph | -H | -H | rac | -H | -H | | -H | -H | 2 | | S | C-CH₃ | C-H | - |
| 244. | -CH₃ | -C(=O)-N(CH₃)CH₂-2-F-Ph | -H | -H | rac | -H | -H | | -H | -H | 2 | | S | C-CH₃ | C-H | - |
| 245. | -NH₂ | -CF₃ | -H | -CH₃ | rac | -H | -H | | -H | -H | 2 | | S | C-CH₃ | C-H | - |
| 246. | -NH₂ | -SO₂-CH₃ | -NH₂ | -CH₃ | rac | -H | -H | | -H | -H | 2 | | S | C-H | C-H | - |
| 247. | -NH₂ | -SO₂-CH₃ | -H | -H | rac | -CH₃ | -H | rac | -H | -H | 2 | | S | C-H | C-H | - |
| 248. | -NH₂ | -CF₃ | -H | -H | rac | -H | -H | | | | 0 | | O | C-H | C-H | -CO- |
| 249. | -NH₂ | -CF₃ | -H | -H | rac | -H | -H | | -H | -H | 1 | | O | | | - |
| 250. | -NH₂ | -CF₃ | -H | -H | rac | -H | -H | | -H | -H | 2 | | C-H | C-H | O | - |
| 251. | -(CH₂)₃C(=O)- | | -H | -H | rac | -H | -H | | -H | -H | 2 | | C-H | C-H | O | - |
| 252. | -(CH₂)₃C(=N<Z>OH)- | | -H | -H | rac | -H | -H | | -H | -H | 2 | | C-H | C-H | O | - |
| 253. | -(CH₂)₃C(=N<E>OH)- | | -H | -H | rac | -H | -H | | -H | -H | 2 | | C-H | C-H | O | - |
| 254. | -(CH₂)₃C(=N<E>OCH₃)- | | -H | -H | rac | -H | -H | | -H | -H | 2 | | C-H | C-H | O | - |
| 255. | -(CH₂)₃C(=N<Z>OCH₃)- | | -H | -H | rac | -H | -H | | -H | -H | 2 | | C-H | C-H | O | - |
| 256. | -NH₂ | -CF₃ | -H | -H | rac | -H | -H | | -H | -H | 1 | | S | C-Cl | C-H | - |
| 257. | -NH₂ | -CF₃ | -H | -H | rac | -H | -H | | -H | -H | 2 | | S | C-CH₃ | C-H | - |
| 258. | -CH₃ | -COCH₃ | -H | -H | rac | -H | -H | | -H | -H | 2 | | S | C-Cl | C-H | - |
| 259. | -CH₃ | -C(=N<Z>OH)CH₃ | -H | -H | rac | -H | -H | | -H | -H | 2 | | S | C-Cl | C-H | - |
| 260. | -CH₃ | -C(=N<E>OH)CH₃ | -H | -H | rac | -H | -H | | -H | -H | 2 | | S | C-Cl | C-H | - |
| 261. | -CH₃ | -C(=N<Z>OCH₃)CH₃ | -H | -H | rac | -H | -H | | -H | -H | 2 | | S | C-Cl | C-H | - |
| 262. | -CH₃ | -C(=N<E>OCH₃)CH₃ | -H | -H | rac | -H | -H | | -H | -H | 2 | | S | C-Cl | C-H | - |
| 263. | -C(CH₃)=CH-C(=O)-N(-T9)- | | -H | -H | rac | -H | -H | | -H | -H | 2 | | S | C-CH₃ | C-H | - |
| 264. | -NH₂ | -SO-CH₃ | -CF₂H | -H | rac | -H | -H | | -H | -H | 2 | | S | C-CH₃ | C-H | - |
| 265. | -NH₂ | -3-F-Ph | -H | -H | rac | -H | -H | | -H | -H | 2 | | S | C-CH₃ | C-H | - |
| 266. | -NH₂ | -C(=O)-NHCH₃ | -H | -H | rac | -H | -H | | -H | -H | 2 | | S | C-CH₃ | C-H | - |
| 267. | -NH₂ | -C(=O)-NH₂ | -H | -H | rac | -H | -H | | -H | -H | 2 | | S | C-CH₃ | C-H | - |
| 268. | -NH₂ | -C(=O)--CH₃ | CF₃ | -H | rac | -H | -H | | -H | -H | 2 | | S | C-CH₃ | C-H | - |
| 269. | -CF₃ | -C(=O)-CH₃ | -H | -H | rac | -H | -H | | -H | -H | 2 | | S | C-CH₃ | C-H | - |
| 270. | -OCH₃ | -CF₃ | -H | -H | rac | -H | -H | | -H | -H | 1 | | S | C-H | C-H | - |
| 271. | T10 | -CF₃ | -H | -H | rac | -H | -H | | -H | -H | 1 | | S | C-H | C-H | - |
| 272. | -OCH₃ | -CF₃ | -H | -H | rac | -H | -H | | -H | -H | 1 | | S | C-CH₃ | C-H | - |
| 273. | T11 | -CF₃ | -H | -H | rac | -H | -H | | -H | -H | 1 | | S | C-CH₃ | C-H | - |
| 274. | -OCH₃ | -CF₃ | -H | -H | rac | -H | -H | | -H | -H | 2 | | C-H | C-CH₃ | S | - |

| Nr. | R¹ | R² | R³ | R⁴ | St N R⁴ | R⁵ | R⁶ | St R⁵ R⁶ | R⁷ | R⁸ | n | St R⁷ R⁸ | A¹-R⁹ | A²-R¹⁰ | A³-R¹¹ | X |
|---|---|---|---|---|---|---|---|---|---|---|---|---|---|---|---|---|
| 275. | -NH₂ | -C(=CH₂)-CF₃ | -H | -H | rac | -H | -H | | -H | -H | 2 | | S | C-CH₃ | C-H | - |
| 276. | -CH₃ | -C=N(<Z>-OCH₂CH₃)-CH₃ | -H | -H | rac | -H | -H | | -H | -H | 2 | | S | C-Cl | C-H | - |
| 277. | -CH₃ | -C(=N(<E>-OCH₂CH₃)-CH₃ | -H | -H | rac | -H | -H | | -H | -H | 2 | | S | C-Cl | C-H | - |
| 278. | -CH₃ | -C(=N(<Z>-OCH₂Ph-CH₃ | -H | -H | rac | -H | -H | | -H | -H | 2 | | S | C-Cl | C-H | - |
| 279. | -CH₃ | -C(=N(<E>-OCH₂Ph)-CH₃ | -H | -H | rac | -H | -H | | -H | -H | 2 | | S | C-Cl | C-H | - |
| 280. | -NH₂ | -Cl | -CF₃ | -H | R | -H | -H | | -H | -H | 2 | | S | C-CH₃ | C-H | - |
| 281. | -NH₂ | -Cl | -CF₃ | -H | rac | -H | -H | | -H | -H | 2 | | S | C-CH₃ | C-CH₃ | - |
| 282. | -NH₂ | -CN | -H | -H | R | -H | -H | | -H | -H | 2 | | S | C-CH₃ | C-H | - |
| 283. | -NH₂ | -C(=O)-CH₃ | -H | -H | R | -H | -H | | -H | -H | 2 | | S | C-CH₃ | C-H | - |
| 284. | -NH₂ | -Br | -CHF₂ | -H | rac | -H | -H | | -H | -H | 2 | | S | C-CH₃ | C-H | - |
| 285. | -NH₂ | -CF₃ | -H | -H | rac | -H | -H | | -H | -H | 2 | | C-H | C-CN | S | - |
| 286. | -NH₂ | -C=N(<E>-OH)-CH₃ | -H | -H | R | -H | -H | | -H | -H | 2 | | S | C-CH₃ | C-H | - |
| 287. | -NH₂ | -SO₂-CH₃ | -CHF₂ | -H | rac | -H | -H | | -H | -H | 2 | | S | C-CH₃ | C-H | - |
| 288. | -NH₂ | -C(=N(<E>-OCH(CH₃)₂)-CH₃ | -H | -H | rac | -H | -H | | -H | -H | 2 | | S | C-CH₃ | C-H | - |
| 289. | -NH₂ | -H | -CHF₂ | -H | rac | -H | -H | | -H | -H | 2 | | S | C-CH₃ | C-H | - |
| 290. | -NH₂ | -T12 | -H | -H | rac | -H | -H | | -H | -H | 2 | | S | C-CH₃ | C-H | - |
| 291. | -NH₂ | -C(=N(<Z>-OCH₂Ph)-CH₃ | -H | -H | rac | -H | -H | | -H | -H | 2 | | S | C-CH₃ | C-H | - |
| 292. | -NH₂ | -C(=N(<Z>-OCH₃)-CH₃ | -H | -H | rac | -H | -H | | -H | -H | 2 | | S | C-H | C-H | - |
| 293. | -NH₂ | -C(=O)OCH₂-CH₃ | -H | -H | rac | -H | -H | | -H | -H | 2 | | S | C-CH₃ | C-H | - |
| 294. | -NH₂ | -Ph-3-CF₃ | -H | -H | rac | -H | -H | | -H | -H | 2 | | S | C-CH₃ | C-H | - |
| 295. | -NH₂ | -C(=N(<E>-OCH₃)-CH₃ | -H | -H | rac | -H | -H | | -H | -H | 2 | | S | C-CH₃ | C-H | - |
| 296. | -NH₂ | -Ph | -H | -H | rac | -H | -H | | -H | -H | 2 | | S | C-CH₃ | C-H | - |
| 297. | -NH₂ | -Ph-3-Cl | -H | -H | rac | -H | -H | | -H | -H | 2 | | S | C-CH₃ | C-H | - |
| 298. | -NH₂ | -SO₂-CH₃ | -CF₃ | -H | rac | -H | -H | | -H | -H | 2 | | S | C-CH₃ | C-H | - |
| 299. | -NH₂ | -T13 | -CHF₂ | -H | rac | -H | -H | | -H | -H | 2 | | S | C-CH₃ | C-H | - |
| 300. | -NH₂ | -T14 | -CHF₂ | -H | rac | -H | -H | | -H | -H | 2 | | S | C-CH₃ | C-H | - |
| 301. | -NH₂ | -C(=O)-CH₃ | -H | -H | R | -H | -H | | -H | -H | 2 | | S | C-H | C-H | - |
| 302. | -NH₂ | -Ph-3-F | -CHF₂ | -H | rac | -H | -H | | -H | -H | 2 | | S | C-CH₃ | C-H | - |
| 303. | -CH₃ | -C(=O)-CH₃ | -H | -H | R | -H | -H | | -H | -H | 2 | | S | C-Cl | C-H | - |
| 304. | -NH₂ | -Ph-3-CH₃ | -CHF₂ | -H | rac | -H | -H | | -H | -H | 2 | | S | C-CH₃ | C-H | - |
| 305. | -NH₂ | -C(=N(<E>-OCH₂Ph)-CH₃ | -H | -H | rac | -H | -H | | -H | -H | 2 | | S | C-CH₃ | C-H | - |
| 306. | -NH₂ | -CH₂OH | -H | -H | R | -H | -H | | -H | -H | 2 | | S | C-CH₃ | C-H | - |
| 307. | -NH₂ | -Br | -CF(CH₃)₂ | -H | rac | -H | -H | | -H | -H | 2 | | S | C-CH₃ | C-H | - |
| 308. | -NH₂ | -Cl | -CHFCH₃ | -H | rac | -H | -H | | -H | -H | 2 | | S | C-CH₃ | C-H | - |
| 309. | -NH₂ | -SOCH₃ | -CHFCH₃ | -H | rac | -H | -H | | -H | -H | 2 | | S | C-CH₃ | C-H | - |
| 310. | -NH₂ | -C(=N(<E>-OCH₃)-CH₃ | -H | -H | rac | -H | -H | | -H | -H | 2 | | S | C-H | C-H | - |
| 311. | -NH₂ | -CH₃ | -CF(CH₃)₂ | -H | rac | -H | -H | | -H | -H | 2 | | S | C-CH₃ | C-H | - |
| 312. | -NH₂ | -Ph-3-NO₂ | -H | -H | rac | -H | -H | | -H | -H | 2 | | S | C-CH₃ | C-H | - |
| 313. | -NH₂ | -Ph-3-CN | -H | -H | rac | -H | -H | | -H | -H | 2 | | S | C-CH₃ | C-H | - |
| 314. | -NH₂ | -Br | -CHF₂ | -H | rac | -H | -H | | -H | -H | 2 | | S | C-CH₃ | C-H | - |
| 315. | -NH₂ | -SO₂-CH₃ | -CF(CH₃)₂ | -H | rac | -H | -H | | -H | -H | 2 | | S | C-CH₃ | C-H | - |
| 316. | -NH₂ | -T13 | -CHFCH₃ | -H | rac | -H | -H | | -H | -H | 2 | | S | C-CH₃ | C-H | - |
| 317. | -NH₂ | -Ph-3-F | -CHFCH₃ | -H | rac | -H | -H | | -H | -H | 2 | | S | C-CH₃ | C-H | - |
| 318. | -NH₂ | -SO₂-CH₃ | -CHFCH₃ | -H | rac | -H | -H | | -H | -H | 2 | | S | C-CH₃ | C-H | - |
| 319. | -NH₂ | -CN | -CF(CH₃)₂ | -H | rac | -H | -H | | -H | -H | 2 | | S | C-CH₃ | C-H | - |
| 320. | -NH₂ | -CN | -CHFCH₃ | -H | rac | -H | -H | | -H | -H | 2 | | S | C-CH₃ | C-H | - |
| 321. | -NH₂ | -Ph-3-F | -CF(CH₃)₂ | -H | rac | -H | -H | | -H | -H | 2 | | S | C-CH₃ | C-H | - |
| 322. | -CH₃ | -C≡C-Ph-4-CH₂CH₃ | -H | -H | rac | -H | -H | | -H | -H | 2 | | S | C-CH₃ | C-H | - |
| 323. | -NH₂ | -T13 | -CF(CH₃)₂ | -H | rac | -H | -H | | -H | -H | 2 | | S | C-CH₃ | C-H | - |
| 324. | -CH₃ | -C≡C-Ph-4-O-CH₃ | -H | -H | rac | -H | -H | | -H | -H | 2 | | S | C-CH₃ | C-H | - |
| 325. | -CF₃ | -Cl | -H | -H | R | -H | -H | | -H | -H | 2 | | S | C-CH₃ | C-H | - |
| 326. | -CF₃ | -Cl | -H | -H | R | -H | -H | | -H | -H | 2 | | S | C-H | C-H | - |
| 327. | -CH₃ | -C(=O)-T15 | -H | -H | rac | -H | -H | | -H | -H | 2 | | S | C-CH₃ | C-H | - |
| 328. | -NH₂ | -C(=O)H | -H | -H | rac | -H | -H | | -H | -H | 2 | | S | C-CH₃ | C-H | - |
| 329. | -NH₂ | -CN | -CHF₂ | -H | rac | -H | -H | | -H | -H | 2 | | S | C-CH₃ | C-H | - |
| 330. | -CH₃ | -C(=O)-T16 | -H | -H | rac | -H | -H | | -H | -H | 2 | | S | C-CH₃ | C-H | - |
| 331. | -CH₃ | -C(=O)NH-T17 | -H | -H | rac | -H | -H | | -H | -H | 2 | | S | C-CH₃ | C-H | - |
| 332. | -CF₃ | -C(=O)T18 | -H | -H | rac | -H | -H | | -H | -H | 2 | | S | C-CH₃ | C-H | - |
| 333. | -CF₃ | -C(=O)NH-T17 | -H | -H | rac | -H | -H | | -H | -H | 2 | | S | C-CH₃ | C-H | - |
| 334. | -CF₃ | -C(=O)N(CH₃)CH₂-2-F-Ph | -H | -H | rac | -H | -H | | -H | -H | 2 | | S | C-CH₃ | C-H | - |
| 335. | -CF₃ | -C(=O)-T16 | -H | -H | rac | -H | -H | | -H | -H | 2 | | S | C-CH₃ | C-H | - |
| 336. | -CF₃ | -C(=O)-T15 | -H | -H | rac | -H | -H | | -H | -H | 2 | | S | C-CH₃ | C-H | - |
| 337. | -(CH₂)₃-C(=O)- | | -H | -H | rac | -H | -H | | -H | -H | 2 | | S | C-CH₃ | C-CH₃ | - |
| 338. | -(CH₂)₂-NH-C(=O)- | | -H | -H | rac | -H | -H | | -H | -H | 2 | | S | C-CH₃ | C-CH₃ | - |
| 339. | -(CH₂)₂-NH-C(=O)- | | -H | -H | rac | -H | -H | | -H | -H | 2 | | S | C-H | C-H | - |
| 340. | -(CH₂)₂-NH-C(=O)- | | -H | -H | rac | -H | -H | | -H | -H | 2 | | S | C-CH₃ | C-H | - |
| 341. | -NH₂ | -C=N(<E>-OH)-CH₃ | -H | -H | rac | -H | -H | | -H | -H | 2 | | S | C-CH₃ | C-H | - |
| 342. | -NH₂ | -C(=N(<Z>-OCH(CH₃)₂)-CH₃ | -H | -H | rac | -H | -H | | -H | -H | 2 | | S | C-CH₃ | C-H | - |
| 343. | -NH-(CH₂)₂-C(=O)- | | -H | -H | rac | -H | -H | | -H | -H | 2 | | S | C-CH₃ | C-H | - |
| 344. | -NH-CH₂-CH(C(=O)OCH₃)-C(=O)- | | -H | -H | rac | -H | -H | | -H | -H | 2 | | S | C-CH₃ | C-H | - |
| 345. | -N(CH₃)-(CH₂)₂-C(=O)- | | -H | -H | rac | -H | -H | | -H | -H | 2 | | S | C-CH₃ | C-H | - |
| 346. | -N(CH₃)-CH₂-CH(C(=O)OCH₃)-C(=O)- | | -H | -H | rac | -H | -H | | -H | -H | 2 | | S | C-CH₃ | C-H | - |
| 347. | -N(T19)-(CH₂)₂-C(=O)- | | -H | -H | rac | -H | -H | | -H | -H | 2 | | S | C-CH₃ | C-H | - |
| 348. | -N(T19)-CH₂-CH(C(=O)OCH₃)-C(=O)- | | -H | -H | rac | -H | -H | | -H | -H | 2 | | S | C-CH₃ | C-H | - |
| 349. | -CH₃ | -C(=N<Z>OH)CH₃ | -H | -H | R | -H | -H | | -H | -H | 2 | | S | C-Cl | C-H | - |
| 350. | -CH₃ | -C(=N<E>OH)CH₃ | -H | -H | R | -H | -H | | -H | -H | 2 | | S | C-Cl | C-H | - |
| 351. | -Cl | -CF₃ | -H | -H | rac | -H | -H | | -H | -H | 2 | | S | C-CH₃ | C-H | - |
| 352. | -NH₂ | -C(=N<E> CH₃)CH₃ | -H | -H | R | -H | -H | | -H | -H | 2 | | S | C-CH₃ | C-H | - |

| | | | | | | | | | | | | | | | | |
|---|---|---|---|---|---|---|---|---|---|---|---|---|---|---|---|---|
| T1: *2-methyl-4,5,6,7-tetrahydrobenzothiophen-4-yl* *T2: N2-5-(trifluoromethyl)pyrimidine-2,4-diamine* *T3: 2-ethyl-4,5,6,7-tetrahydrobenzothiophen-4-yl* *T4: 2-methyl-4,5,6,7-tetrahydrobenzothiophen-7-yl* *T5: (4R)-2-methyl-4,5,6,7-tetrahydrobenzothiophen-4-yl* *T6: (E)-pyrrolidin-1-ylmethyleneamino* *T7: (E)-1-piperidylmethyleneamino* *T8: 2,3-dimethyl-4,5,6,7-tetrahydrobenzothiophen-4-yl* *T9: cyclopentyl* *T10: 2-chlor-4,5,6,7-tetrahydrobenzothiophen-7-yl* *T11: 2-methyl-5,6-dihydro-4H-cyclopenta[b]thiophen-4-yl* *T12: 5-(trifluoromethyl)-pyrid-3-yl* *T13: 5-fluoro-pyrid-3-yl* *T14: 5-chloro-pyrid-3-yl* *T15: 4-(2-pyridyl)piperazine-1-yl* *T16: 4-(2-fluorophenyl)piperazine-1-yl* *T17: (1R,2S)-2,6-dimethylindan-1-yl* *T18: azepinyl* *T19: cyclopropyl* | | | | | | | | | | | | | | | | |

Weiterer Gegenstand der vorliegenden Erfindung sind Verfahren zur Herstellung entsprechender Verbindungen der allgemeinen Formel (I) und/oder deren Salze und/oder deren agrochemisch verträglichen quaternierten Stickstoff-Derivate:
a.) Zur Herstellung von Verbindungen der allgemeinen Formel (I) 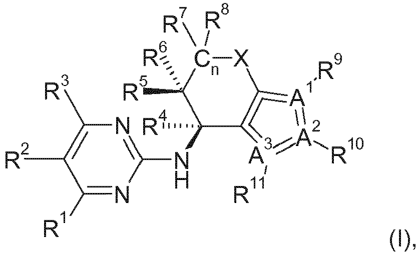 in welchen die Reste R¹ bis R¹¹, A¹ bis A³, n und X die vorstehenden Bedeutungen aufweisen, wird eine Verbindung der allgemeinen Formel (II) 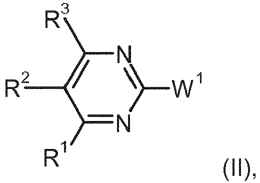
   worin R¹ bis R³ die vorstehende Bedeutung aufweisen und
   W¹ für einen austauschfähigen Rest oder eine Abgangsgruppe steht, mit einem Amin der allgemeinen Formel (III) oder einem Säureadditionssalz des Amins der allgemeinen Formel (III)

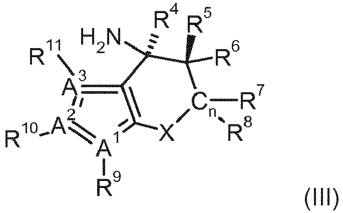

   umgesetzt, wobei die Reste R⁴ bis R¹¹, A¹ bis A³, n und X die vorstehende Bedeutung aufweisen.
   Der austauschfähige Rest W¹ bzw. die Abgangsgruppe W¹ steht für Fluor, Chlor, Brom, Jod, ein (C₁-C₄)-Alkylsulfanyl oder ein (C₁-C₄)-Alkylsulfinyl oder ein (C₁-C₄)-Alkylsulfonyl, ein unsubstituiertes oder ein substituiertes Phenyl-(C₁-C₄)-alkylsulfonyl oder ein (C₁-C₄)-Alkylphenyl-sulfonyl.
   Gegebenenfalls kann ein Rest W¹ in eine andere, besser austauschbare Gruppe überführt werden. So kann beispielsweise im Sinne eines Zweistufen-Eintopfverfahren ein (C₁-C₄)-Alkylsulfanyl mit einem Oxidationsmittel wie m-Chlorperbenzoesäure oder Oxone® in ein (C₁-C₄)-Alkylsulfinyl oder ein (C₁-C₄)-Alkylsulfonyl oder Mischungen davon überführt werden und anschließend mit einem Amin der allgemeinen Formel (III) oder einem Säureadditionssalz unter Verwendung einer Hilfsbase wie z.B. Triethylamin oder Kaliumcarbonat zur Reaktion gebracht werden.
   Gegebenenfalls kann die Umsetzung auch durch unterschiedliche Hilfsstoffe katalysiert werden, wie beispielswiese durch die Reagenzien Kaliumphosphat, Kupfer(I)iodid und N,N-diethyl-2-hydroxybenzamide oder im Sinne der Buchwald-Hartwig-Kupplung durch spezielle Übergangsmetallkatalysatorsysteme.
   Die Verbindungen der allgemeinen Formel (II) sind kommerziell verfügbar oder können nach bekannten Verfahren hergestellt werden.
   Die Amine der allgemeinen Formel (III) oder die Säureadditionssalz davon sind kommerziell verfügbar oder deren Synthese ist in DE 19921883 beschrieben.
b.) Verbindungen der allgemeinen Formel (I) können auch dadurch hergestellt werden, dass zunächst eine Verbindung der allgemeinen Formel (II-a), 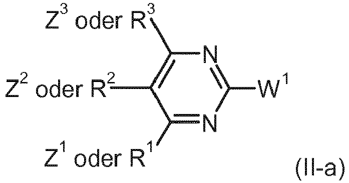 wobei mindestens eine der Reste R¹ bis R³ ein Rest Z¹ bis Z³, worin Z¹ bis Z³ ausgewählt ist aus COOH, COO(C₁-C₆)-Alkyl, Nitril, C₂-C₆-Alkinyl, Halogen, Acetyl, Carbonyl und (C₁-C₆)-Alkylmercapto ist, der verändert oder ausgetauscht werden kann, analog den unter a. beschriebenen Verfahren mit einem Amin der Formel (III) oder einem Säureadditionssalz davon in ein Intermediat der Formel (I-a) 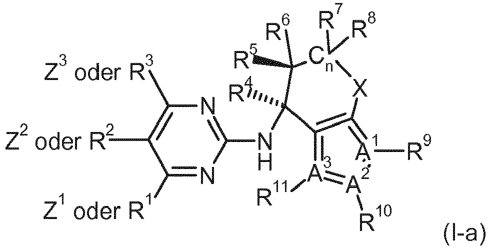
   überführt werden und dann zum Beispiel der Rest Z² in ein Keton überführt werden. So kann beispielsweise in dem Fall, dass Z² = (C₂-C₆)-Alkinyl ist, die (C₂-C₆)-Alkinyl-Gruppe in -C(=O)-CH₂-(C₁-C₄)-Alkinyl überführt werden oder wenn Z² = Trimethylsilylacetylene ist in Ethinyl überführt werden; beispielsweise kann der Rest Z² = COOH oder COO(C₁-C₆)-Alkyl nach in der Literatur bekannten Verfahren in C(=O)-R¹ überführt werden.
   Gegebenenfalls kann auch ein Rest Z² in einen zunächst anderen Rest Z² überführt werden. So kann beispielsweise nach den zuvor beschriebenen Verfahren zunächst ein Intermediat des Typs (I-a) hergestellt werden, in dem der Rest Z² ein Halogen ist und das Halogen nach literaturbekannten Verfahren in ein (C₂-C₆)-Alkinyl oder ein 1-(C₁-C₆)-Alkoxyl-(C₂-C₆)-Alkenyl überführt wird und dann der Rest Z² in eine C(=O)-R¹ umgewandelt wird.
   In dem Fall, dass Z¹ bis Z³ mindestens ein Keton ist, kann dieses beispielsweise mit Hydroxylamin oder substituierten Hydroxylaminen beziehungsweise aus den jeweiligen Salzformen der Regenzien in situ freigesetzen Komponenten zu den jeweiligen Zielprodukten umgesetzt werden.
   In dem Fall, dass Z¹ bis Z³ mindestens ein Halogen, bevorzugt Brom oder Jod ist, kann dieses mit zum Beispiel unter Palladium-Katalyse mit Alkenen oder Alkinen zu Zielstrukturen umgesetzt werden, die zum Beispiel C₂-C₆)-Alkinyle oder C₂-C₆)-Alkenyle als R¹ bis R³ tragen.
   In dem Fall, dass Z¹ bis Z³ mindestens ein Nitril ist, kann dieses beispielsweise mit Basen wie Natronlauge oder Kaliumhydroxyd zu Carbonsäureamiden R¹ bis R³ verseift werden.
   In dem Fall, dass Z¹ bis Z³ mindestens ein Carbonsäureester ist, kann dieses beispielsweise mit basen wie Natronlauge oder Kaliumhydroxyd zu Carbonsäuren R¹ bis R³ verseift werden.
   In dem Fall, dass Z¹ bis Z³ mindestens eine Carbonsäure ist, kann diese beispielsweise mit Aminen unter Verwendung von Hilfsreagenzien wie T3P zu substituierten Carbonsäureamiden R¹ bis R³ umgesetzt werden.
   In dem Fall, dass Z¹ bis Z³ mindestens eine Carbonsäure ist, kann diese in ein Carbonsäurechlorid überführt und dann mit Aminen zu substituierten Carbonsäureamiden R¹ bis R³ umgesetzt werden.
   In dem Fall, dass Z¹ und Z³ mindestens ein Halogenatom ist, kann dieses beispielsweise mit Aminen gegebenenfalls unter Verwendung von zusätzlichen Basen wie Triethylamin zu Aminen in der Position R¹ und R³ umgesetzt werden.
   In dem Fall, dass Z¹ und Z³ mindestens ein Halogenatom ist, kann dieses beispielsweise mit Alkoholen gegebenenfalls unter Verwendung von zusätzlichen Basen wie Natrium, Natriumhydrid oder dem Alkoholat des betreffenden Alkohols zu Alkoxyderivaten in der Position R¹ und R³ umgesetzt werden.
   In dem Fall, dass Z¹ und Z³ mindestens ein Halogenatom ist, kann dieses beispielsweise mit Thiolen gegebenenfalls unter Verwendung von zusätzlichen
   Basen wie Natrium, Natriumhydrid oder dem Thiolat des betreffenden Thiols zu Alkylthioderivaten in der Position R¹ und R³ umgesetzt werden.
   In dem Fall, dass Z¹ bis Z³ mindestens ein Schwefelatom enthält, kann dieses mit Oxidationsmitteln wie beispielsweise Oxone oder m-Chlorperbenzoesäure oxidiert werden.
   In dem Fall, dass Z¹ bis Z³ mindestens ein Halogenatom ist, bevorzugt Brom oder Jod ist, kann dieses beispielsweise palladiumkatalysiert mit Phenyl- oder Heterocyclylboronsäuren zu Phenyl oder Heterocyclylderivaten in der Position R¹ bis R³ umgesetzt werden.
c.) Verbindungen der allgemeinen Formel (I) können auch dadurch hergestellt werden, in dem Amidine des Typs (IV) oder Säureadditionssalze davon 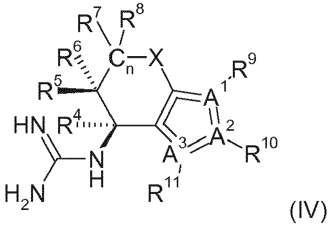 mit einem Keton der Formel (V) 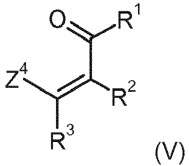 in der Rest Z⁴ für ein (C₁-C₆)-Alkoxy oder Di-(C₁-C₆)-Alkylamino steht, kondensiert werden.
d.) Verbindungen der allgemeinen Formel (I) können auch in einer Drei-Komponenten-Reaktion dadurch hergestellt werden, in dem man Amidine des Typs (IV) oder Säureadditionssalze 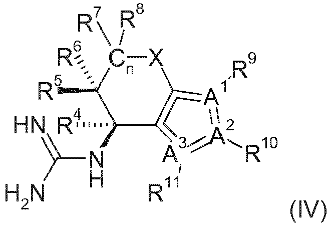 mit einem Keton der Formel (VI). 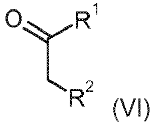 und mit einem Fragment (VII) in dem Z⁵ für ein (C₁-C₆)-Alkoxy oder Di-(C₁-C₆)-Alkylamino und Z⁶ für ein (C₁-C₆)-Alkoxy steht, 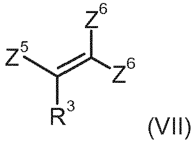 kondensiert.
e.) Verbindungen der allgemeinen Formel (II) in denen der Rest W¹ für (C₁-C₄)-Alkylsulfanyl steht können analog den unter c.) oder d.) beschrieben Verfahren hergestellt werden, wobei statt (IV) S-(C₁-C₄)-Alkylisothioharnstoffe oder deren Säureadditionssalze eingesetzt werden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Reaktionsstationen (reaction stations) der Firma Radleys, Shirehill, Saffron Walden, Essex, CB 11 3AZ, England oder MultiPROBE Automated Workstations der Firma Perkin Elmer, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma Teledyne ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und A. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Herbizide werden in landwirtschaftlich genutzten Kulturen während verschiedener Anbauphasen eingesetzt. So erfolgt die Applikation einiger Produkte schon vor oder während der Saat. Andere werden ausgebracht, bevor die Kulturpflanze aufläuft, d.h. bevor der Keimling die Erdoberfläche durchbricht (Vorauflauf-Herbizide). Nachauflauf-Herbizide schließlich werden verwendet, wenn von der Kulturpflanze entweder bereits die Keimblätter oder Laubblätter ausgebildet sind.

Die erfindungsgemäßen Verbindungen können dabei sowohl im Vorlauf als auch im Nachlauf angewendet werden, wobei eine Verwendung der erfindungsgemäßen Verbindungen im Vorlauf bevorzugt ist.

Die Vorauflauf-Behandlung schließt sowohl die Behandlung der Anbaufläche vor der Aussaat (ppi = pre plant incorporation) als auch die Behandlung der angesäten, aber noch nicht bewachsenen Anbauflächen ein.

Die erfindungsgemäßen Verbindungen der Formel (I) und deren Salze, im folgenden auch synonym zusammen auch als Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnem seien beispielhaft einige Vertreter der mono- und dikotylen Unkrautflora genannt, die durch die erfindungsgemäßen Verbindungen der allgemeinen Formel (I) kontrolliert werden können, ohne daß durch die Nennung eine Beschränkung auf bestimmte Arten erfolgen soll.

Auf der Seite der monokotylen Unkrautarten werden z. B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea, sowie Cyperusarten vorwiegend aus der annuellen Gruppe und auf Seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern. Außerdem wird herbizide Wirkung bei dikotylen Unkräutern wie Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium beobachtet.

Werden die erfindungsgemäßen Verbindungen der allgemeinen Formel (I) vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe der allgemeinen Formel (I) auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle, Raps und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen der allgemeinen Formel (I) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z. B. WO 92/011376, WO 92/014827, WO 91/019806),
- transgene Kulturpflanzen, welche gegen bestimmte Herbizide vom Typ Glufosinate (vgl. z. B. EP 0242236, EP 0242246) oder Glyphosate (WO 92/000377) oder der Sulfonylharnstoffe (EP 0257993, US 5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924, EP 0193259).
- transgene Kulturpflanzen mit modifizierter Fettsäurezusammensetzung (WO 91/013972).
- gentechnisch veränderte Kulturpflanzen mit neuen Inhalts- oder Sekundärstoffen z. B. neuen Phytoalexinen, die eine erhöhte Krankheitsresistenz verursachen (EP 0309862, EP 0464461)
- gentechnisch veränderte Pflanzen mit reduzierter Photorespiration, die höhere Erträge und höhere Stresstoleranz aufweisen (EP 0305398)
- transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) als Herbizide zur Bekämpfung von Schadpflanzen in transgenen Kulturpflanzen.

Die Verbindungen der allgemeinen Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösemittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z. B. Insektiziden, Akariziden, Herbiziden, Fungiziden, sowie mit Safenern, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-Dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösemittel z. B. Butanol, Cyclohexanon, Dimethylformamid, Xylol oder auch höhersiedenden Aromaten oder Kohlenwasserstoffen oder Mischungen der organischen Lösemittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Cadodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z. B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rühren, Kolloidmühlen und/oder statischen Mischern unter Verwendung von wäßrigen organischen Lösemitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z. B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z. B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe u.a. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösemittel, Füll-, Träger- und Farbstoffe, Entschäumer, Verdunstungshemmer und den pH-Wert und die Viskosität beeinflussende Mittel.

Die Verbindungen der allgemeinen Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der allgemeinen Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

Die vorliegende Erfindung wird ahand der nachfolgenden Beispiele näher erläutert, welche die Erfindung jedoch keinesfalls beschränken.

### A. Synthesebeispiele

### 5-fluoro-N2-(2-methyl-4,5,6,7-tetrahydro-1-benzothiophen-4-yl)pyrimidine-2,4-diamine (Bsp.: 12):

0.15 g (1.01 mmol) 2-chloro-5-fluoro-pyrimidin-4-amine, 0.249 g (1.22 mmol) (2-methyl-4,5,6,7-tetrahydrobenzothiophen-4-yl)ammonium chloride und 0.63 g (0.68 ml, 3.05 mol) Dicyclohexylethylamin in 2.0 ml 1-Methyl-2-pyrrolidon werden in einer geschlossenen Küvette in der Mikrowelle 90 Minuten auf 180 °C (Biotage Initiator, http://www.biotage.com/product-page/biotage-initiator) erhitzt. Das so erhaltene Rohgemisch wird auf Kieselgel aufgezogen und säulenchromatographisch mit Heptan/Essigester als Laufmittel gereinigt. Nach Einengen werden 0.072 g 5-fluoro-N2-(2-methyl-4,5,6,7-tetrahydro-1-benzothiophen-4-yl)pyrimidine-2,4-diamine (wachsartig) erhalten (Ausbeute 22 % bei 85 % Reinheit).

### 5-(trifluoromethyl)-N2-(2,6,6-trimethyl-4,5,6,7-tetrahydro-1-benzofuran-4-yl)pyrimidine-2,4-diamine (Bsp.: 21):

0.25 g eines ca 1:1 Gemisches aus 2-chloro-5-(trifluoromethyl)pyrimidin-4-amine und 4-chloro-5-(trifluoromethyl)pyrimidin-2-amine, 0.295 g (1.22 mmol) 2,6,6-trimethyl-4,5,6,7-tetrahydro-1-benzofuran-4-amine und 0.53 g (0.58 ml, 2.53 mol) Dicyclohexylethylamin in 2.0 ml 1-Methyl-2-pyrrolidon werden in einer geschlossenen Küvette in der Mikrowelle 150 Minuten auf 180 °C (Biotage Initiator, http://www.biotage.com/product-page/biotage-initiator) erhitzt. Das so erhaltene Rohgemisch wird auf Kieselgel aufgezogen und säulenchromatographisch mit Heptan/Essigester als Laufmittel getrennt und gereinigt. Nach Einengen werden 0.326 g 5-(trifluoromethyl)-N2-(2,6,6-trimethyl-4,5,6,7-tetrahydro-1-benzofuran-4-yl)pyrimidine-2,4-diamine (fest, 90 % Reinheit) und als zweite Fraktion 5-(trifluoromethyl)-N4-(2,6,6-trimethyl-4,5,6,7-tetrahydro-1-benzofuran-4-yl)pyrimidine-2,4-diamine (fest, Schmelzpunkt 182.6 °C, 90 % Reinheit) erhalten.

### N2-(5,6-dihydro-4H-cyclopenta[b]thiophen-6-yl)-5-methylsulfonyl-pyrimidine-2,4-diamine (Bsp.: 33):

Unter Rühren werden zu einer auf 0 - 5 ° C gekühlten Lösung aus 0.10 g 2-methylsulfanyl-5-methylsulfonyl-pyrimidin-4-amine (0.43 mmol) in 3 ml trichlormethan 0.205 g (0.866 mmol) 73 %ige meta-Chlorperbenzoesaeure geben. Das Gemisch wird ca 2 Stunden gerührt, dann werden 0.32 g (0.45 ml, 3.03 mmol) trietahylamin und anschließend 0.08 g (0.46 mmol) 5,6-dihydro-4H-cyclopenta[b]thiophen-6-ylammonium chloride hinzugefügt. Das Reaktionsgemisch wird unter Rühren 180 min auf leichten Rückfluss erhitzt und über Nacht stenen gelassen. Das so erhaltene Rohgemisch wird auf Kieselgel aufgezogen und säulenchromatographisch mit Heptan/Essigester als Laufmittel getrennt und gereinigt. Nach Einengen werden 0.087 g N2-(5,6-dihydro-4H-cyclopenta[b]thiophen-6-yl)-5-methylsulfonyl-pyrimidine-2,4-diamine (fest, Schmelzpunkt 175.9 °C; 62 % Ausbeute bei 95 % Reinheit) erhalten.

### 5-propylsulfonyl-N2-(4,5,6,7-tetrahydrobenzothiophen-4-yl)pyrimidine-2,4-diamine (Bsp.: 37):

1.00 g (6.80 mmol) 2-propylsulfonylacetonitrile und 0.97 g (1.08 ml, 8.15 mmol) N,N-Dimethylformamiddimethylacetal werden in 5 ml Methanol zwei Stunden gerührt. Das Reaktionsgemisch wird dann am Rotationsverdampfer eingeengt. Der erhaltene Feststoff wird in wenig Heptan aufgenommen und abgesaugt. Man erhält 1.05 g 3-(dimethylamino)-2-propylsulfonyl-prop-2-enenitrile (fest, Schmelzpunkt 123.6 °C, Ausbeute 73 % bei 95 % Reinheit).

0.16 g (0.79 mmol) 3-(dimethylamino)-2-propylsulfonyl-prop-2-enenitrile, 0.20 g (0.86 mmol) 1-(4,5,6,7-tetrahydro-1-benzothiophen-4-yl)guanidine und 0.214 g (0.218 ml, 1.18 mmol) Natriummethanolat als 30 %ige Lösung in Methanol werden in 5 ml Methanol eine Stunde zum Rückfluß erhitzt. Das Reaktionsgemisch wird auf Kieselgel aufgezogen und säulenchromatographisch mit Heptan/Essigester als Laufmittel getrennt. Nach Einengen werden 0.216 g 5-propylsulfonyl-N2-(4,5,6,7-tetrahydrobenzothiophen-4-yl)pyrimidine-2,4-diamine (fest, Schmelzpunkt 189.9 °C) erhalten (Ausbeute 74 % bei 95 % Reinheit).

### 5-nitro-N2-(2,6,6-trimethyl-4,5,6,7-tetrahydro-1-benzofuran-4-yl)pyrimidine-2,4-diamine (Bsp.: 40):

0.15 g (0.85 mmol) 2-chloro-5-nitro-pyrimidin-4-amine, 0.185 g (0.82 mmol) 2,6,6-trimethyl-4,5,6,7-tetrahydro-1-benzofuran-4-amine und 0.174 g (0.24 ml, 1.71 mol) triethylamin in 1.5 ml N,N-Dimethylacetamid werden in einer geschlossenen Küvette in der Mikrowelle 45 Minuten auf 120 °C (Biotage Initiator, http://www.biotage.com/product-page/biotage-initiator) erhitzt. Das so erhaltene Rohgemisch wird auf Kieselgel aufgezogen und säulenchromatographisch mit Heptan/Essigester als Laufmittel gereinigt. Nach Einengen werden 0.095 g 5-nitro-N2-(2,6,6-trimethyl-4,5,6,7-tetrahydro-1-benzofuran-4-yl)pyrimidine-2,4-diamine (fest) erhalten (Ausbeute 28 % bei 85 % Reinheit).

### N2-[(4R)-2-methyl-4,5,6,7-tetrahydrobenzothiophen-4-yl]-5-(trifluoromethyl)pyrimidine-2,4-diamine (Bsp.: 47):

0.75 g (3.48 mmol) 2,4-dichloro-5-(trifluoromethyl)pyrimidine werden in 10 ml Tetrahydrofuran vorgelegt und auf 0 °C gekühlt, dann wird 1.036 g Zinkchlorid (10.86 ml einer 0.7 mol Lösung in Tetrahydrofuran, 7.60 mol) zugetropft und eine Stunde gerührt. Nachfolgend werden 0.81 g (3.97 mmol) [(4R)-2-methyl-4,5,6,7-tetrahydrobenzothiophen-4-yl]ammonium chloride zugegeben und dann werden 0.84 g (1.156 ml, 8.27 mmol) triethylamin zugetropft. Die Reaktionsmischung wird zwei Stunden gerührt und auf Raumtemperatur kommen gelassen. Nach stehen lassen über Nacht wird das Reaktionsgemisch auf Kieselgel aufgezogen. Die auf Kieselgel aufgezogene Reaktionsmischung wird auf eine mit Kieselgel (ca 1 cm hohe Schicht) bedeckten Fritte aufgetragen und das Rohprodukt wird mit Essigester herausgewaschen. Nach Einengen der organischen Phase erhält man 1.23 g 4-chloro-N-[(4R)-2-methyl-4,5,6,7-tetrahydrobenzothiophen-4-yl]-5-(trifluoromethyl)pyrimidin-2-amine (ca 70 %). Dieses Gemsich wird ohne weitere Reinigung in die Folgestufe eingesetzt.

1.23 g eines Gemisches aus 4-chloro-N-[(4R)-2-methyl-4,5,6,7-tetrahydrobenzothiophen-4-yl]-5-(trifluoromethyl)pyrimidin-2-amine (ca 70 %) und 2-chloro-N-[(4R)-2-methyl-4,5,6,7-tetrahydrobenzothiophen-4-yl]-5-(trifluoromethyl)pyrimidin-4-amine (ca 20 %) werden in 3.5 ml Ammoniak in Methanol (ca 12 % Lösung, ca 3.6 mmol) in einer geschlossenen Küvette in der Mikrowelle 90 Minuten auf 110 °C (Biotage Initiator, http://www.biotage.com/product-page/biotageinitiator) erhitzt. <Der am Gerät angezeigte Druck im Vial betrug nach dem Aufheitzen ca 8 bar.). Nach Abühlen und Entspannen des Druckes wird das so erhaltene Rohgemisch wird auf Kieselgel aufgezogen und säulenchromatographisch mit Heptan/Essigester als Laufmittel getrennt und gereinigt. Nach Einengen werden 0.721 g N2-[(4R)-2-methyl-4,5,6,7-tetrahydrobenzothiophen-4-yl]-5-(trifluoromethyl)pyrimidine-2,4-diamine (95 % Reinheit) und als zweite Fraktion N4-[(4R)-2-methyl-4,5,6,7-tetrahydrobenzothiophen-4-yl]-5-(trifluoromethyl)pyrimidine-2,4-diamine (95 % Reinheit) erhalten.

### N-(1,3-dimethyl-4,5,6,7-tetrahydro-2-benzothiophen-4-yl)-5,5-dioxo-7,8-dihydro-6H-thiopyrano[3,2-d]pyrimidin-2-amine (Bsp.: 68):

2.5 g (16.8 mmol) 1,1-dioxothian-3-one und 2.0 g (16.8 mmol) N,N-Dimethylformamiddimethylacetal werden in 7 ml N,N-Dimethylacetamid vorgelegt und 30 Minuten gerührt, dann werden 2.59 g (9,27 mmol) 2-Methyl-2-thiopseudourea-sulfat und 1.88 g (2.59 ml, 18.5 mmol) triethylamin zugefügt und das Gemisch in einer geschlossenen Küvette in der Mikrowelle 40 Minuten auf 160 °C (Biotage Initiator, http://www.biotage.com/product-page/biotage-initiator) erhitzt. Das so erhaltene Rohgemisch wird auf Kieselgel aufgezogen und säulenchromatographisch mit Heptan/Essigester als Laufmittel gereinigt. Nach Einengen werden 2.04 g 2-methylsulfanyl-7,8-dihydro-6H-thiopyrano[3,2-d]pyrimidine 5,5-dioxide (fest) (Ausbeute 51 % bei 95 % Reinheit) erhalten.

Unter Rühren werden zu einer auf 0 - 5 ° C gekühlten Lösung aus 0.15 g 2-methylsulfanyl-7,8-dihydro-6H-thiopyrano[3,2-d]pyrimidine 5,5-dioxide (0.62 mmol) in 5 ml Trichlormethan 0.293 g (1.24 mmol) 73 %ige meta-Chlorperbenzoesaeure geben. Das Gemisch wird ca 2 Stunden gerührt, dann werden 0.33 g (0.45 ml, 3.10 mmol) triethylamin und anschließend 0.13 g (0.71 mmol) 1,3-dimethyl-4,5,6,7-tetrahydro-2-benzothiophen-4-amine hinzugefügt. Das Reaktionsgemisch wird unter Rühren 180 min auf leichten Rückfluss erhitzt und über Nacht stenen gelassen. Das so erhaltene Rohgemisch wird auf Kieselgel aufgezogen und säulenchromatographisch mit Heptan/Essigester als Laufmittel getrennt und gereinigt. Nach Einengen werden 0.044 g N-(1,3-dimethyl-4,5,6,7-tetrahydro-2-benzothiophen-4-yl)-5,5-dioxo-7,8-dihydro-6H-thiopyrano[3,2-d]pyrimidin-2-amine (fest; 19 % bei 95 % Reinheit) erhalten.

### N2-(2,3-dimethyl-4,5,6,7-tetrahydrobenzothiophen-4-yl)-5-(trifluoromethyl)pyrimidine-2,4-diamine (Bsp.: 213):

0.40 g (1.84 mmol) 2,4-dichloro-5-(trifluoromethyl)pyrimidine werden in 10 ml Tetrahydrofuran vorgelegt und auf 0 °C gekühlt, dann wird 0.55 g Zinkchlorid (5.80 ml einer 0.7 mol Lösung in Tetrahydrofuran, 4.07 mol) zugetropft und eine Stunde gerührt. Nachfolgend werden 0.44 g (2.03 mmol) (2,3-dimethyl-4,5,6,7-tetrahydrobenzothiophen-4-yl)ammonium chloride zugegeben und dann werden 0.45 g (0.62 ml, 4.42 mmol) triethylamin zugetropft. Die Reaktionsmischung wird zwei Stunden gerührt und auf Raumtemperatur kommen gelassen. Nach stehen lassen über Nacht wird das Reaktionsgemisch auf Kieselgel aufgezogen. Die auf Kieselgel aufgezogene Reaktionsmischung wird auf eine mit Kieselgel (ca 1 cm hohe Schicht) bedeckten Fritte aufgetragen und das Rohprodukt wird mit Essigester herausgewaschen. Nach Einengen der organischen Phase wird das Rohgemisch in ein für Mikrowelle geeignetes Vial überführt und 5 ml Ammoniak in Methanol (ca 12 % Lösung, ca 6 mmol) hinzugefügt. In einer geschlossenen Küvette wird das Gemisch in der Mikrowelle 90 Minuten auf 110 °C (Biotage Initiator, http://www.biotage.com/product-page/biotage-initiator) erhitzt. <Der am Gerät angezeigte Druck im Vial betrug nach dem Aufheitzen ca 9bar.). Nach Abühlen und Entspannen des Druckes wird das so erhaltene Rohgemisch wird auf Kieselgel aufgezogen und säulenchromatographisch mit Heptan/Essigester als Laufmittel getrennt und gereinigt. Nach Einengen werden 0.261 g N2-(2,3-dimethyl-4,5,6,7-tetrahydrobenzothiophen-4-yl)-5-(trifluoromethyl)pyrimidine-2,4-diamine (fest, Schmelzpunkt 190.7 °C, 95 % Reinheit) und als zweite Fraktion 0.078 g N4-(2,3-dimethyl-4,5,6,7-tetrahydrobenzothiophen-4-yl)-5-(trifluoromethyl)pyrimidine-2,4-diamine (fest, 95 % Reinheit) erhalten.

**Tabelle 2 (Chemisch Physikalische Charakterisierung ausgewählter Synthesebeispiele)**

| Tabelle 2 Verbindung | Beschreibung |
|---|---|
| 2 | fest; Smp.: 198.7 °C; logp (HCOOH): 2.53; 1H-NMR (CDCl₃, 400 MHZ, δ in ppm): 1.75 (m, 3H, 3H von CH₂); 2.05 (m, 1H, 1H von CH₂); 2.25 (s, 6H, 2*3H von thiophen-CH₃); 2.45 (m, 1H, 1H von CH₂); 2.65 (m, 1H, 1H von CH₂); 4.90 (m, 1H, 1H von CH); 5.15 (br, 2H, NH₂); 5.75 (br, 1H, 1H von NH); 7.90 und 8.25 (2*br, 1H, Pyr-6H); |
| 4 | fest; Smp.: 190.9 °C; logp (HCOOH): 1.73; 1H-NMR (CDCl₃, 400 MHZ, δ in ppm): 2.20 (m, 1H, 1H von CH₂); 2.45 (s, 3H, 3H von thiophen-CH₃); 2.80 - 3.00 (m, 3H, 3H von CH₂); 3.05 (s, 3H, 3H von SO₂-CH₃); 5.30 (m, 1H, 1H von CH); 5.50 (br, 2H, NH₂); 5.90 (br, 1H, 1H von NH); 6.55 (s, 1H, thiophen-H); 8.25 und 8.50 (2*br, 1H, Pyr-6H); |
| 5 | fest; logp (HCOOH): 5.72; 1H-NMR (CDCl₃, 400 MHZ, δ in ppm): 1.85 (m, 6H, 6H von CH₂); 2.10 (m, 2H, 2H von CH₂); 2.40 (s, 6H, 2*3H von thiophen-CH₃); 2.60 - 2.80 (m, 4H, 4H von CH₂); 5.00 - 5.40 (m, 3H, 2H von CH, 1H von NH); 6.50 (br, 1H, 1H von NH); 6.50 (s, 1H, thiophen-H); 7.95 und 8.20 (2*br, 1H, Pyr-6H); |
| 6 | fest; Smp.: 212.4 °C; logp (HCOOH): 2.44; 1H-NMR (CDCl₃, 400 MHZ, δ in ppm): 1.75 (m, 3H, 3H von CH₂); 2.05 (m, 1H, 1H von CH₂); 2.25 (s, 6H, 2*3H von thiophen-CH₃); 2.40 (m, 1H, 1H von CH₂); 2.65 (m, 1H, 1H von CH₂); 3.05 (s, 3H, 3H von SO₂-CH₃); 5.10 (m, 1H, 1H von CH); 5.15 (br, 2H, 2H von NH₂); 5.75 (br, 1H, 1H von NH); 8.20 und 8.50 (2*br, 1H, Pyr-6H); |
| 7 | Oelig |
| 8 | oelig; logp (HCOOH): 3.19; 1H-NMR (CDCl₃, 400 MHZ, δ in ppm): 1.85 (m, 3H, 3H von CH₂); 2.05 (m, 3H, 3H von CH₂); 2.40 (s, 3H, 3H von CH₃); 2.60 (m, 4H, 4H von CH₂); 2.80 (m, 1H, 1H von CH₂); 2.90 (m, 1H, 1H von CH₂); 5.40 (m, 1H, 1H von CH); 5.85 (br, 1H, 1H von NH); 6.45 (s, 1H, thiophen-H);); 8.80 und 8.95 (2*br, 1H, Pyr-6H); |
| 9 | oelig; logp (HCOOH): 3.23; 1H-NMR (CDCl₃, 400 MHZ, δ in ppm): 1.85 (m, 3H, 3H von CH₂); 2.10 (m, 3H, 3H von CH₂); 2.40 (s, 3H, 3H von CH₃); 2.60 (m, 2H, 2H von CH₂); 2.75 (m, 3H, 3H von CH₂); 2.90 (m, 1H, 1H von CH₂); 5.25 (m, 1H, 1H von CH); 5.85 (br, 1H, 1H von NH); 6.50 (s, 1H, thiophen-H);); 8.80 und 8.95 (2*br, 1H, Pyr-6H); |
| 10 | oelig; logp (HCOOH): 4.76; 1H-NMR (CDCl₃, 400 MHZ, δ in ppm): 1.85 (m, 3H, 3H von CH₂); 2.10 (m, 1H, 1H von CH₂); 2.40 (s, 3H, 3H von thiophen-CH₃); 2.60 - 2.80 (m, 2H, 2H von CH₂); 3.90 und 4.00 (2*br, 3H, OCH₃); 5.00 - 5.40 (m, 1H, 1H von CH); 6.00 (br, 1H, 1H von NH); 6.50 (s, 1H, thiophen-H); 8.05 und 8.30 (2*br, 1H, Pyr-6H); |
| 11 | fest; Smp.: 134.4 °C; logp (HCOOH): 2,20; 1H-NMR (CDCl₃, 400 MHZ, δ in ppm): 1.85 (m, 3H, 3H von CH₂); 2.10 (m, 1H, 1H von CH₂); 2.40 (s, 3H, 3H von thiophen-CH₃); 2.60 - 2.80 (m, 2H, 2H von CH₂); 4.80 - 5.50 (m, 4H, 1H von CH, 1H von NH; 2H von NH₂); 6.50 (s, 1H, thiophen-H); 8.05 und 8.20 (2*br, 1H, Pyr-6H); |
| 12 | oelig; logp (HCOOH): 1.07; 1H-NMR (CDCl₃, 400 MHZ, δ in ppm): 1.85 (m, 3H, 3H von CH₂); 2.10 (m, 1H, 1H von CH₂); 2.40 (s, 3H, 3H von thiophen-CH₃); 2.60 - 2.80 (m, 2H, 2H von CH₂); 4.80 - 5.50 (br, 4H, 1H von CH, 1H von NH; 2H von NH₂); 6.50 (s, 1H, thiophen-H); 7.80 und 8.00 (2*br, 1H, Pyr-6H); |
| 13 | fest; Smp.: 222.1 °C; logp (HCOOH): 1.53; 1H-NMR (CDCl₃, 400 MHZ, δ in ppm): 1.80 (m, 1H, 1H von CH₂); 1.90 (m, 2H, 2H von CH₂); 2.05 (m, 1H, 1H von CH₂); 2.60 (m, 2H, 2H von CH₂); 3.05 (s, 3H, 3H von SO₂-CH₃); 5.00, 5.15, 5.40, 5.65 (jeweils br, 4H, 1H von CH, 1H von NH, 2H, NH₂); 6.30 (s, 1H, furan-H); 8.30 und 8.50 (2*br, 1H, Pyr-6H); |
| 15 | oelig; logp (HCOOH): 2.36; 1H-NMR (CDCl₃, 400 MHZ, δ in ppm): 1.05 (s, 3H, 3H von CH₃); 1.10 (s, 3H, 3H von CH₃); 1.35 (m, 1H, 1H von CH₂); 1.95 (m, 1H, 1H von CH₂); 2.20 (s, 3H, 3H von furan-CH₃); 2.40 (dd, 2H, 2H von CH₂); 3.00 (s, 3H, 3H von SO₂-CH₃); 5.00, 5.15, 5.20, 5.45 (jeweils br, 4H, 1H von CH, 1H von NH, 2H, NH₂); 5.90 (s, 1H, furan-H); 8.30 und 8.50 (2*br, 1H, Pyr-6H); |
| 16 | oelig; logp (HCOOH): 3.12; 1H-NMR (CDCl₃, 400 MHZ, δ in ppm): 1.75 (m, 1H, 1H von CH₂); 1.85 (m, 2H, 2H von CH₂); 2.05 (m, 1H, 1H von CH₂); 2.40 (s, 3H, 3H von CH₃); 2.75 (m, 2H, 2H von CH₂); 3.30 (s, 3H, 3H von SO₂CH₃); 5.00 bis 5.60 und 7.80 - 8.10 (jeweils br, 4H, 1H von CH, 1H von NH; 2H von NH₂; 1H); 6.50 (s, 1H, thiophen-H); |
| 17 | oelig; logp (HCOOH): 2.11; 1H-NMR (CDCl₃, 400 MHZ, δ in ppm): 1.30 (d, 3H, 3H von CH₃); 2.40 (s, 3H, 3H von thiophen-CH₃); 2.45 (m, 1H, 1H von CH₂); 2.60 (m, 1H, 1H von CH₂); 3.05 (s, 3H, 3H von SO₂-CH₃); 3.15 (m, 1H, 1H von CH₂); 4.90, 5.05, 5.50, 5.85 (jeweils br, 4H, 1H von CH, 1H von NH, 2H, NH₂); 6.50 (s, 1H, thiophen-H); 8.25 und 8.45 (2*br, 1H, Pyr-6H); |
| 19 | oelig; logp (HCOOH): 2.21; 1H-NMR (CDCl₃, 400 MHZ, δ in ppm): 1.85 (m, 3H, 1H von CH₂); 2.10 (m, 1H, 1H von CH₂); 2.40 (s, 3H, 3H von thiophen-CH₃); 2.55 (m, 2H, 2H von CH₂); 3.05 (s, 3H, 3H von SO₂-CH₃); 5.15, 5.30, 5.70, 6.05 (jeweils br, 4H, 1H von CH, 1H von NH, 2H, NH₂); 6.40 (s, 1H, thiophen-H); 8.25 und 8.45 (2*br, 1H, Pyr-6H); |
| 21 | fest; logp (HCOOH): 2.23; 1H-NMR (CDCl₃, 400 MHZ, δ in ppm): 1.05 (s, 3H, 3H von CH₃); 1.10 (s, 3H, 3H von CH₃); 1.35 (m, 1H, 1H von CH₂); 1.95 (m, 1H, 1H von CH₂); 2.20 (s, 3H, 3H von furan-CHs); 2.35 (dd, 2H, 2H von CH₂); 3.00 (s, 3H, 3H von SO₂-CH₃); 4.9 - 5.30 (br, 4H, 1H von CH, 1H von NH, 2H, NH₂); 5.85 (s, 1H, furan-H); 8.05-8.25 (br, 1H, Pyr-6H); |
| 22 | oelig; logp (HCOOH): 1.07; 1H-NMR (CDCl₃, 400 MHZ, δ in ppm): 1.75 (m, 1H, 1H von CH₂); 1.85 (m, 2H, 2H von CH₂); 2.10 (m, 1H, 1H von CH₂); 2.65 (m, 2H, 2H von CH₂); 5.05 und 5.60 (br, 4H, 1H von CH, 1H von NH; 2H von NH₂); 6.70 (s, 1H, thiophen-H); 8.00-8.25 (br, 1H, Pyr-6H); |
| 23 | fest; Smp.: 196.2 °C; logp (HCOOH): 1.82; 1H-NMR (CDCl₃, 400 MHZ, δ in ppm): 1.85 (m, 3H, 3H von CH₂); 2.10 (m, 1H, 1H von CH₂); 2.85 (m, 2H, 2H von CH₂); 3.00 (s, 3H, 3H von SO₂-CH₃); 5.15, 5,30, 5.50, 5.85 (jeweils br, 4H, 1H von CH, 1H von NH; 2H von NH₂); 6.85 (d, 1H, thiophen-H); 7.05 (d, 1H, thiophen-H); 8.25 und 8.50 (br, 1H, Pyr-6H); |
| 24 | fest; logp (HCOOH): 1.82; 1H-NMR (CDCl₃, 400 MHZ, δ in ppm): 1.85 (m, 3H, 3H von CH₂); 2.10 (m, 1H, 1H von CH₂); 2.85 (m, 2H, 2H von CH₂); 3.00 (s, 3H, 3H von SO₂-CH₃); 5.15, 5,30, 5.50, 5.65 (jeweils br, 4H, 1H von CH, 1H von NH; 2H von NH₂); 6.85 (d, 1H, thiophen-H); 7.05 (d, 1H, thiophen-H); 8.30 und 8.50 (br, 1H, Pyr-6H); |
| 27 | oelig; logp (HCOOH): 1.95; 1H-NMR (CDCl₃, 400 MHZ, δ in ppm): 1.85 (m, 3H, 3H von CH₂); 2.10 (m, 1H, 1Hvon CH₂); 2.85 (m, 2H, 2H von CH₂); 3.10 (s, 1H, 1H von SO-NH); 5.15, 5,30, 5.50, 5.65 (jeweils br, 4H, 1H von CH, 1H von NH; 2H von NH₂); 6.85 (d, 1H, thiophen-H); 7.05 (d, 1H, thiophen-H); 7.50 (m, 3H, 3H von Ar-H); 8.00 (m, 2H, 2H von Ar-H); 8.40 und 8.60 (br, 1H, Pyr-6H); |
| 28 | Oelig |
| 29 | oelig; logp (HCOOH): 1.73; 1H-NMR (CDCl₃, 400 MHZ, δ in ppm): 2.20 (m, 1H, 1H von CH₂); 2.70 (m, 1H, 1H von CH₂); 2.85 - 3.05 (m, 2H, 2H von CH₂); 4.90 - 5.70 (2*br, 4H, 1H von CH; 1H von NH; 2H von NH₂); 6.8 (d, 1H, thiophen-H); 8.15 (br, 1H, Pyr-6H); |
| 30 | oelig; logp (HCOOH): 4.27; 1H-NMR (CDCl₃, 400 MHZ, δ in ppm): 1.85 (m, 3H, 3H von CH₂); 2.05 (m, 1H, 1H von CH₂); 2.40 (s, 3H, 3H von CH₃); 2.75 (m, 2H, 2H von CH₂); 5.00 bis 5.60 und 7.80 bis 8.10 (jeweils br, 4H, 1H von CH, 1H von NH; 2H von NH₂; 1H); 6.55 (s, 1H, thiophen-H); 8.70 und 8.90 (2*br, 1H, Pyr-6H); |
| 32 | fest; Smp.: 189.5 °C; logp (HCOOH): 1.53; |
| 33 | fest; Smp.: 175.9 °C; logp (HCOOH): 1.57; 1H-NMR (CDCl₃, 400 MHZ, δ in ppm): 2.30 (m, 1H, 1H von CH₂); 2.70 (m, 1H, 1H von CH₂); 2.85 - 3.00 (m, 2H, 2H von CH₂); 3.05 (s, 3H, 3H von SO₂-CH₃); 5.40 - 5.85 (jeweils br, 4H, 1H von CH, 1H von NH; 2H von NH₂); 6.70 (d, 1H, thiophen-H); 7.25 (d, 1H, thiophen-H); 8.30 und 8.50 (2*br, 1H, Pyr-6H); |
| 35 | oelig; logp (HCOOH): 3.44; 1H-NMR (CDCl₃, 400 MHZ, δ in ppm): 1.85 (m, 3H, 3H von CH₂); 2.05 (m, 1H, 1H von CH₂); 2.40 (s, 3H, 3H von CH₃); 2.75 (m, 2H, 2H von CH₂); 2.95 (s, 3H, 3H von SOCH₃); 5.00 bis 5.60 und 7.80 - 8.10 (jeweils br, 4H, 1H von CH, 1H von NH; 2H von NH₂; 1H); 6.55 (s, 1H, thiophen-H); |
| 36 | fest; Smp.: 243.3 °C; logp (HCOOH): 2.12; 1H-NMR (CDCl₃, 400 MHZ, δ in ppm): 1.00 (m, 2H, 2H von cyclopropyl); 1.25 (m, 2H, 2H von cyclopropyl); 1.85 (m, 3H, 3H von CH₂); 2.10 (m, 1H, 1H von CH₂); 2.55 (m, 1H, 1H von cyclopropyl); 2.85 (m, 2H, 2H von CH₂); 5.15, 5,30, 5.50, 5.65 (jeweils br, 4H, 1H von CH, 1H von NH; 2H von NH₂); 6.90 (d, 1H, thiophen-H); 7.05 (d, 1H, thiophen-H); 8.20 und 8.40 (br, 1H, Pyr-6H); |
| 37 | fest; Smp.: 189.9 °C; logp (HCOOH): 2.33; 1H-NMR (CDCl₃, 400 MHZ, δ in ppm): 1.00 (t, 3H, 3H von CH₃); 1.70 - 1.95 (m, 5H, 3H von CH₂, 2H von CH₂); 2.10 (m, 1H, 1H von CH₂); 2.85 (m, 2H, 2H von CH₂); 3.05 (m, 2H, 2H von CH₂); 5.15, 5,20, 5.50, 5.85 (jeweils br, 4H, 1H von CH, 1H von NH; 2H von NH₂); 6.85 (d, 1H, thiophen-H); 7.05 (d, 1H, thiophen-H); 8.20 und 8.40 (br, 1H, Pyr-6H); |
| 38 | oelig; logp (HCOOH): 2.54; 1H-NMR (CDCl₃, 400 MHZ, δ in ppm): 2.20 (m, 1H, 1H von CH₂); 2.45 (s, 3H, 3H von CH₃); 2.70 - 3.00 (m, 3H, 3H von CH₂); 4.10 (m, 2H, 2H von CH₂-Ph); 4.85 (m, 1H, 1H von CH) 5.20 - 5.85 (jeweils br, 4H, 1H von NH; 1H von NH; 2H von NH₂); 6.55 (s, 1H, thiophen-H); 7.00 und 7.25( jeweils m, jeweils 2 H, jeweils 2 H von Ph); 8.20 und 8.45 (2*br, 1H, Pyr-6H); |
| 39 | oelig; logp (HCOOH): 3.00; 1H-NMR (CDCl₃, 400 MHZ, δ in ppm): 1.75 (m, 1H, 1H von CH₂); 1.85 (m, 2H, 2H von CH₂); 2.05 (m, 1H, 1H von CH₂); 2.40 (s, 3H, 3H von CH₃); 2.75 (m, 2H, 2H von CH₂); 2.90 (s, 3H, 3H von SOCH₃); 5.00 bis 5.60 und 7.80 - 8.10 (jeweils br, 4H, 1H von CH, 1H von NH; 2H von NH₂; 1H); 6.50 (s, 1H, thiophen-H); |
| 40 | fest; logp (HCOOH): 3.20; |
| 42 | oelig; logp (HCOOH): 2.33; 1H-NMR (CDCl₃, 400 MHZ, δ in ppm): 1.80 (m, 1H, 1H von CH₂); 1.90 (m, 2H, 2H von CH₂); 2.10 (m, 1H, 1H von CH₂); 2.65 (m, 2H, 2H von CH₂); 3.05 (s, 3H, 3H von SO₂-CH₃); 5.05, 5.15, 5.40 und 5.70 (jeweils br, 4H, 1H von CH, 1H von NH; 2H von NH₂); 6.70 (s, 1H, thiophen-H); 8.30 und 8.45 (2*br, 1H, Pyr-6H); |
| 44 | oelig; logp (HCOOH): 2.94; 1H-NMR (CDCl₃, 400 MHZ, δ in ppm): 1.80 (m, 1H, 1H von CH₂); 1.90 (m, 2H, 2H von CH₂); 2.10 (m, 1H, 1H von CH₂); 2.40 (s, 3H, 3H von CH₃); 2.45 (m, 2H, 2H von CH₂); 2.65 - 2.95 (m, 3H, 2*2H von CH₂); 3.30 (m, 2H, 2H von CH₂); 5.15 (br, 1H, 1H von NH); 5.70 und 5.90 (br, 1H, 1H von CH); 6.50 (s, 1H, thiophen-H); 8.65 und 8.80 (2*br, 1H, Pyr-6H); |
| 45 | fest; Smp.: 250.9 °C; logp (HCOOH): 0.91; 1H-NMR (CDCl₃, 400 MHZ, δ in ppm): 2.85 (m, 1H, 1H von CH₂); 3.05 (m, 2H, 2H von CH₂); 3.50 (m, 1H, 1H von CH₂); 5.40 bis 6.05 (jeweils br, 4H, 1H von CH, 1H von NH; 2H von NH₂); 7.15 (d, 1H, thiophen-H); 7.95 (d, 1H, thiophen-H); 8.35 und 8.45 (br, 1H, Pyr-6H); |
| 46 | oelig; logp (HCOOH): 1.91; 1H-NMR (CDCl₃, 400 MHZ, δ in ppm): 1.80 - 1.95 (m, 3H, 3H von CH₂); 2.10 (m, 1H, 1H von CH₂); 2.85 (m, 2H, 2H von CH₂); 4.90 bis 5.70 (jeweils br, 4H, 1H von CH, 1H von NH; 2H von NH₂); 6.90 (d, 1H, thiophen-H); 7.05 (d, 1H, thiophen-H); 8.00 und 8.20 (br, 1H, Pyr-6H); |
| 47 | oelig; logp (HCOOH): 2.62; 1H-NMR (CDCl₃, 400 MHZ, δ in ppm): 1.80 - 1.95 (m, 3H, 3H von CH₂); 2.10 (m, 1H, 1H von CH₂); 2.40 (s, 3H, 3H von CH₃); 2.70 (m, 2H, 2H von CH₂); 4.90 bis 5.90 (jeweils br, 4H, 1H von CH, 1H von NH; 2H von NH₂); 6.55 (s, 1H, thiophen-H); 7.90 und 8.20 (br, 1H, Pyr-6H); |
| 48 | oelig; logp (HCOOH): 2.57; 1H-NMR (CDCl₃, 400 MHZ, δ in ppm): 2.10 (m, 1H, 1H von CH₂); 2.70 (m, 1H, 1H von CH₂); 2.85 (m, 2H, 2H von CH₂); 5.00 bis 5.90 (jeweils br, 4H, 1H von CH, 1H von NH; 2H von NH₂); 6.65 (s, 1H, thiophen-H); 8.15 (br, 1H, Pyr-6H); |
| 49 | fest; logp (HCOOH): 2.32; |
| 50 | oelig; logp (HCOOH): 2.15; 1H-NMR (CDCl₃, 400 MHZ, δ in ppm): 2.10 (m, 1H, 1H von CH₂); 2.70 (m, 1H, 1H von CH₂); 2.85 (m, 2H, 2H von CH₂); 3.05 (s, 3H, 3H von SO₂-CH₃); 5.25 bis 6.10 (jeweils br, 4H, 1H von CH, 1H von NH; 2H von NH₂); 6.65 (s, 1H, thiophen-H); 8.25 und 8.50 (br, 1H, Pyr-6H); |
| 52 | fest; Smp.: 178.0 °C; logp (HCOOH): 2.36; 1H-NMR (DMSO, 400 MHZ, δ in ppm): 1.70 (m, 2H, 2H von CH₂); 1.85 - 2.00 (m, 2H, 2H von CH₂); 2.30 (s, 3H, 3H von CH₃); 2.65 (m, 2H, 2H von CH₂); 5.00 (br, 1H, 1H von CH); 6.45 (2s, 1H, thiophen-H); 7.00 und 7.20 (2br, 2H, 2H von NH₂); 7.50 und 7.70 (2d, 1H von NH; 1H von NH); 8.15 und 8.30 (br, 1H, Pyr-6H); |
| 53 | fest; logp (HCOOH): 2.41; 1H-NMR (CDCl₃, 400 MHZ, δ in ppm): 1.75 - 2.00 (m, 3H, 3H von CH₂); 2.10 (m, 1H, 1H von CH₂); 2.65 (m, 2H, 2H von CH₂); 3.05 (s, 3H, 3H von SO₂-CH₃); 5.15 bis 5.75 (jeweils br, 4H, 1H von CH, 1H von NH; 2H von NH₂); 6.60 (s, 1H, thiophen-H); 8.30 und 8.50 (br, 1H, Pyr-6H); |
| 54 | oelig; logp (HCOOH): 2.35; 1H-NMR (CDCl₃, 400 MHZ, δ in ppm): 1.80 - 2.00 (m, 4H, 2*2H von CH₂); 2.10 (m, 4H, 2*2H von CH₂); 2.70 (m, 4H, 2*2H von CH₂); 2.75 und 2.85 (2*m, 2H, 2H von CH₂); 5.20 (br, 1H, 1H von CH); 5.80 (br, 1H, 1H von CH); 6.30 (s, 1H, furan-H); 8.80 und 8.95 (br, 1H, Pyr-6H); |
| 55 | oelig; logp (HCOOH): 2.46; 1H-NMR (CDCl₃, 400 MHZ, δ in ppm): 1.85 - 2.00 (m, 3H, 3H von CH₂); 2.10 (m, 1H, 1H von CH₂); 2.40 (s, 3H, 3H von CH₃); 2.60 (m, 2H, 2H von CH₂); 4.90 bis 5.75 (jeweils br, 4H, 1H von CH, 1H von NH; 2H von NH₂); 6.45 (s, 1H, thiophen-H); 7.00 und 7.20 (2br, 2H, 2H von NH₂); 8.00 und 8.20 (br, 1H, Pyr-6H); |
| 56 | oelig; logp (HCOOH): 2.66; |
| 59 | fest; Smp.: 215.3 °C; logp (HCOOH): 2.21; 1H-NMR (DMSO, 400 MHZ, δ in ppm): 1.85 - 2.00 (m, 3H, 3H von CH₂); 2.05 (m, 1H, 1H von CH₂); 2.35 (s, 3H, 3H von CH₃); 2.70 (m, 2H, 2H von CH₂); 3.05 (s, 3H, 3H von SO₂-CH₃); 5.05, 5.15, 5.45 und 5.70 (jeweils br, 4H, 1H von CH, 1H von NH; 2H von NH₂); 6.50 (s, 1H, thiophen-H); 8.30 und 8.50 (br, 1H, Pyr-6H); |
| 60 | fest; Smp.: 218.2 °C; logp (HCOOH): 2.57; 1H-NMR (CDCl₃, 400 MHZ, δ in ppm): 1.85 - 2.10 (m, 4H, 4H von CH₂); 2.50 (s, 3H, 3H von CH₃); 2.60 (m, 2H, 2H von CH₂); 3.05 (s, 3H, 3H von SO₂-CH₃); 5.20 und 5.80 (jeweils br, 2H, 1H von CH, 1H von NH); 6.30 (s, 1H, furan-H); 8.60 und 8.80 (br, 1H, Pyr-6H); |
| 61 | fest; Smp.: 169.1 °C; |
| 62 | oelig; logp (HCOOH): 1.78; 1H-NMR (CDCl₃, 400 MHZ, δ in ppm): 1.70 - 2.00 (m, 4H, 4H von CH₂); 2.20 (s, 3H, 3H von CH₃); 2.55 (m, 2H, 2H von CH₂); 4.90 bis 5.75 (jeweils br, 4H, 1H von CH, 1H von NH; 2H von NH₂); 5.90 (s, 1H, furan-H); 8.30 und 8.50 (br, 1H, Pyr-6H); |
| 63 | fest; logp (HCOOH): 2.40; 1H-NMR (CDCl₃, 400 MHZ, δ in ppm): 1.35 (d, 6H, 6H von von CH(CH₃)₂); 2.20 (m, 1H, 1H von CH₂); 2.40 (s, 3H, 3H von CH₃); 2.80 - 3.00 (m, 3H, 3H von CH₂); 3.15 (sept, 1H, 1H von CH(CH₃)₂); 5.30 - 5.80 (jeweils br, 4H, 1H von CH, 1H von NH; 2H von NH₂); 6.50 (s, 1H, thiophen-H); 8.20 und 8.40 (br, 1H, Pyr-6H); |
| 64 | fest, logp (HCOOH): 2.71; 1H-NMR (CDCl₃, 400 MHZ, δ in ppm): 1.75 (m, 3H, 3H von CH₂); 2.05 (m, 1H, 1H von CH₂); 2.25 (s, 6H, 2*3H von thiophen-CH₃); 2.40 (s, 3H, 3H von pyrimidin-CH₃); 2.45 (m, 1H, 1H von CH₂); 2.65 (m, 1H, 1H von CH₂); 5.00 - 5.40 (br, 4H, 1H von CH; 2H von NH₂; 1H von NH); |
| 68 | fest; |
| 69 | fest; Smp.: 129.9 °C; logp (HCOOH): 1.86; 1H-NMR (CDCl₃, 400 MHZ, δ in ppm):): 1.85 (m, 3H, 3H von CH₂); 2.15 (m, 1H, 1H von CH₂); 2.70 (m, 2H, 2H von CH₂); 3.05 (s, 3H, 3H von SO₂-CH₃); 5.25 - 5.80 (br, 4H, 1H von CH; 2H von NH₂; 1H von NH); 6.80 (d, 1H, thiophen-H); 7.15 (d, 1H, thiophen-H); 8.35 und 8.50 (br, 1H, Pyr-6H); |
| 70 | Oelig |
| 71 | fest; logp (HCOOH): 2.98; 1H-NMR (CDCl₃, 400 MHZ, δ in ppm):): 1.85 (m, 3H, 3H von CH₂); 2.10 (m, 1H, 1H von CH₂); 2.50 (s, 3H, 3H von acetyl-CH₃); 2.60 und 2.65 (br, 3H, 3H von pyrimidin-CH₃); 2.80 (m, 2H, 2H von CH₂); 5.30 (br, 1H, 1H von CH); 5.70 (br, 1H, 1H von NH); 6.90 (d, 1H, thiophen-H); 7.10 (d, 1H, thiophen-H); 8.65 und 8.80 (br, 1H, Pyr-6H); |
| 72 | oelig; logp (HCOOH): 3.90; 1H-NMR (CDCl₃, 400 MHZ, δ in ppm): 1.85 (m, 3H, 3H von CH₂); 2.05 (m, 1H, 1H von CH₂); 2.40 (s, 3H, 3H von CH₃); 2.75 (m, 2H, 2H von CH₂); 5.00 bis 5.60 und 8.30 bis 8.50 (jeweils br, 4H, 1H von CH, 1H von NH; 2H von NH₂; 1H); 6.55 (s, 1H, thiophen-H); 8.60 und 8.80 (2*br, 1H, Pyr-6H); |
| 73 | oelig; logp (HCOOH): 2.38; |
| 74 | logp (HCOOH): 2.13; 1H-NMR (CDCl₃, 400 MHZ, δ in ppm):): 1.85 (m, 2H, 2H von CH₂); 2.10 (m, 2H, 2H von CH₂); 2.35 (s, 3H, 3H von thiophen-CH₃); 2.60 (s, 3H, 3H von pyrimidin-CH₃); 2.65 (m, 1H, 1H von CH₂); 2.80 (m, 1H, 1H von CH₂); 5.15 (br, 1H, 1H von CH); 5.90 (br, 2H, 2H von NH₂); 6.40 (d, 1H, thiophen-H); |
| 76 | fest; logp (HCOOH): 3.01; 1H-NMR (CDCl₃, 400 MHZ, δ in ppm):): 1.40 (s, 9H, 3*3H C(CH₃)₃); 1.85 (m, 3H, 3H von CH₂); 2.05 (m, 1H, 1H von CH₂); 2.40 (s, 3H, 3H von thiophen-CH₃); 2.75 (m, 2H, 2H von CH₂); 5.00 - 5.70 (jeweils br, 4H, 1H von CH, 1H von NH; 2H von NH₂); 6.55 (d, 1H, thiophen-H); 8.30 und 8.45 (br, 1H, Pyr-6H); |
| 77 | fest; logp (HCOOH): 2.67; 1H-NMR (CDCl₃, 400 MHZ, δ in ppm): 1.40 (s, 9H, 3*3H C(CH₃)₃); 2.10 (m, 1H, 1H von CH₂); 2.40 (s, 3H, 3H von thiophen-CH₃); 2.80 - 3.00 (m, 3H, 3H von CH₂); 5.00 - 5.70 (jeweils br, 4H, 1H von CH, 1H von NH; 2H von NH₂); 6.55 (d, 1H, thiophen-H); 8.20 und 8.40 (br, 1H, Pyr-6H); |
| 80 | fest; Smp.: 94.4 °C; logp (HCOOH): 5.60; 1H-NMR (CDCl₃, 400 MHZ, δ in ppm): 1.85 (m, 3H, 3H von CH₂); 2.05 (m, 1H, 1H von CH₂); 2.35 (s, 3H, 3H von thiophen-CH₃); 2.35 (s, 3H, 3H von thiophen-CH₃); 2.50 und 2.55 (br, 3H, 3H von pyrimidin-CH₃); 2.70 (m, 2H, 2H von CH₂); 5.15 (br, 1H, 1H von CH); 5.90 (br, 1H, 1H von NH); 6.55 (d, 1H, thiophen-H); 8.20 und 8.40 (br, 1H, Pyr-6H); |
| 150 | oelig; logp (HCOOH): 1.51; 1H-NMR (CDCl₃, 400 MHZ, δ in ppm): 1.85 (m, 3H, 3H von CH₂); 2.05 (m, 1H, 1H von CH₂); 2.35 (s, 3H, 3H von CH₃); 2.40 (s, 3H, 3H von CH₃); 2.75 (m, 2H, 2H von CH₂); 5.00 bis 5.60 und 7.80 bis 8.10 (jeweils br, 4H, 1H von CH, 1H von NH; 2H von NH_{2;} 1H); 6.55 (s, 1H, thiophen-H); 8.50 bis 8.85 (br, 1H, Pyr-6H); |
| 213 | fest; Smp.: 190.7 °C; logp (HCOOH): 2.66; 1H-NMR (CDCl₃, 400 MHZ, δ in ppm): 1.90 (m, 3H, 3H von CH₂); 1.95 (s, 3H, 3H von thiophen-CH₃); 2.05 (m, 1H, 1H von CH₂); 2.30 (s, 3H, 3H von thiophen-CH₃); 2.60 (m, 1H, 1H von CH₂); 2.75 (m, 1H, 1H von CH₂); 4.80 - 6.50 (jeweils br, 4H, 1H von CH, 1H von NH; 2H von NH₂); 7.70 und 8.20 (br, 1H, Pyr-6H); |
| 215 | oelig; logp (HCOOH): 1.66; 1H-NMR (CDCl₃, 400 MHZ, δ in ppm): 2.25 (m, 1H, 1H von CH₂); 2.85 (m, 1H, 1H von CH₂); 3.00 (m, 2H, 2H von CH₂); 4.90 - 6.30 (jeweils br, 4H, 1H von CH, 1H von NH; 2H von NH₂); 6.90 (d, 1H, thiophen-H); 7.20 (d, 1H, thiophen-H); 7.90 und 8.30 (br, 1H, Pyr-6H); |
| 223 | fest; logp (HCOOH): 2.57; 1H-NMR (CDCl₃, 400 MHZ, δ in ppm): 1.90 (m, 3H, 3H von CH₂); 2.05 (m, 1H, 1H von CH₂); 2.80 (m, 2H, 2H von CH₂); 5.10 (m, 1H, 1H von CH); 5.30 (br, 1H, 1H von NH); 6.90 (d, 1H, 1H von thiophen-H); 7.10 (d, 1H, 1H von thiophen-H); 8.20 (s, 2H, 2H von Pyr-4H und Pyr-6H); |
| 224 | fest; logp (HCOOH): 3.12; 1H-NMR (CDCl₃, 400 MHZ, δ in ppm): 1.90 (m, 3H, 3H von CH₂); 2.05 (m, 1H, 1H von CH₂); 2.40 (s, 3H, 3H von CH₃); 2.75 (m, 2H, 2H von CH₂); 5.00 (m, 1H, 1H von CH); 5.35 (br, 1H, 1H von NH); 6.50 (s, 1H, 1H von thiophen-H); 8.15 (s, 2H, 2H von Pyr-4H und Pyr-6H); |
| 240 | fest; Smp.: 118.0 °C; logp (HCOOH): 4.55; 1H-NMR (CDCl₃, 400 MHZ, δ in ppm): 1.40 (t, 3H, 3H von CH₃); 1.85 (m, 3H, 3H von CH₂); 2.10 (m, 1H, 1H von CH₂); 2.35 (s, 3H, 3H von CH₃); 2.55 bis 2.80 (m, 5H, 3H von Pyrimidin-CH_{3;} 2H von CH₂); 5.15 und 5.70 (jeweils br, 2H, 1H von NH; 1H von CH); 6.55 (s, 1H, thiophen-H); 8.70 und 8.90 (jeweils br, 1H, Pyr-6H); |
| 256 | fest; logp (HCOOH): 2.30; 1H-NMR (CDCl₃, 400 MHZ, δ in ppm): 2.15 (m, 1H, 1H von CH₂); 2.45 (s, 3H, 3H von Thiophen-CH₃); 2.80 bis 3.00 (m, 3H, 3H von CH₂); 4.90 bis 5.70 (br, 4H, 1H von CH; 1H von NH; 2H von NH₂), 6.70 (s, 1H, thiophen-H); 8.10 (br, 1H, Pyr-6H); |
| 257 | oelig; logp (HCOOH): 2.02; 1H-NMR (CDCl₃, 400 MHZ, δ in ppm): 2.10 (m, 1H, 1H von CH₂); 2.80 bis 3.05 (m, 3H, 3H von CH₂); 4.90 bis 5.70 (br, 4H, 1H von CH; 1H von NH; 2H von NH₂), 6.55 (s, 1H, thiophen-H); 8.00 bis 8.30 (br, 1H, Pyr-6H); |
| 258 | fest; Smp.: 155.9 °C; logp (HCOOH): 2.65; 1H-NMR (CDCl₃, 400 MHZ, δ in ppm): 1.80 bis 2.20 (m, 4H, 4H von CH₂); 2.50 (s, 3H, 3H von Acetyl-CH₃); 2.60 bis 2.80 (m, 5H, 2H von CH₂; 3H von Pyrimidin-CH₃); 5.25 und 6.20 (br, 2H, 1H von CH; 1H von NH), 6.70 (s, 1H, thiophen-H); 8.65 und 8.90 (jeweils br, 1H, Pyr-6H); |
| 263 | oelig; logp (HCOOH): 4.84; 1H-NMR (CDCl₃, 400 MHZ, δ in ppm): 1.60 (m, 4H, 4H von CH₂); 1.80 bis 2.05 (m, 6H, 6H von CH₂); 2.35 (s, 3H, 3H von CH₃); 2.40 (s, 3H, 3H von CH₃); 2.30 bis 2.45 (m, 1H, 1H von CH₂); 2.75 (m, 2H, 2H von CH₂); 5.15 und 5.70 (jeweils br, 2H, 1H von NH; 1H von CH); 6.20 (s, 1H, pyrido-H); 6.55 (s, 1H, thiophen-H); 8.40 und 8.60 (jeweils br, 1H, Pyr-6H); |
| 264 | oelig; logp (HCOOH): 2.77; 1H-NMR (CDCl₃, 400 MHZ, δ in ppm): 1.90 (m, 3H, 3H von CH₂); 2.05 (m, 1H, 1H von CH₂); 2.40 (s, 3H, 3H von CH₃); 2.75 (m, 2H, 2H von CH₂); 2.90 (s, 3H, 3H von -SO-CH₃); 4.90 - 5.50 und 7.90 (jeweils br, 4H, 1H von CH, 1H von NH; 2H von NH₂); 6.30 (t, 1H, 1H von CF₂H); 6.55 (s, 1H, thiophen-H); |
| 265 | oelig; logp (HCOOH): 1.35; 1H-NMR (CDCl₃, 400 MHZ, δ in ppm): 1.90 (m, 3H, 3H von CH₂); 2.05 (m, 1H, 1H von CH₂); 2.40 (s, 3H, 3H von CH₃); 2.75 (m, 2H, 2H von CH₂); 4.80 und 5.10 (jeweils br, 4H, 1H von CH, 1H von NH; 2H von NH₂); 6.55 (s, 1H, thiophen-H); 7.05, 7.15, 7.20 und 7.40 (jeweils m, 4H, 4H von Ph); 7.90 (s, 1H, Pyr-6H); |
| 266 | logp (HCOOH): 1.17; 1H-NMR (CDCl₃, 400 MHZ, δ in ppm): 1.90 (m, 3H, 3H von CH₂); 2.05 (m, 1H, 1H von CH₂); 2.40 (s, 3H, 3H von CH₃); 2.70 (m, 2H, 2H von CH₂); 2.95 (d, 3H, 3H von -HN-CH₃); 5.00 und 5.90 sowie 6.60 - 7.00 (jeweils br, 5H, 1H von CH, 1H von NH; 1H von CONH; 2H von NH₂); 6.55 (s, 1H, thiophen-H); 7.50 und 8.20 (jeweils br, 1H, Pyr-6H); |
| 267 | logp (HCOOH): 1.09; 1H-NMR (CDCl₃, 400 MHZ, δ in ppm): 1.90 (m, 3H, 3H von CH₂); 2.05 (m, 1H, 1H von CH₂); 2.40 (s, 3H, 3H von CH₃); 2.70 (m, 2H, 2H von CH₂); 2.95 (d, 3H, 3H von -HN-CH₃); 5.00 und 5.80 sowie 6.60 - 7.60 (jeweils br, 7H, 1H von CH, 1H von NH; 1H von CONH₂; 2H von NH_{2;} 1H, Pyr-4H); 6.55 (s, 1H, thiophen-H); |
| 268 | oelig; logp (HCOOH): 3.83; 1H-NMR (CDCl₃, 400 MHZ, δ in ppm): 1.80 (m, 3H, 3H von CH₂); 2.05 (m, 1H, 1H von CH₂); 2.40 (s, 3H, 3H von CH₃); 2.50 (s, 3H, 3H von COCH₃); 2.75 (m, 2H, 2H von CH₂); 5.00 bis 5.50 sowie 6.00 - 6.50 (jeweils br, 4H, 1H von CH, 1H von NH; 2H von NH_{2;} 1H); 6.55 (s, 1H, thiophen-H); |
| 275 | oelig; logp (HCOOH): 1.24; 1H-NMR (CDCl3, 400 MHZ, δ in ppm): 1.85 (m, 3H, 3H von CH2); 2.05 (m, 1H, 1H von CH2); 2.40 (s, 3H, 3H von CH3); 2.75 (m, 2H, 2H von CH2); 4.60 bis 5.40 (jeweils br, 4H, 1H von CH, 1H von NH; 2H von NH2; 1H); 5.70 und 6.15 (2*s, 2*1H, C=C-H); 6.55 (s, 1H, thiophen-H); 7.80 (br, 1H, Pyr-6H); |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277° C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,

   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Versuchsbeschreibung

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

In den nachfolgenden Tabellen werden folgende Abkürzungen verwendet:

**Unerwünschte Pflanzen / Weeds:**

| | | | |
|---|---|---|---|
| ABUTH: | *Abutilon theophrasti* | ALOMY: | *Alopecurus myosuroides* |
| AMARE: | *Amaranthus retroflexus* | AVEFA: | *Avena fatua* |
| CYPES: | *Cyperus esculentus* | ECHCG: | *Echinochloa crus-galli* |
| LOLMU: | *Lolium multiflorum* | MATIN: | *Matricaria inodora* |
| PHBPU: | *Ipomoea purpurea* | POLCO: | *Polygonum convolvulus* |
| SETVI: | *Setaria viridis* | STEME: | *Stellaria media* |
| VERPE: | *Veronica persica* | VIOTR: | *Viola tricolor* |

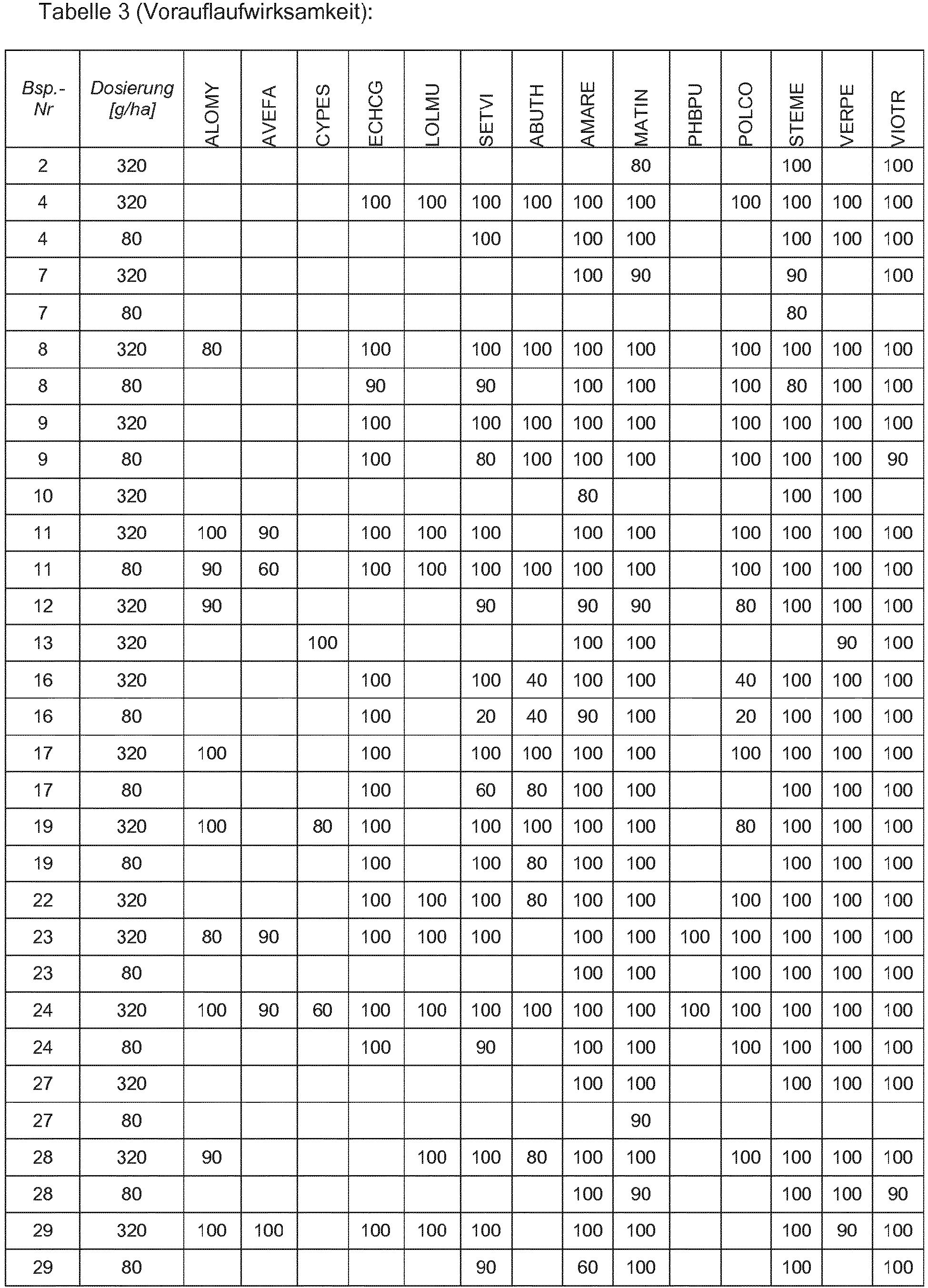

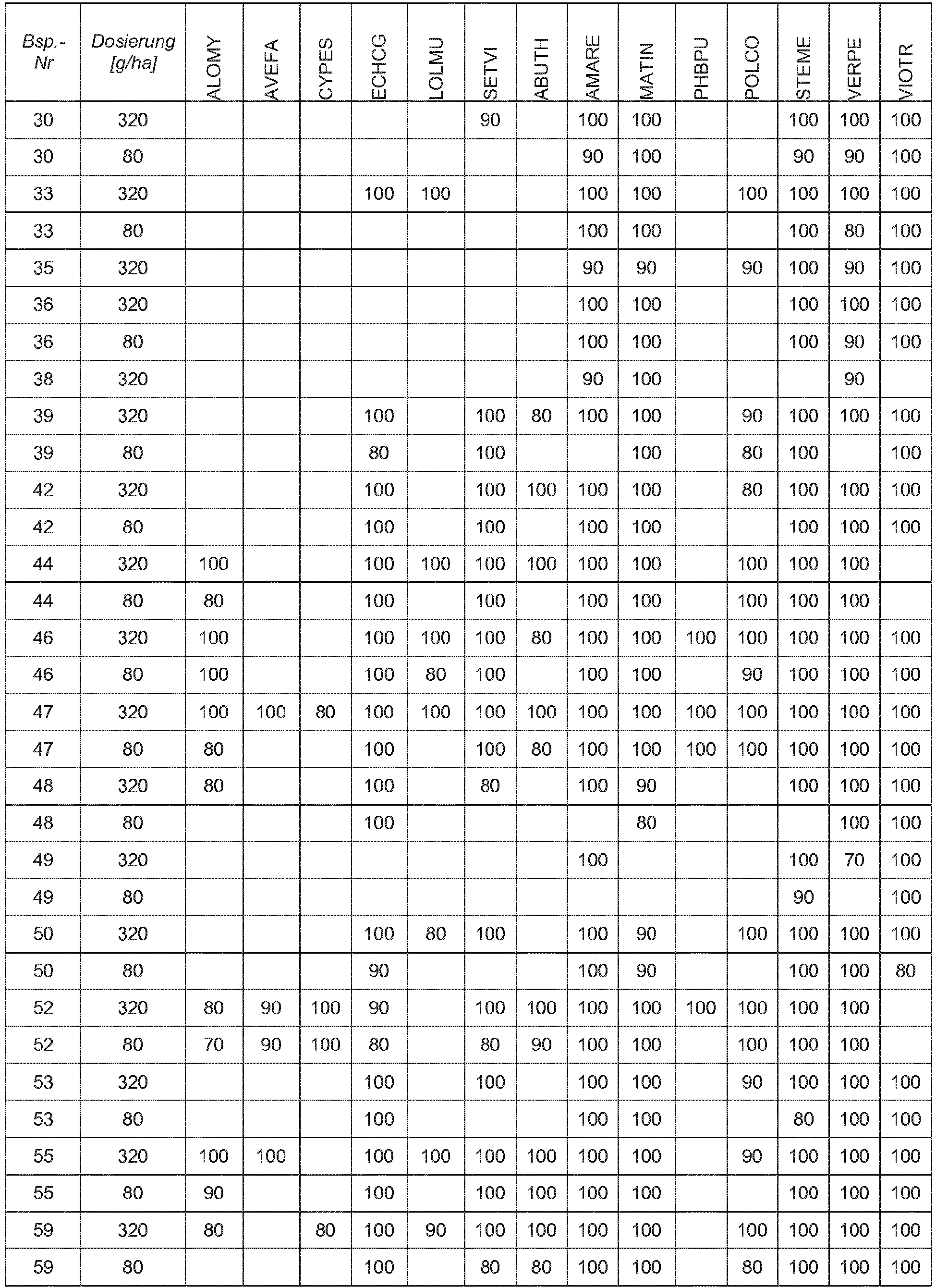

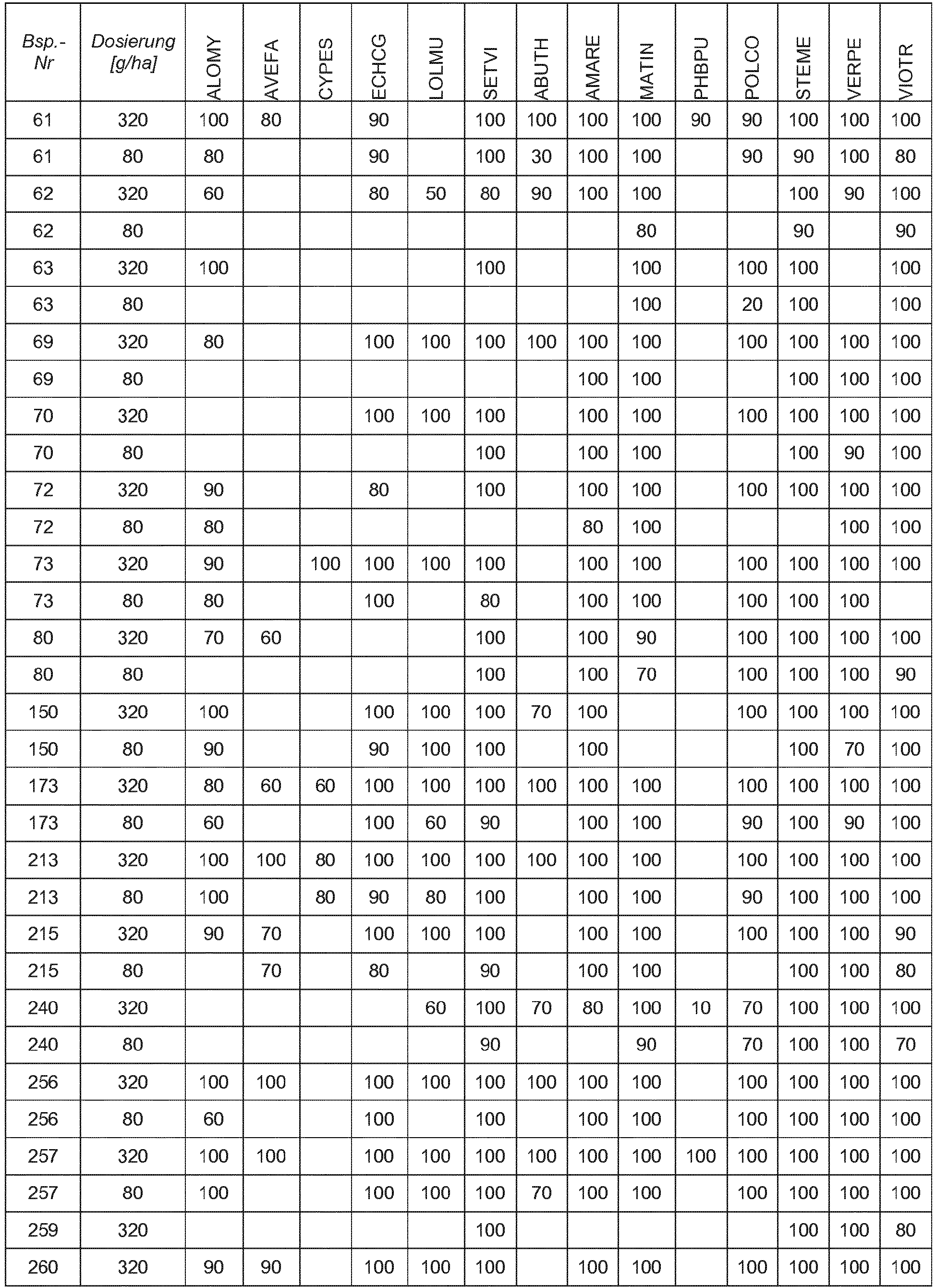

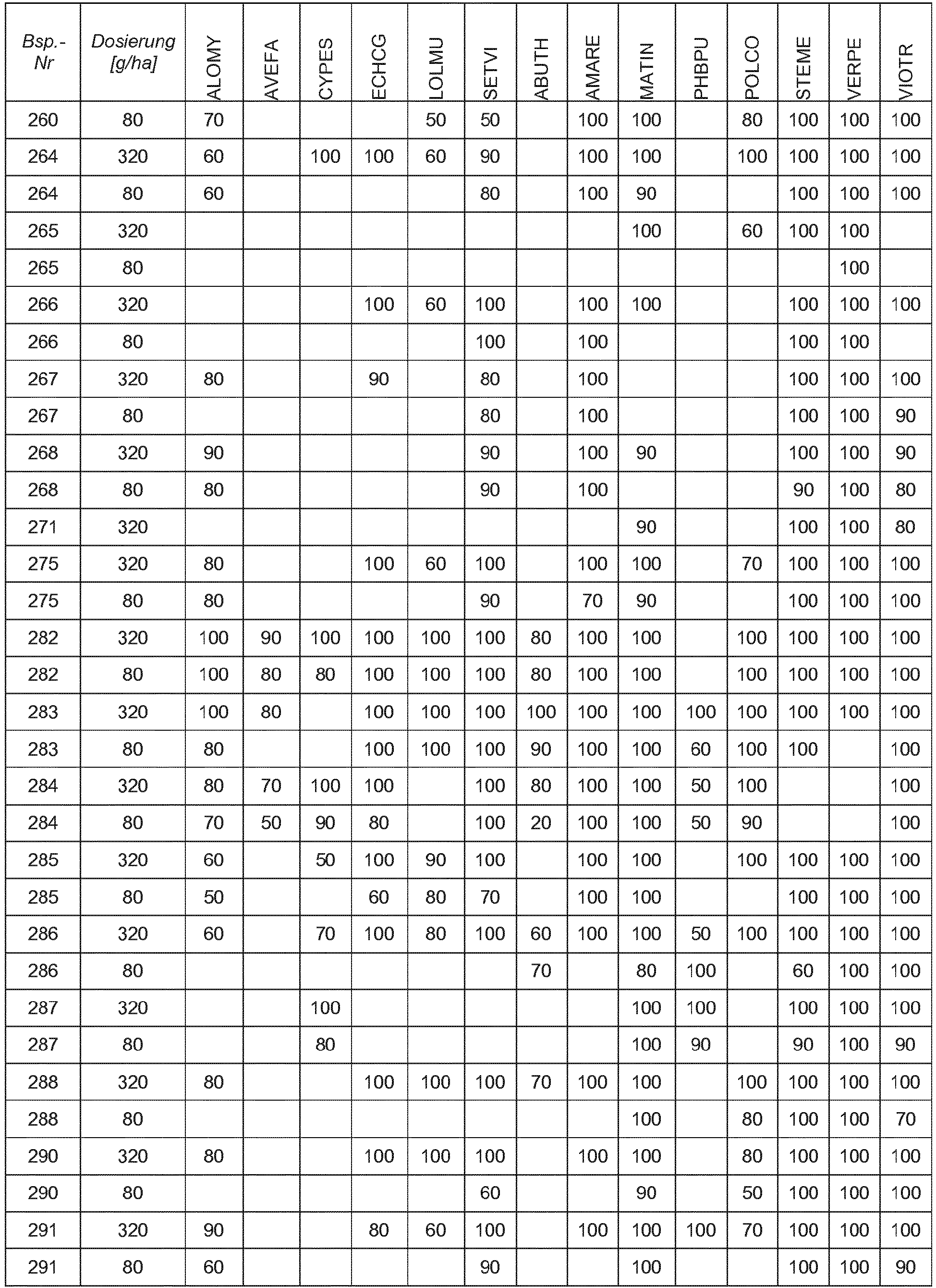

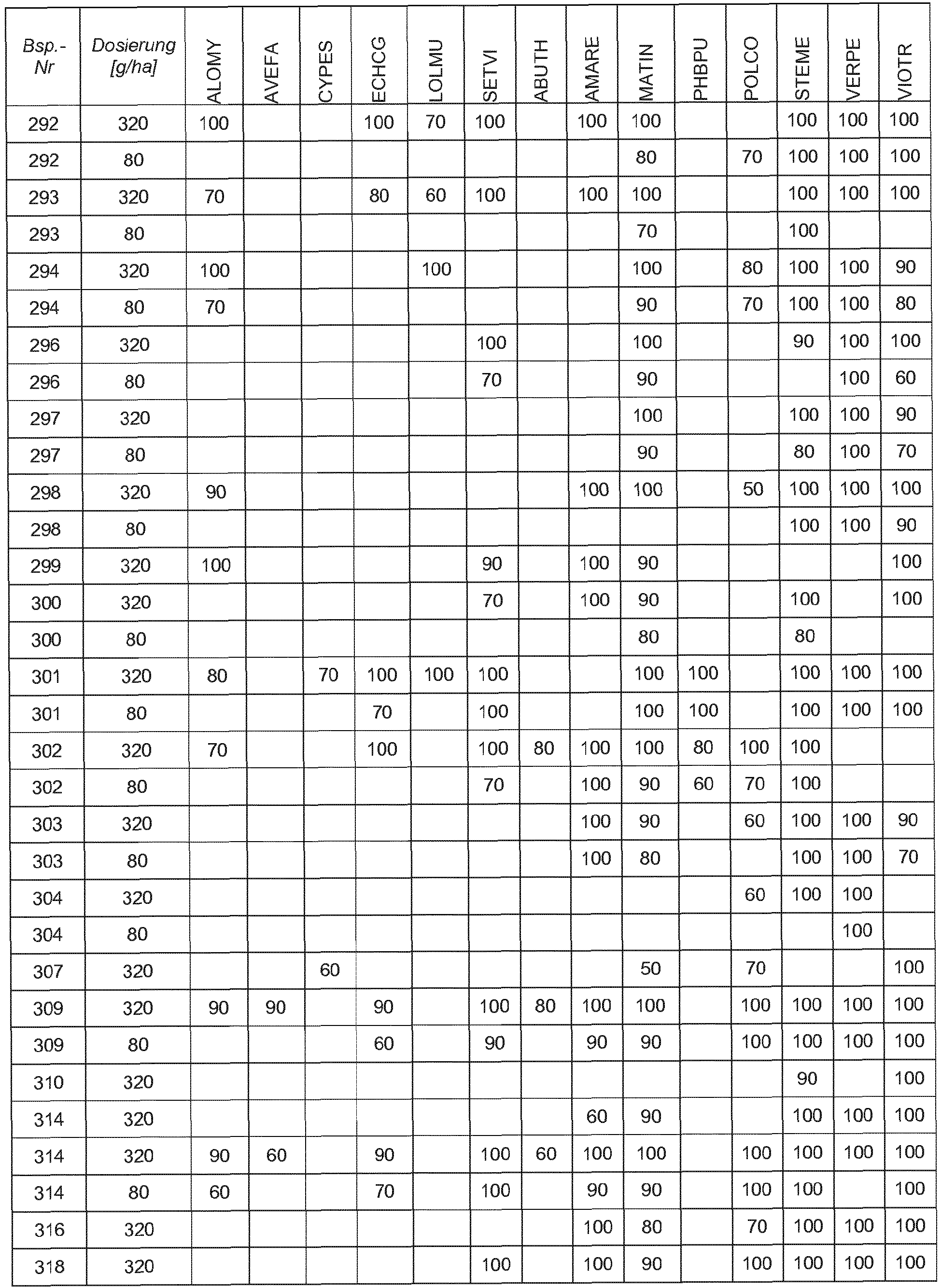

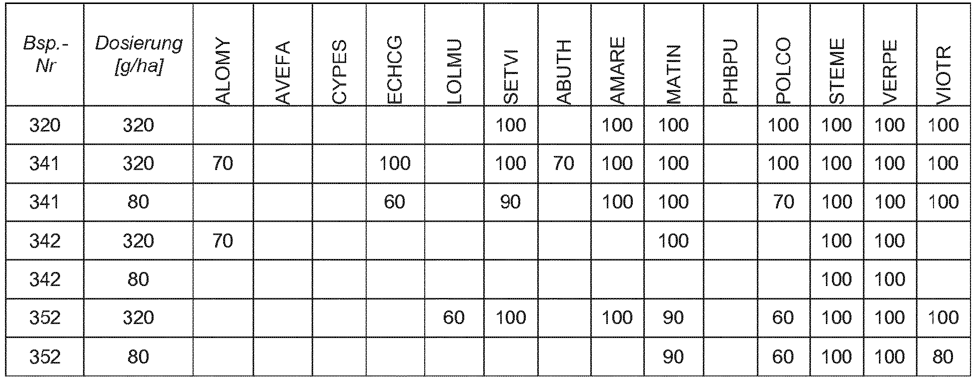

Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Verbindungen aus Tabelle 3 sehr gute herbizide Wirkung gegen Schadpflanzen wie Avena fatua, Stellaria media, Echinochloa crus-galli, Lolium multiflorum, Setaria viridis, Abutilon theophrasti, Amaranthus retroflexus und Alopecurus myosuroides im Vorauflaufverfahren bei einer Aufwandmenge von 0,32 kg und weniger Aktivsubstanz pro Hektar. Die erfindungsgemäßen Verbindungen eignen sich deshalb im Vorauflaufverfahren zur Bekämpfung von unerwünschtem Pflanzenwuchs.

### 2. Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

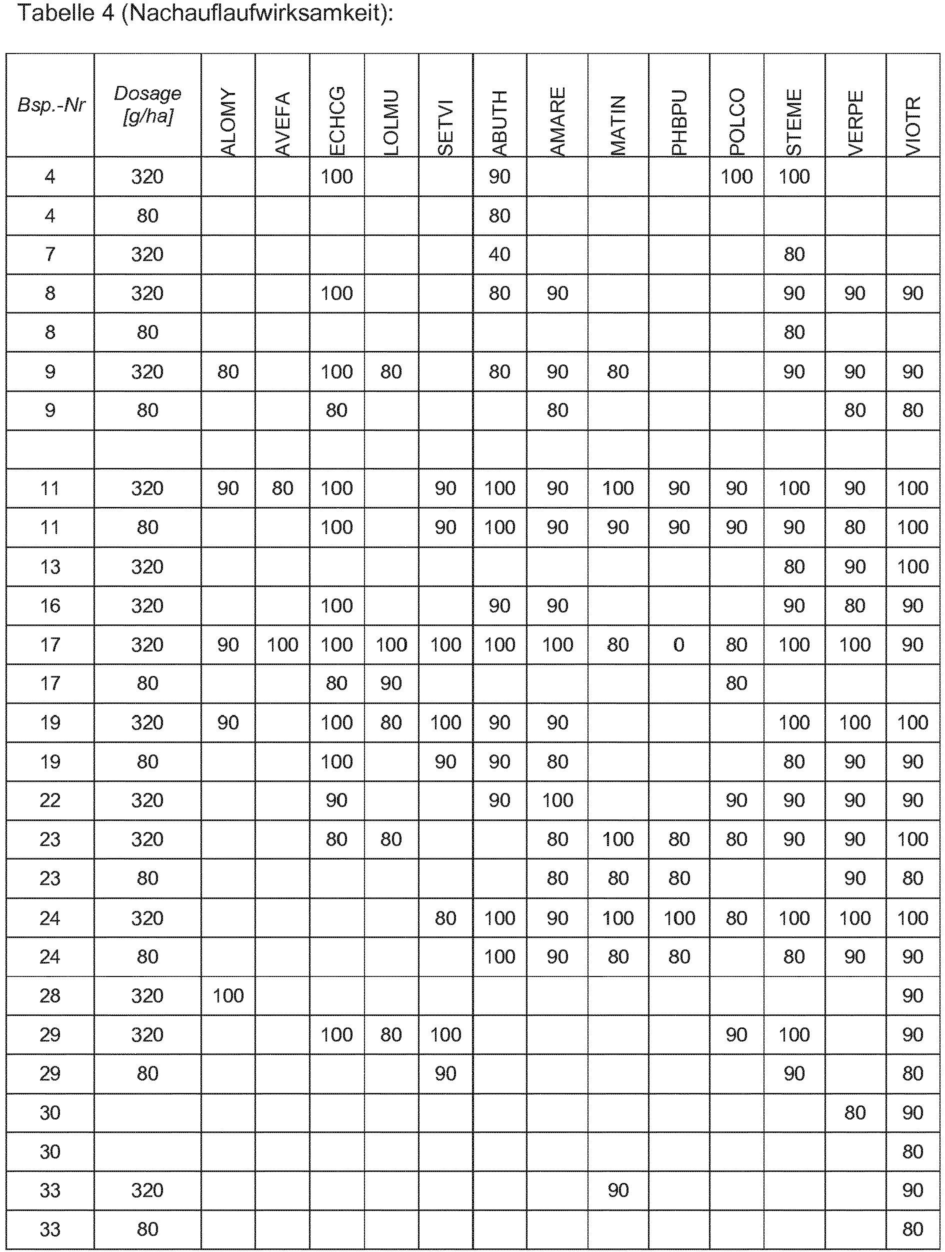

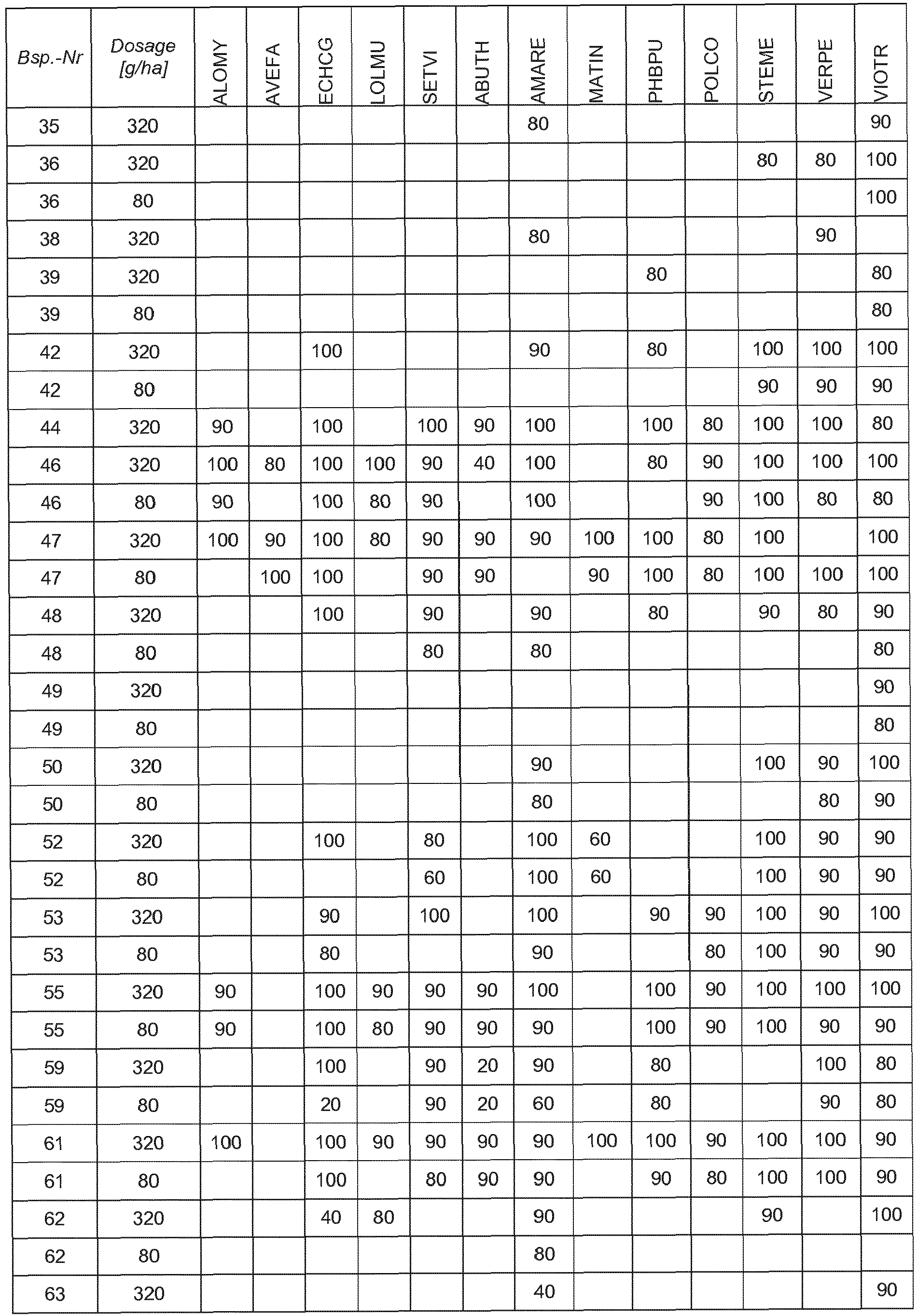

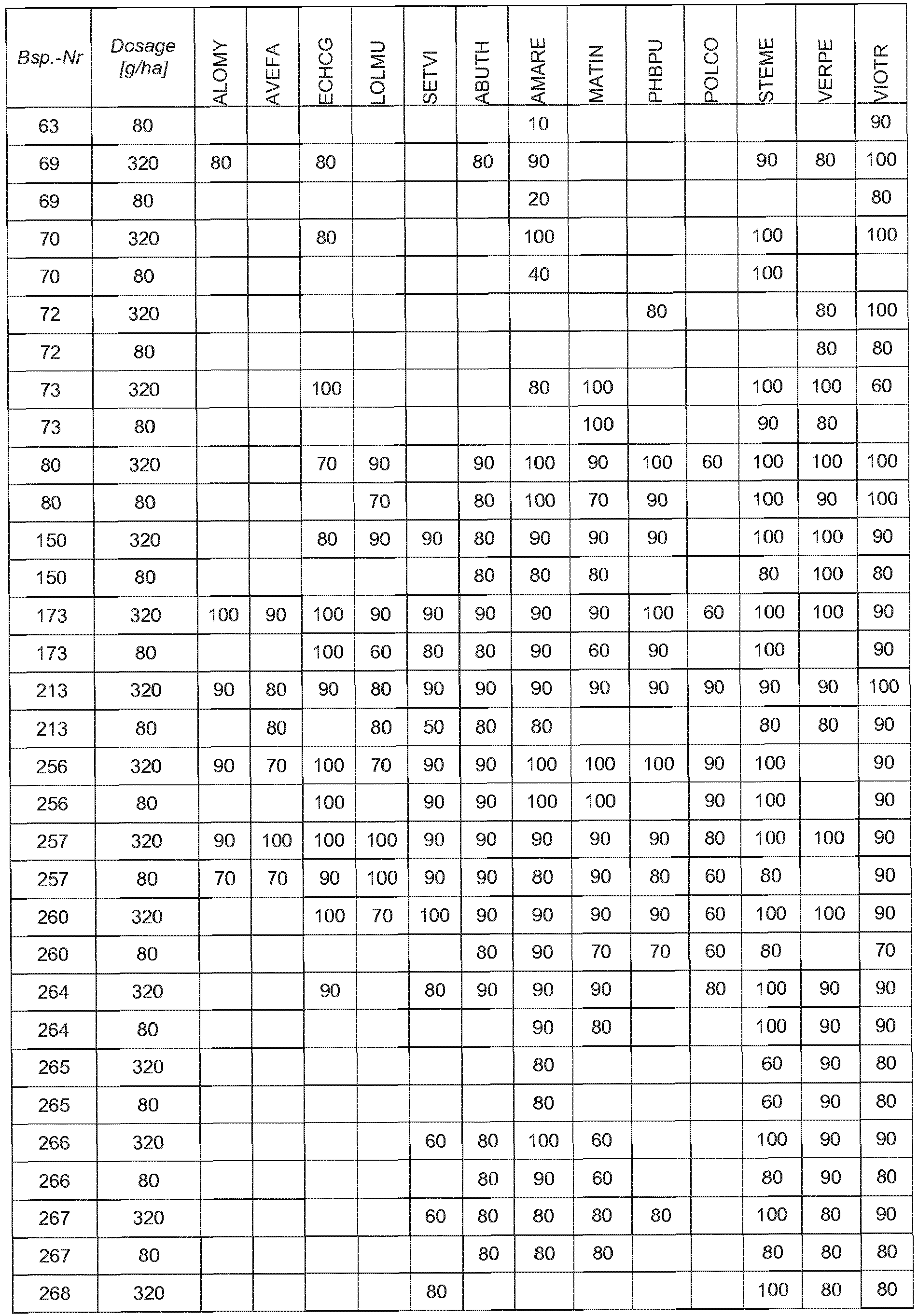

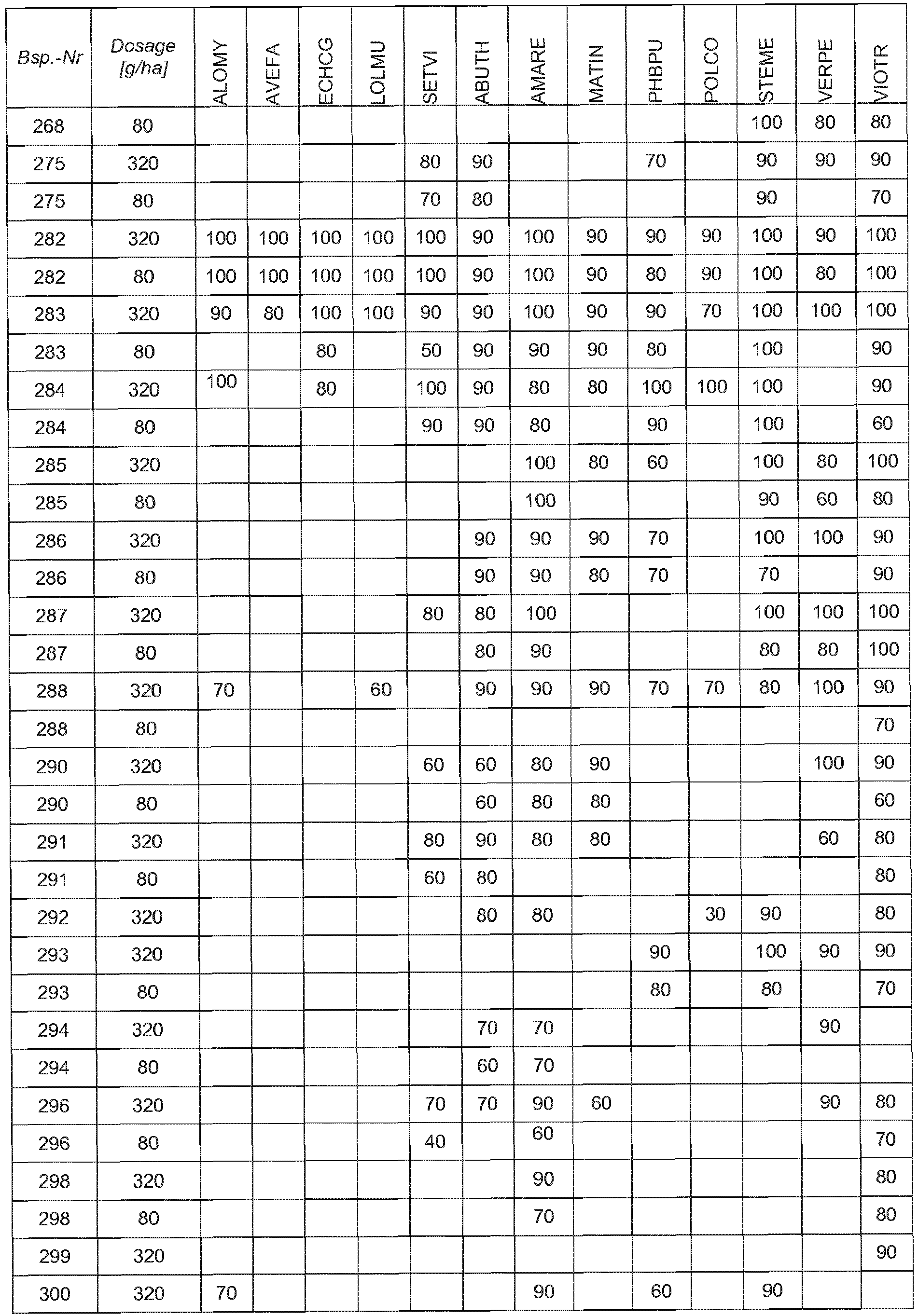

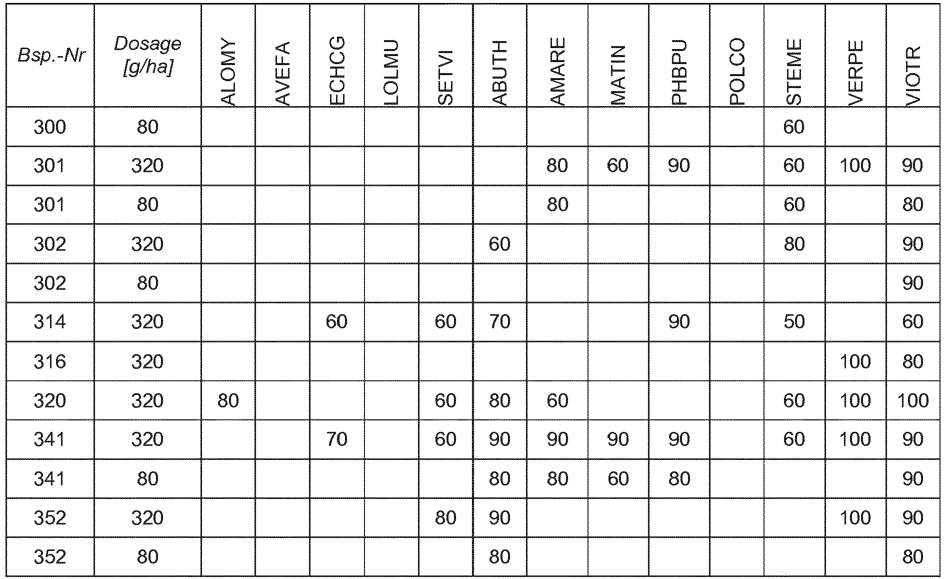

Wie die Ergebnisse zeigen, weisen erfindungsgemäße Verbindungen eine gute herbizide Nachauflaufwirksamkeit gegen ein breites Spektrum von Ungräsern und Unkräutern auf. Beispielsweise haben die Verbindungen aus Tabelle 4 sehr gute herbizide Wirkung gegen Schadpflanzen wie Avena fatua, Stellaria media, Echinochloa crus-galli, Lolium multiflorum, Setaria viridis, Abutilon theophrasti, Amaranthus retroflexus und Alopecurus myosuroides im Nachauflaufverfahren bei einer Aufwandmenge von 0,32 kg und weniger Aktivsubstanz pro Hektar. Die erfindungsgemäßen Verbindungen eignen sich deshalb im Nachauflaufverfahren zur Bekämpfung von unerwünschtem Pflanzenwuchs.

## Patentansprüche

1. Verbindungen der allgemeinen Formel (I) 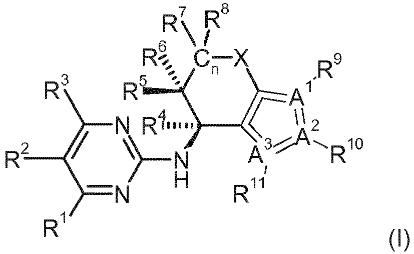 und deren agrochemisch verträglichen Salze, wobei
A¹, A² und A³ jeweils unabhängig voneinander ausgewählt sind aus O, S, CR⁹, CR¹⁰ oder CR¹¹, wobei genau ein Atom aus A¹, A² und A³ O oder S ist;
R⁹, R¹⁰ und R¹¹ jeweils unabhängig voneinander ausgewählt sind aus der Gruppe, bestehend aus Wasserstoff, Halogen, Cyano, C(O)OH, C(O)NH₂, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkenyl, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl und Nitro;
R¹ und R² jeweils unabhängig voneinander, ausgewählt sind aus der Gruppe, bestehend aus
- Halogen, Hydroxy, Nitro, Amino, Cyano, C(O)NH₂;
- (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
- (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
- (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl;
- Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können;
- (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
- Aminocarbonyl-(C₁-C₆)-alkyl, Di-(C₁-C₆)-Alkylaminocarbonyl-(C₁-C₆)-alkyl;
- N-((C₁-C₆)-Haloalkanoyl)-amino-carbonyl, Mono-((C₆-C₁₄)-aryl)-aminocarbonyl, Di-((C₆-C₁₄)-aryl)-amino-carbonyl;
- (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy;
- (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy;
- (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy;
- Hydroxy-(C₁-C₆)-alkyl, Hydroxy-(C₁-C₆)-alkoxy Cyano-(C₁-C₆)-alkoxy, Cyano-(C₁-C₆)-alkyl; und
- (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio und (C₃-C₆)-Alkinylthio;
R³ ausgewählt ist aus der Gruppe, bestehend aus
- Wasserstoff, Halogen, Hydroxy, Nitro, Amino, Cyano, C(O)OH, C(O)NH₂;
- (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
- (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
- (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl;
- Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können;
- (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
- Aminocarbonyl-(C₁-C₆)-alkyl, Di-(C₁-C₆)-Alkylaminocarbonyl-(C₁-C₆)-alkyl;
- N-((C₁-C₆)-Haloalkanoyl)-amino-carbonyl, Mono-((C₆-C₁₄)-aryl)-aminocarbonyl, Di-((C₆-C₁₄)-aryl)-amino-carbonyl;
- (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy;
- (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-CyCloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy;
- (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy;
- Hydroxy-(C₁-C₆)-alkyl, Hydroxy-(C₁-C₆)-alkoxy Cyano-(C₁-C₆)-alkoxy, Cyano-(C₁-C₆)-alkyl; und
- (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio und (C₃-C₆)-Alkinylthio, oder
R¹ mit R² über eine Bindung miteinander verbunden sein kann, so dass sich ein 5- bis 7-gliedriger teilhydrierter Carbocyclus oder Heterocyclus mit mindestens einem Heteroatom ausgewählt aus N, O, S und P ergibt, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl und (C₁-C₆)-Haloalkyl substituiert ist, und
R³ wie oben definiert ist, bevorzugt jedoch Wasserstoff, Methyl oder Amino ist;
R⁴ ausgewählt ist aus der Gruppe bestehend aus Wasserstoff, (C₁-C₆)-Alkyl, (C₁-C₆)-Alkoxy, (C₁-C₆)-Alkoxy-(C₁-C₆)-Alkyl, (C₁-C₆)-Alkoxycarbonyl und Aminocarbonyl;
R⁵ und R⁶ jeweils unabhängig voneinander ausgewählt sind aus der Gruppe, bestehend aus Wasserstoff, Hydroxy, (C₁-C₆)-Alkyl, (C₁-C₆)-Alkylphenyl, (C₁-C₆)-Haloalkyl, (C₁-C₆)-Alkoxy und (C₁-C₆)-Halogenalkoxy; oder die Reste R⁵ und R⁶ zusammen mit dem Kohlenstoffatom, an welchem sie gebunden sind, einen 3- bis 7-gliedrigen Ring bilden;
R⁷ und R⁸ jeweils unabhängig voneinander ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl; oder die Reste R⁷ und R⁸ zusammen eine (C₁-C₇)-Alkylengruppe bilden, die ein oder mehrere Sauerstoff- und/oder Schwefelatome enthalten können, wobei die (C₁-C₇)-Alkylengruppe durch Halogen einfach oder mehrfach substituiert sein kann und die jeweilgen Halogensubstituenten gleich oder verschieden sein können;
X für eine Bindung (falls n=1 oder 2 ist) steht, oder ausgewählt ist aus der Gruppe bestehend aus O, S, CH₂, C=O, NH, CR¹²R^{13,} und NR¹⁴, CH₂O und CH₂S, wobei bei den beiden letztgenannten Gruppen das Kohlenstoffatom an den aromatischen Teil und das Heteroatom O oder S an den teilhydrierten Teil des Amins gebunden ist;
R¹² und R¹³ jeweils unabhängig voneinander ausgewählt sind aus der Gruppe, bestehend aus Wasserstoff, (C₁-C₆)-Alkyl und (C₁-C₆)-Haloalkyl;
R¹⁴ ausgewählt ist aus der Gruppe bestehend aus Wasserstoff, (C₁-C₆)-Alkyl und (C₁-C₆)-Haloalkyl; und
n die Laufzahl 0, 1 oder 2 ist.

2. Verbindungen der allgemeinen Formel (I) nach Anspruch 1, **dadurch gekennzeichnet, dass** A¹, A² und A³ jeweils unabhängig voneinander ausgewählt sind aus S, CR⁹, CR¹⁰ oder CR¹¹, wobei genau ein Atom aus A¹, A² und A³ S ist.

3. Verbindungen der allgemeinen Formel (I) nach Anspruch 1 oder 2, **dadurch gekennzeichnet, dass** die Reste R⁹, R¹⁰ und R¹¹ jeweils unabhängig voneinander ausgewählt sind aus der Gruppe, bestehend aus Wasserstoff Halogen und (C₁-C₆)-Alkyl.

4. Verbindungen der allgemeinen Formel (I) nach einem der Ansprüche 1 bis 3, **dadurch gekennzeichnet, dass**
A¹, A² und A³ jeweils unabhängig voneinander, ausgewählt sind aus S, CR⁹, CR¹⁰ oder CR¹¹, wobei genau ein Atom aus A¹, A² und A³ S ist, und
R⁹, R¹⁰ und R¹¹ jeweils unabhängig voneinander, ausgewählt sind aus der Gruppe bestehend aus Wasserstoff, Chlor und (C₁-C₃)-Alkyl.

5. Verbindungen der allgemeinen Formel (I) nach Anspruch 1 bis 4, **dadurch gekennzeichnet, dass** genau ein Rest R⁹, R¹⁰ oder R¹¹ Methyl ist und die anderen Reste Wasserstoff sind.

6. Verbindungen der allgemeinen Formel (I) nach einem der Ansprüche 1 bis 5, **dadurch gekennzeichnet, dass** die Reste R¹ und R² jeweils unabhängig voneinander ausgewählt sind aus der Gruppe bestehend aus Halogen, Hydroxy, Nitro, Amino, Cyano, (C₁-C₆)-Alkyl, (C₁-C₆)-Halogenalkyl, (C₁-C₆)-Alkylcarbonyl, (C₁-C₆)-Alkylsulfonyl, (C₁-C₆)-Alkylthio, (C₁-C₆)-Alkylsulfinyl und (C₁-C₆)-Haloalkylsulfonyl und R³ ausgewählt ist aus der Gruppe bestehend aus Wasserstoff, Halogen, Hydroxy, Nitro, Amino, Cyano, (C₁-C₆)-Alkyl, (C₁-C₆)-Halogenalkyl, (C₁-C₆)-Alkylcarbonyl, (C₁-C₆)-Alkylsulfonyl, (C₁-C₆)-Alkylthio, (C₁-C₆)-Alkylsulfinyl und (C₁-C₆)-Haloalkylsulfonyl.

7. Verbindungen der allgemeinen Formel (I) nach einem der Ansprüche 1 bis 6, **dadurch gekennzeichnet, dass** die Reste R¹, R² und R³ jeweils verschieden voneinander sind und R¹ = Amino, R² = Trifluoromethyl oder Methylsulfonyl, und R³ = Wasserstoff oder Methyl bedeutet.

8. Verbindungen der allgemeinen Formel (I) nach einem der Ansprüche 1 bis 7, **dadurch gekennzeichnet, dass** die Reste R¹ mit R² über eine Bindung miteinander verbunden sind, so dass sich ein 5- oder 6-gliedriger teilhydrierter Carbocyclus oder Heterocyclus mit mindestens einem Heteroatom ausgewählt aus N, O, S und P bildet, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogene substituiertes Phenyl, (C₁-C₆)-Alkyl, (C₃-C₆)-Cycloalkyl, und (C₁-C₆)-Haloalkyl substituiert ist, und
R³ ausgewählt ist aus der Gruppe bestehend aus Wasserstoff, Amino, Methyl und Trifluoromethyl.

9. Verbindungen der allgemeinen Formel (I) nach einem der Ansprüche 1 bis 8, **dadurch gekennzeichnet, dass** der Rest R⁴ ausgewählt ist aus der Gruppe bestehend aus Wasserstoff, CH₃, CH₂CH₂OCH₃, COOCH₃ und CONH₂.

10. Verbindungen der allgemeinen Formel (I) nach einem der Ansprüche 1 bis 9, **dadurch gekennzeichnet, dass** die Reste R⁵ und R⁶ jeweils unabhängig voneinander ausgewählt sind aus der Gruppe bestehend aus Wasserstoff, Hydroxy, (C₁-C₆)-Alkyl, (C₁-C₆)-Alkylphenyl und (C₁-C₆)-Alkoxy.

11. Verbindungen der allgemeinen Formel (I) nach einem der Ansprüche 1 bis 10, **dadurch gekennzeichnet, dass** die Reste R⁷ und R⁸ jeweils unabhängig voneinander ausgewählt sind aus der Gruppe bestehend aus Wasserstoff, (C₁-C₆)-Alkyl und (C₆-C₁₄)-Aryl.

12. Verbindungen der allgemeinen Formel (I) nach einem der Ansprüche 1 bis 11, **dadurch gekennzeichnet, dass** X für eine Bindung (falls n=1 oder 2 ist) steht oder ausgewählt ist aus der Gruppe bestehend aus O, S, CH₂, C=O, NH, CHCH₃, NCH₃, C(CH₃)₂, OCH₂ und SCH₂, wobei bei den beiden letztgenannten das Kohlenstoffatom an den aromatischen Teil und das Heteroatom O oder S an den teilhydrierten Teil des Amins gebunden ist.

13. Verbindungen der allgemeinen Formel (I) nach einem der Ansprüche 1 bis 12, **dadurch gekennzeichnet, dass** die Laufzahl n 1 oder 2 beträgt.

14. Verbindungen der allgemeinen Formel (I) nach Anspruch 1, **dadurch gekennzeichnet, dass**
R¹ und R² und unabhängig voneiander ausgewählt sind aus der Gruppe bestehend aus Amino, Trifluoromethyl, (C₁-C₃)-Alkyl, (C₁-C₆)-Alkylsulfonyl;
R³ ausgewählt ist aus der Gruppe bestehend aus Wasserstoff, Amino, Trifluoromethyl, (C₁-C₆)-Alkyl, (C₁-C₆)-Alkylsulfonyl; oder
R¹ mit R² über eine Bindung miteinander verbunden sind, so dass sich ein 5- oder 6-gliedriger teilhydrierter Carbocyclus oder Heterocyclus mit mindestens einem Heteroatom ausgewählt aus N, O, S und P bildet, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere Halogenatome substituiertes Phenyl, (C₁-C₆)-Alkyl, (C₃-C₆)-Cycloalkyl, und (C₁-C₆)-Haloalkyl substituiert ist, und
R³ ausgewählt ist aus Wasserstoff, Methyl, Amino und Trifluoromethyl;
R⁴ ausgewählt ist aus der Gruppe bestehend aus Wasserstoff, CH₃, CH₂CH₂OCH₃, COOCH₃ und CONH₂;
R⁵ und R⁶ jeweils unabhängig voneinander ausgewählt sind aus der Gruppe bestehend aus Wasserstoff, (C₁-C₃)-Alkyl und (C₁-C₆)-Alkoxy;
R⁷ und R⁸ jeweils unabhängig voneinander ausgewählt sind aus der Gruppe, bestehend aus Wasserstoff, (C₁-C₆)-Alkyl und (C₆-C₁₄)-Aryl;
A¹, A² und A³ jeweils unabhängig voneinander ausgewählt sind aus S, CR⁹, CR¹⁰, CR¹¹, wobei genau ein Atom aus A¹, A² und A³ S ist;
R⁹, R¹⁰ und R¹¹ jeweils unabhängig voneinander ausgewählt sind aus der Gruppe bestehend aus Wasserstoff, Halogen und (C₁-C₆)-Alkyl;
X für eine Bindung (falls n=1 oder 2 ist) steht oder ausgewählt ist aus der Gruppe bestehend aus O, S, CH₂, =O, NH, CHCH₃, NCH₃, C(CH₃)₂, OCH₂ und SCH₂, wobei bei den beiden letztgenannten das Kohlenstoffatom an den aromatischen Teil und das Heteroatom O oder S an den teilhydrierten Teil des Amins gebunden ist; und
n die Laufzahl 1 oder 2 ist.

15. Verbindungen der allgemeinen Formel (I) 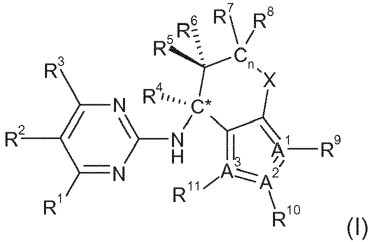 nach einem der Ansprüche 1 bis 14, **dadurch gekennzeichnet, dass** das chirale Kohlenstoffatom mit der Kennzeichnung (*) eine (R)-Konfiguration aufweist.

16. Verbindungen der allgemeinen Formel (I) 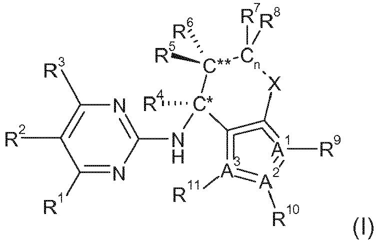 nach Anspruch 15, **dadurch gekennzeichnet, dass** das chirale Kohlenstoffatom mit der Kennzeichnung (*) eine (R)-Konfiguration und das chirale Kohlenstoffatom mit der Kennzeichnung (**) eine (S)-Konfiguration aufweist.

17. Verwendung von Verbindungen der allgemeinen Formel (I) 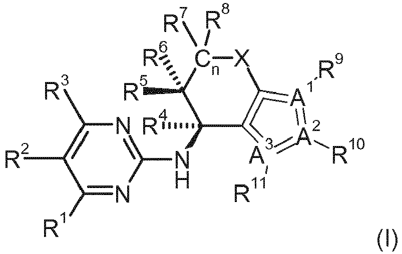 und deren agrochemisch verträglichen Salze, wobei
A¹, A² und A³ jeweils unabhängig voneinander ausgewählt sind aus O, S, CR⁹, CR¹⁰ oder CR¹¹, wobei genau ein Atom aus A¹, A² und A³ O oder S ist;
R⁹, R¹⁰ und R¹¹ jeweils unabhängig voneinander ausgewählt sind aus der Gruppe, bestehend aus Wasserstoff, Halogen, Cyano, C(O)OH, C(O)NH₂, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkenyl, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl und Nitro;
R¹, R² und R³ jeweils unabhängig voneinander, ausgewählt sind aus der Gruppe, bestehend aus
- Wasserstoff, Halogen, Hydroxy, Nitro, Amino, Cyano, C(O)OH, C(O)NH₂;
- (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
- (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
- (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl;
- Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können;
- (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
- Aminocarbonyl-(C₁-C₆)-alkyl, Di-(C₁-C₆)-Alkylaminocarbonyl-(C₁-C₆)-alkyl;
- N-((C₁-C₆)-Haloalkanoyl)-amino-carbonyl, Mono-((C₆-C₁₄)-aryl)-amino-carbonyl, Di-((C₆-C₁₄)-aryl)-amino-carbonyl;
- (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy;
- (C₃-C₈)-Cycloalkyl, welches gegebenenfalls am Cycloalkylrest durch (C₁-C₆)-Alkyl und/oder Halogen substituiert sein kann; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy;
- (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy;
- Hydroxy-(C₁-C₆)-alkyl, Hydroxy-(C₁-C₆)-alkoxy Cyano-(C₁-C₆)-alkoxy, Cyano-(C₁-C₆)-alkyl; und
- (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio und (C₃-C₆)-Alkinylthio, oder
R¹ mit R² über eine Bindung miteinander verbunden sein kann, so dass sich ein 5- bis 7-gliedriger teilhydrierter Carbocyclus oder Heterocyclus mit mindestens einem Heteroatom ausgewählt aus N, O, S und P ergibt, der optional mit einem oder mehreren Substituenten ausgewählt aus der Gruppe bestehend aus Hydroxy, =O, =N-O-H, =N-O-(C₁-C₆)-Alkyl, =N-O-Benzyl, =N-O-Phenyl, Phenyl, durch ein oder mehrere gleiche oder verschiedene Halogenatome substituiertes Phenyl, (C₁-C₆)-Alkyl, (C₃-C₆)-Cycloalkyl und (C₁-C₆)-Haloalkyl substituiert ist, und
R³ wie oben definiert ist, bevorzugt jedoch Wasserstoff oder Amino ist;
R⁴ ausgewählt ist aus der Gruppe bestehend aus Wasserstoff, (C₁-C₆)-Alkyl, (C₁-C₆)-Alkoxy, (C₁-C₆)-Alkoxy-(C₁-C₆)-Alkyl, (C₁-C₆)-Alkoxycarbonyl und Aminocarbonyl;
R⁵ und R⁶ jeweils unabhängig voneinander ausgewählt sind aus der Gruppe, bestehend aus Wasserstoff, Hydroxy, (C₁-C₆)-Alkyl, (C₁-C₆)-Alkylphenyl, (C₁-C₆)-Haloalkyl, (C₁-C₆)-Alkoxy und (C₁-C₆)-Halogenalkoxy; oder die Reste R⁵ und R⁶ zusammen mit dem Kohlenstoffatom, an welchem sie gebunden sind, einen 3- bis 7-gliedrigen Ring bilden;
R⁷ und R⁸ jeweils unabhängig voneinander ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl; oder die Reste R⁷ und R⁸ zusammen eine (C₁-C₇)-Alkylengruppe bilden, die ein oder mehrere Sauerstoff- und/oder Schwefelatome enthalten können, wobei die (C₁-C₇)-Alkylengruppe durch Halogen einfach oder mehrfach substituiert sein kann und die jeweilgen Halogensubstituenten gleich oder verschieden sein können;
X für eine Bindung (falls n=1 oder 2 ist) steht, oder ausgewählt ist aus der Gruppe bestehend aus O, S, CH₂, C=O, NH, CR¹²R¹³, und NR¹⁴, CH₂O und CH₂S, wobei bei den beiden letztgenannten Gruppen das Kohlenstoffatom an den aromatischen Teil und das Heteroatom O oder S an den teilhydrierten Teil des Amins gebunden ist;
R¹² und R¹³ jeweils unabhängig voneinander ausgewählt sind aus der Gruppe, bestehend aus Wasserstoff, (C₁-C₆)-Alkyl und (C₁-C₆)-Haloalkyl;
R¹⁴ ausgewählt ist aus der Gruppe bestehend aus Wasserstoff, (C₁-C₆)-Alkyl und (C₁-C₆)-Haloalkyl; und
n die Laufzahl 0, 1 oder 2 ist.

18. Verfahren zur Herstellung von Verbindungen der allgemeinen Formel (I) und/oder deren agrochemisch verträglichen Salze und/oder deren agrochemisch verträgliche quaternierten Stickstoff-Derivate 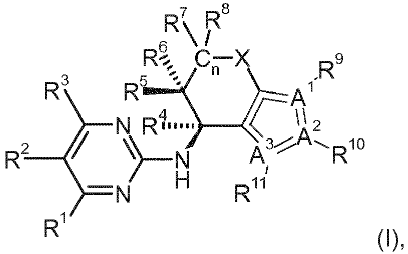 worin die Reste R¹ bis R¹⁴ und X wie in einem der Ansprüche 1 bis 14 definiert sind, und wobei
- eine Verbindung der allgemeinen Formel (II) 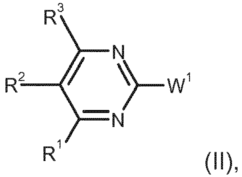
worin R¹ bis R³ wie in einem der Ansprüche 1 bis 14 definiert sind, und W¹ für einen austauschfähigen Rest oder eine Abgangsgruppe steht,
umgesetzt wird mit einem Amin der allgemeinen Formel (III) oder mit einem Säureadditionssalz des Amins der allgemeinen Formel (III) 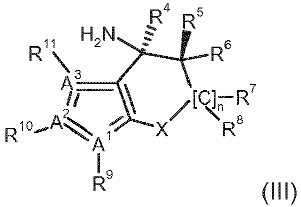
wobei die Reste R⁴ bis R¹¹, A¹ bis A³, n und X wie in einem der Ansprüche 1 bis 14 definiert sind.

19. Verfahren nach Anspruch 18, wobei der austauschfähige Rest bzw. die Abgangsgruppe Z¹ für Fluor, Chlor, Brom, Jod, ein (C₁-C₄)-Alkylsulfanyl oder ein (C₁-C₄)-Alkylsulfinyl oder ein (C₁-C₄)-Alkylsulfonyl, ein unsubstituiertes oder ein substituiertes Phenyl-(C₁-C₄)-alkylsulfonyl oder ein (C₁-C₄)-Alkylphenyl-sulfonyl steht.

20. Verfahren nach Anspruch 18, wobei eine Verbindung der allgemeinen Formel (II-a) 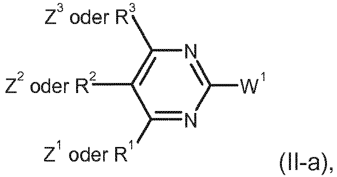 worin Z¹ bis Z³ für COOH, COO(C₁-C₆)-Alkyl, Nitril, C₂-C₆-Alkinyl, Halogen, Acetyl, Carbonyl und (C₁-C₆)-Alkylmercapto stehen und W¹ für einen austauschfähigen Rest oder eine Abgangsgruppe gemäß Anspruch 19 steht, umgesetzt wird mit einem Amin oder einem Säureadditionssalz der allgemeinen Formel (III) 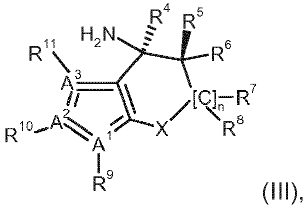 und zunächst ein Intermediat der Formel (I-a) erhalten wird 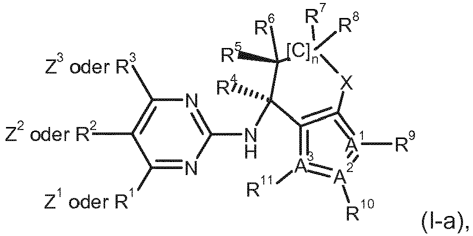 und das erhaltende Intermediat der Formel (I-a) anschließend nach bekannten Verfahren in die Verbindung (I) überführt wird.

21. Verfahren zur Herstellung von Verbindungen der allgemeinen Formel (I) und/oder deren agrochemisch verträglichen Salze und/oder deren agrochemisch verträgliche quaternierten Stickstoff-Derivate 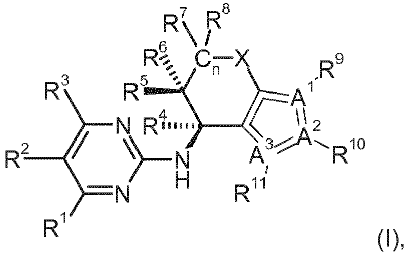
worin die Reste R¹ bis R¹⁴ und X wie in einem der Ansprüche 1 bis 16 definiert sind, und wobei
eine Verbindung der allgemeinen Formel (IV) oder dessen Säureadditionssalz 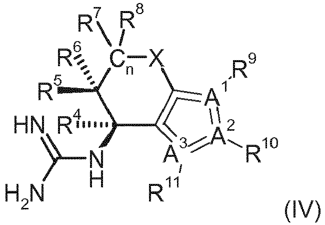 kondensiert wird mit einer Verbindung der allgemeinen Formel (V) 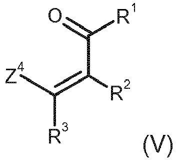
worin der Rest Z⁴ für ein (C₁-C₆)-Alkoxy oder Di-(C₁-C₆)-Alkylamino steht und R¹ bis R³ gemäß den Ansprüchen 1 bis 14 definiert sind.

22. Verfahren nach Anspruch 21, wobei die Verbindung der Formel (IV) oder dessen Säureadditionssalz 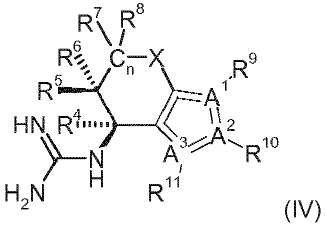 kondensiert wird mit einer Verbindung der Formel (VI) 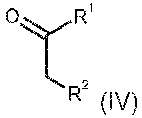 worin R¹ und R² wie in Anspruch 1 bis 14 definiert,
und einer Verbindung der Formel (VII) 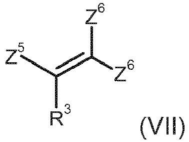 worin Z⁵ für ein (C₁-C₆)-Alkoxy oder Di-(C₁-C₆)-Alkylamino und Z⁶ für ein (C₁-C₆)-Alkoxy steht und R³ wie in Anspruch 1 bis 16 definiert.

23. Herbizides Mittel oder pflanzenwachstumsregulierendes Mittel, **dadurch gekennzeichnet, dass** es eine oder mehrere Verbindungen der allgemeinen Formel (I) oder deren Salze nach einem der Ansprüche 1 bis 16 enthält.

24. Verfahren zur Bekämpfung von Schadpflanzen oder zur Wachstumsregulierung von Pflanzen, **dadurch gekennzeichnet, dass** man eine wirksame Menge von einer oder mehreren Verbindungen der allgemeinen Formel (I) oder deren Salzen nach einem der Ansprüche 1 bis 16 auf Pflanzen, Pflanzenteile, Pflanzensamen oder auf eine Anbaufläche appliziert.

25. Verwendung von Verbindungen der allgemeinen Formel (I) oder deren Salzen nach einem der Ansprüche 1 bis 16 als Herbizide oder als Pflanzenwachstumsregulatoren.

26. Verwendung nach Anspruch 25, **dadurch gekennzeichnet, dass** die Verbindungen der allgemeinen Formel (I) oder deren Salze zur Bekämpfung von Schadpflanzen oder zur Wachstumsregulierung von Pflanzen in Kulturen von Nutz- oder Zierpflanzen eingesetzt werden.

27. Verwendung nach Anspruch 26, **dadurch gekennzeichnet, dass** die Kulturpflanzen transgene Kulturpflanzen sind.

## Claims

1. Compounds of the general formula (I) 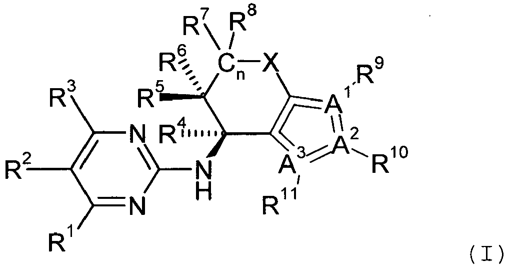 and the agrochemically acceptable salts thereof, where
A¹, A² and A³ each independently of one another are selected from the group consisting of O, S, CR9, CR¹⁰ and CR¹¹, where exactly one atom of A¹, A² and A³ represents O or S;
R⁹, R¹⁰ and R¹¹ each independently of one another are selected from the group consisting of hydrogen, halogen, cyano, C(O)OH, C(O)NH₂, (C₁-C₆)-alkyl, (C₁-C₆)-alkylcarbonyl, (C₁-C₆)-alkyloxycarbonyl, (C₁-C₆)-alkylaminocarbonyl, (C₁-C₆) -dialkylaminocarbonyl, (C₁-C₆)-haloalkyl, (C₁-C₆) -alkoxy, (C₁-C₆)-haloalkoxy, (C₂-C₆)-alkenyl, (C₂-C₆)-alkynyl, (C₂-C₆)-haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl, (C₂-C₆)-haloalkynyloxycarbonyl and nitro;
R¹ and R² each independently of one another are selected from the group consisting of
- halogen, hydroxy, nitro, amino, cyano, C(O)NH₂;
- (C₁-C₆)-alkyl, (C₁-C₆)-haloalkyl, (C₁-C₆)-alkylcarbonyl, (C₁-C₆) -haloalkylcarbonyl, (C₁-C₆)-alkylcarbonyloxy, (C₁-C₆)-haloalkylcarbonyloxy, (C₁-C₆)-alkylcarbonyl-(C₁-C₄)-alkyl;
- (C₁-C₆) -alkoxy, (C₁-C₆)-haloalkoxy, (C₁-C₆)-alkoxycarbonyl, (C₁-C₆)-haloalkoxycarbonyl, (C₁-C₆) -alkoxycarbonyl-(C₁-C₆)-alkyl, (C₁-C₆)-haloalkoxycarbonyl- (C₁-C₆) -alkyl, (C₁-C₆)-alkoxycarbonyl- (C₁-C₆)-haloalkyl, (C₁-C₆)-haloalkoxycarbonyl-(C₁-C₆)-halogenalkyl;
- (C₂-C₆)-alkenyl, (C₂-C₆)-haloalkenyl, (C₂-C₆)-alkenylcarbonyl, (C₂-C₆)-haloalkenylcarbonyl, (C₂-C₆)-alkenyloxy, (C₂-C₆)-haloalkenyloxy, (C₂-C₆)-alkenyloxycarbonyl, (C₂-C₆)-haloalkenyloxycarbonyl;
- (C₂-C₆)-alkynyl, (C₂-C₆)-haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl, (C₂-C₆)-haloalkynyloxycarbonyl;
- tri-(C₁-C₆)-alkylsilyl-(C₂-C₆)-alkynyl, di-(C₁-C₆)-alkylsilyl- (C₂-C₆)-alkynyl, mono-(C₁-C₆)-alkylsilyl- (C₂-C₆)-alkynyl; phenylsilyl-(C₂-C₆)-alkynyl;
- (C₆-C₁₄)-aryl, (C₆-C₁₄)-aryloxy, (C₆-C₁₄)-arylcarbonyl and (C₆-C₁₄)-aryloxycarbonyl which may each be substituted at the aryl moiety by halogen, (C₁-C₆) -alkyl and/or (C₁-C₆)-haloalkyl;
- (C₆-C₁₄)-aryl-(C₁-C₆)-alkyl, (C₆-C₁₄)-aryl-(C₁-C₆)-alkoxy, (C₆-C₁₄)-aryl-(C₁-C₆) -alkylcarbonyl, (C₆-C₁₄)-aryl-(C₁-C₆)-alkylcarbonyloxy, (C₆-C₁₄)-aryl-(C₁-C₆)-alkoxycarbonyl, (C₆-C₁₄)-aryl-(C₁-C₆)-alkoxycarbonyloxy;
- aminocarbonyl-(C₁-C₆)-alkyl, di-(C₁-C₆)-alkylaminocarbonyl-(C₁-C₆)-alkyl;
- N-((C₁-C₆)-haloalkanoyl)-amino-carbonyl, mono-((C₆-C₁₄)-aryl)-amino-carbonyl, di-((C₆-C₁₄)-aryl)-amino-carbonyl;
- (C₁-C₆)-alkoxy-(C₁-C₆)-alkyl, (C₁-C₆) -alkoxy-(C₁-C₆)-alkoxy, (C₁-C₆)-alkoxycarbonyl-(C₁-C₆)-alkoxy;
- (C₃-C₈)-cycloalkyl, which may optionally be substituted at the cycloalkyl radical by (C₁-C₆)-alkyl and/or halogen; (C₃-C₈)-cycloalkoxy, (C₃-C₈) -cycloalkyl-(C₁-C₆)-alkyl, (C₃-C₈)-cycloalkyl-(C₁-C₆)-haloalkyl, (C₃-C₈)-cycloalkyl-(C₁-C₆)-alkoxy, (C₃-C₈) -cycloalkyl-(C₁-C₆)-haloalkoxy, (C₃-C₈)-cycloalkylcarbonyl, (C₃-C₈)-cycloalkoxycarbonyl, (C₃-C₈) -cycloalkyl- (C₁-C₆)-alkylcarbonyl, (C₃-C₈)-cycloalkyl-(C₁-C₆)-haloalkylcarbonyl, (C₃-C₈) -cycloalkyl-(C₁-C₆)-alkoxycarbonyl, (C₃-C₈)-cycloalkyl-(C₁-C₆)-haloalkoxycarbonyl, (C₃-C₈)-cycloalkylcarbonyloxy, (C₃-C₈)-cycloalkoxycarbonyloxy, (C₃-C₈)-cycloalkyl-(C₁-C₆)-alkylcarbonyloxy, (C₃-C₈)-cycloalkyl-(C₁-C₆)-haloalkylcarbonyloxy, (C₃-C₈)-cycloalkyl- (C₁-C₆)-alkoxycarbonyloxy, (C₃-C₈)-cycloalkyl-(C₁-C₆)-haloalkoxycarbonyloxy;
- (C₃-C₈)-cycloalkenyl, (C₃-C₈)-cycloalkenyloxy, (C₃-C₈)-cycloalkenyl-(C₁-C₆)-alkyl, (C₃-C₈)-cycloalkenyl-(C₁-C₆)-haloalkyl, (C₃-C₈)-cycloalkenyl-(C₁-C₆) -alkoxy, (C₃-C₈)-cycloalkenyl-(C₁-C₆)-haloalkoxy, (C₃-C₈)-cycloalkenylcarbonyl, (C₃-C₈)-cycloalkenyloxycarbonyl, (C₃-C₈)-cycloalkenyl-(C₁-C₆)-alkylcarbonyl, (C₃-C₈)-cycloalkenyl-(C₁-C₆)-haloalkylcarbonyl, (C₃-C₈)-cycloalkenyl-(C₁-C₆)-alkoxycarbonyl, (C₃-C₈)-cycloalkenyl-(C₁-C₆)-haloalkoxycarbonyl, (C₃-C₈)-cycloalkenylcarbonyloxy, (C₃-C₈)-cycloalkenyloxycarbonyloxy, (C₃-C₈)-cycloalkenyl-(C₁-C₆)-alkylcarbonyloxy, (C₃-C₈)-cycloalkenyl-(C₁-C₆)-haloalkylcarbonyloxy, (C₃-C₈)-cycloalkenyl-(C₁-C₆)-alkoxycarbonyloxy, (C₃-C₈)-cycloalkenyl-(C₁-C₆)-haloalkoxycarbonyloxy;
- hydroxy-(C₁-C₆)-alkyl, hydroxy-(C₁-C₆)-alkoxy, cyano-(C₁-C₆)-alkoxy, cyano-(C₁-C₆)-alkyl; and
- (C₁-C₆)-alkylsulfonyl, (C₁-C₆)-alkylthio, (C₁-C₆)-alkylsulfinyl, (C₁-C₆)-haloalkylsulfonyl, (C₁-C₆)-haloalkylthio, (C₁-C₆)-haloalkylsulfinyl, (C₁-C₆)-alkylsulfonyl-(C₁-C₆)-alkyl, (C₁-C₆)-alkylthio-(C₁-C₆)-alkyl, (C₁-C₆)-alkylsulfinyl-(C₁-C₆)-alkyl, (C₁-C₆)-haloalkylsulfonyl-(C₁-C₆)-alkyl, (C₁-C₆)-haloalkylthio-(C₁-C₆)-alkyl, (C₁-C₆)-haloalkylsulfinyl-(C₁-C₆)-alkyl, (C₁-C₆)-alkylsulfonyl-(C₁-C₆)-haloalkyl, (C₁-C₆)-alkylthio-(C₁-C₆)-haloalkyl, (C₁-C₆)-alkylsulfinyl-(C₁-C₆) -haloalkyl, (C₁-C₆)-haloalkylsulfonyl-(C₁-C₆)-haloalkyl, (C₁-C₆)-haloalkylthio-(C₁-C₆)-haloalkyl, (C₁-C₆)-haloalkylsulfinyl-(C₁-C₆)-haloalkyl, (C₁-C₆)-alkylsulfonyloxy, (C₁-C₆)-haloalkylsulfonyloxy, (C₁-C₆)-alkylthiocarbonyl, (C₁-C₆)-haloalkylthiocarbonyl, (C₁-C₆)-alkylthiocarbonyloxy, (C₁-C₆)-haloalkylthiocarbonyloxy, (C₁-C₆)-alkylthio-(C₁-C₆)-alkyl, (C₁-C₆)-alkylthio-(C₁-C₆)-alkoxy, (C₁-C₆)-alkylthio-(C₁-C₆)-alkylcarbonyl, (C₁-C₆)-alkylthio-(C₁-C₆)-alkylcarbonyloxy; (C₄-C₁₄)-arylsulfonyl, (C₆-C₁₄)-arylthio, (C₆-C₁₄)-arylsulfinyl, (C₃-C₈)-cycloalkylthio, (C₃-C₈)-alkenylthio, (C₃-C₈)-cycloalkenylthio and (C₃-C₆)-alkynylthio;
R³ is selected from the group consisting of
- hydrogen, halogen, hydroxy, nitro, amino, cyano, C(O)OH, C(O)NH₂;
- (C₁-C₆)-alkyl, (C₁-C₆)-haloalkyl, (C₁-C₆)-alkylcarbonyl, (C₁-C₆)-haloalkylcarbonyl, (C₁-C₆)-alkylcarbonyloxy, (C₁-C₆)-haloalkylcarbonyloxy, (C₁-C₆)-alkylcarbonyl-(C₁-C₄)-alkyl;
- (C₁-C₆)-alkoxy, (C₁-C₆) -haloalkoxy, (C₁-C₆)-alkoxycarbonyl, (C₁-C₆) -haloalkoxycarbonyl, (C₁-C₆)-alkoxycarbonyl- (C₁-C₆)-alkyl, (C₁-C₆)-haloalkoxycarbonyl- (C₁-C₆)-alkyl, (C₁-C₆)-alkoxycarbonyl- (C₁-C₆)-haloalkyl, (C₁-C₆)-haloalkoxycarbonyl- (C₁-C₆)-halogenalkyl;
- (C₂-C₆)-alkenyl, (C₂-C₆)-haloalkenyl, (C₂-C₆)-alkenylcarbonyl, (C₂-C₆)-haloalkenylcarbonyl, (C₂-C₆)-alkenyloxy, (C₂-C₆)-haloalkenyloxy, (C₂-C₆)-alkenyloxycarbonyl, (C₂-C₆)-haloalkenyloxycarbonyl;
- (C₂-C₆)-alkynyl, (C₂-C₆)-haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl, (C₂-C₆)-haloalkynyloxycarbonyl;
- tri-(C₁-C₆)-alkylsilyl-(C₂-C₆)-alkynyl, di-(C₁-C₆)-alkylsilyl-(C₂-C₆)-alkynyl, mono-(C₁-C₆)-alkylsilyl-(C₂-C₆)-alkynyl; phenylsilyl-(C₂-C₆)-alkynyl;
- (C₆-C₁₄)-aryl, (C₆-C₁₄)-aryloxy, (C₆-C₁₄)-arylcarbonyl and (C₆-C₁₄)-aryloxycarbonyl which may each be substituted at the aryl moiety by halogen, (C₁-C₆)-alkyl and/or (C₁-C₆)-haloalkyl;
- (C₆-C₁₄)-aryl- (C₁-C₆) -alkyl, (C₆-C₁₄)-aryl- (C₁-C₆)-alkoxy, (C₆-C₁₄)-aryl- (C₁-C₆)-alkylcarbonyl, (C₆-C₁₄)-aryl- (C₁-C₆) -alkylcarbonyloxy, (C₆-C₁₄)-aryl- (C₁-C₆)-alkoxycarbonyl, (C₆-C₁₄)-aryl- (C₁-C₆)-alkoxycarbonyloxy;
- aminocarbonyl-(C₁-C₆)-alkyl, di-(C₁-C₆)-alkylaminocarbonyl-(C₁-C₆)-alkyl;
- N-((C₁-C₆)-haloalkanoyl)-amino-carbonyl, mono-((C₆-C₁₄)-aryl)-amino-carbonyl, di-((C₆-C₁₄)-aryl)-amino-carbonyl;
- (C₁-C₆)-alkoxy-(C₁-C₆)-alkyl, (C₁-C₆)-alkoxy-(C₁-C₆)-alkoxy, (C₁-C₆)-alkoxycarbonyl-(C₁-C₆)-alkoxy;
- (C₃-C₈)-cycloalkyl, which may optionally be substituted at the cycloalkyl radical by (C₁-C₆)-alkyl and/or halogen; (C₃-C₈)-cycloalkoxy, (C₃-C₈)-cycloalkyl-(C₁-C₆)-alkyl, (C₃-C₈)-cycloalkyl-(C₁-C₆)-haloalkyl, (C₃-C₈)-cycloalkyl-(C₁-C₆)-alkoxy, (C₃-C₈)-cycloalkyl-(C₁-C₆)-haloalkoxy, (C₃-C₈)-cycloalkylcarbonyl, (C₃-C₈)-cycloalkoxycarbonyl, (C₃-C₈)-cycloalkyl-(C₁-C₆)-alkylcarbonyl, (C₃-C₈)-cycloalkyl-(C₁-C₆)-haloalkylcarbonyl, (C₃-C₈)-cycloalkyl-(C₁-C₆)-alkoxycarbonyl, (C₃-C₈)-cycloalkyl-(C₁-C₆)-haloalkoxycarbonyl, (C₃-C₈)-cycloalkylcarbonyloxy, (C₃-C₈) cycloalkoxycarbonyloxy, (C₃-C₈)-cycloalkyl-(C₁-C₆)-alkylcarbonyloxy, (C₃-C₈)-cycloalkyl-(C₁-C₆)-haloalkylcarbonyloxy, (C₃-C₈)-cycloalkyl- (C₁-C₆)-alkoxycarbonyloxy, (C₃-C₈)-cycloalkyl-(C₁-C₆)-haloalkoxycarbonyloxy;
- (C₃-C₈)-cycloalkenyl, (C₃-C₈)-cycloalkenyloxy, (C₃-C₈)-cycloalkenyl-(C₁-C₆)-alkyl, (C₃-C₈)-cycloalkenyl-(C₁-C₆)-haloalkyl, (C₃-C₈)-cycloalkenyl-(C₁-C₆)-alkoxy, (C₃-C₈)-cycloalkenyl-(C₁-C₆)-haloalkoxy, (C₃-C₈)-cycloalkenylcarbonyl, (C₃-C₈)-cycloalkenyloxycarbonyl, (C₃-C₈)-cycloalkenyl-(C₁-C₆)-alkylcarbonyl, (C₃-C₈)-cycloalkenyl-(C₁-C₆)-haloalkylcarbonyl, (C₃-C₈)-cycloalkenyl- (C₁-C₆)-alkoxycarbonyl, (C₃-C₈)-cycloalkenyl- (C₁-C₆)-haloalkoxycarbonyl, (C₃-C₈)-cycloalkenylcarbonyloxy, (C₃-C₈)-cycloalkenyloxycarbonyloxy, (C₃-C₈)-cycloalkenyl- (C₁-C₆)-alkylcarbonyloxy, (C₃-C₈)-cycloalkenyl-(C₁-C₆)-haloalkylcarbonyloxy, (C₃-C₈)-cycloalkenyl-(C₁-C₆)-alkoxycarbonyloxy, (C₃-C₈)-cycloalkenyl-(C₁-C₆)-haloalkoxycarbonyloxy;
- hydroxy-(C₁-C₆)-alkyl, hydroxy-(C₁-C₆)-alkoxy, cyano-(C₁-C₆)-alkoxy, cyano-(C₁-C₆)-alkyl; and
- (C₁-C₆)-alkylsulfonyl, (C₁-C₆)-alkylthio, (C₁-C₆)-alkylsulfinyl, (C₁-C₆)-haloalkylsulfonyl, (C₁-C₆)-haloalkylthio, (C₁-C₆)-haloalkylsulfinyl, (C₁-C₆) -alkylsulfonyl-(C₁-C₆)-alkyl, (C₁-C₆)-alkylthio-(C₁-C₆)-alkyl, (C₁-C₆)-alkylsulfinyl-(C₁-C₆) -alkyl, (C₁-C₆)-haloalkylsulfonyl-(C₁-C₆)-alkyl, (C₁-C₆)-haloalkylthio-(C₁-C₆)-alkyl, (C₁-C₆)-haloalkylsulfinyl-(C₁-C₆)-alkyl, (C₁-C₆)-alkylsulfonyl-(C₁-C₆)-haloalkyl, (C₁-C₆)-alkylthio-(C₁-C₆)-haloalkyl, (C₁-C₆)-alkylsulfinyl-(C₁-C₆)-haloalkyl, (C₁-C₆)-haloalkylsulfonyl-(C₁-C₆)-haloalkyl, (C₁-C₆)-haloalkylthio-(C₁-C₆)-haloalkyl, (C₁-C₆)-haloalkylsulfinyl-(C₁-C₆)-haloalkyl, (C₁-C₆)-alkylsulfonyloxy, (C₁-C₆)-haloalkylsulfonyloxy, (C₁-C₆)-alkylthiocarbonyl, (C₁-C₆)-haloalkylthiocarbonyl, (C₁-C₆)-alkylthiocarbonyloxy, (C₁-C₆)-haloalkylthiocarbonyloxy, (C₁-C₆)-alkylthio-(C₁-C₆)-alkyl, (C₁-C₆)-alkylthio- (C₁-C₆)-alkoxy, (C₁-C₆)-alkylthio-(C₁-C₆)-alkylcarbonyl, (C₁-C₆)-alkylthio- (C₁-C₆)-alkylcarbonyloxy; (C₄-C₁₄)-arylsulfonyl, (C₆-C₁₄)-arylthio, (C₆-C₁₄)-arylsulfinyl, (C₃-C₈)-cycloalkylthio, (C₃-C₈)-alkenylthio, (C₃-C₈)-cycloalkenylthio, (C₃-C₆)-alkynylthio; or
R¹ may be attached to R² via a bond, resulting in a 5- to 7-membered partially hydrogenated carbocycle or heterocycle having at least one heteroatom selected from the group consisting of N, O, S and P, which carbocycle or heterocycle is optionally substituted by one or more substituents selected from the group consisting of hydroxy, =O, =N-O-H, =N-O-(C₁-C₆)-alkyl, =N-O-benzyl, =N-O-phenyl, phenyl, phenyl substituted by one or more identical or different halogen atoms, (C₁-C₆)-alkyl, (C₃-C₆)-cycloalkyl and (C₁-C₆)-haloalkyl, and
R³ is as defined above, but preferably represents hydrogen, methyl or amino;
R⁴ is selected from the group consisting of hydrogen, (C₁-C₆) -alkyl, (C₁-C₆) -alkoxy, (C₁-C₆)-alkoxy-(C₁-C₆) -alkyl, (C₁-C₆)-alkoxycarbonyl and aminocarbonyl;
R⁵ and R⁶ are each independently of one another selected from the group consisting of hydrogen, hydroxy, (C₁-C₆)-alkyl, (C₁-C₆)-alkylphenyl, (C₁-C₆)-haloalkyl, (C₁-C₆)-alkoxy and (C₁-C₆)-haloalkoxy; or the radicals R⁵ and R⁶ together with the carbon atom to which they are attached form a 3- to 7-membered ring;
R⁷ and R⁸ are each independently of one another selected from the group consisting of hydrogen, (C₁-C₆)-alkyl, (C₁-C₆)-haloalkyl, (C₁-C₆)-alkoxy, (C₁-C₆)-haloalkoxy, (C₆-C₁₄)-aryl, (C₆-C₁₄)-aryloxy, (C₆-C₁₄)-arylcarbonyl and (C₆-C₁₄)-aryloxycarbonyl; or the radicals R⁷ and R⁸ together form a (C₁-C₇)-alkylene group which may contain one or more oxygen and/or sulfur atoms, where the (C₁-C₇)-alkylene group may be mono- or polysubstituted by halogen and the respective halogen substituents may be identical or different;
X represents a bond (if n=1 or 2) or is selected from the group consisting of O, S, CH₂, C=O, NH, CR¹²R¹³ and NR¹⁴, CH₂O and CH₂S, where in the two last-mentioned groups the carbon atom is attached to the aromatic moiety and the heteroatom O or S is attached to the partially hydrogenated moiety of the amine;
R¹² and R¹³ are each independently of one another selected from the group consisting of hydrogen, (C₁-C₆)-alkyl and (C₁-C₆) -haloalkyl;
R¹⁴ is selected from the group consisting of hydrogen, (C₁-C₆)-alkyl and (C₁-C₆)-haloalkyl; and
n represents the running number 0, 1 or 2.

2. Compounds of the general formula (I) according to Claim 1, **characterized in that** A¹, A² and A³ each independently of one another are selected from the group consisting of S, CR⁹, CR¹⁰ and CR¹¹, where exactly one atom of A¹, A² and A³ represents S.

3. Compounds of the general formula (I) according to Claim 1 or 2, **characterized in that** the radicals R⁹, R¹⁰ and R¹¹, each independently of one another, are selected from the group consisting of hydrogen, halogen and (C₁-C₆)-alkyl.

4. Compound of the general formula (I) according to any of Claims 1 to 3, **characterized in that**
A¹, A² and A³ each independently of one another are selected from the group consisting of S, CR⁹, CR¹⁰ and CR¹¹, where exactly one atom of A¹, A² and A³ represents S, and
R⁹, R¹⁰ and R¹¹ each independently of one another are selected from the group consisting of hydrogen, chlorine and (C₁-C₃)-alkyl.

5. Compounds of the general formula (I) according to any of Claims 1 to 4, **characterized in that** exactly one radical R⁹, R¹⁰ or R¹¹ represents methyl and the other radicals represent hydrogen.

6. Compounds of the general formula (I) according to any of Claims 1 to 5, **characterized in that** the radicals R¹ and R² each independently of one another are selected from the group consisting of halogen, hydroxy, nitro, amino, cyano, (C₁-C₆)-alkyl, (C₁-C₆)-haloalkyl, (C₁-C₆) -alkylcarbonyl, (C₁-C₆)-alkylsulfonyl, (C₁-C₆) -alkylthio, (C₁-C₆)-alkylsulfinyl and (C₁-C₆)-haloalkylsulfonyl and R³ is selected from the group consisting of hydrogen, halogen, hydroxy, nitro, amino, cyano, (C₁-C₆)-alkyl, (C₁-C₆)-haloalkyl, (C₁-C₆) -alkylcarbonyl, (C₁-C₆) -alkylsulfonyl, (C₁-C₆) -alkylthio, (C₁-C₆)-alkylsulfinyl and (C₁-C₆)-haloalkylsulfonyl.

7. Compounds of the general formula (I) according to any of Claims 1 to 6, **characterized in that** the radicals R¹, R² and R³ are each different from one another and R¹ = amino, R² = trifluoromethyl or methylsulfonyl, and R³ = hydrogen or methyl.

8. Compounds of the general formula (I) according to any of Claims 1 to 7, **characterized in that** the radical R¹ is attached to R² via a bond, resulting in a 5- or 6-membered partially hydrogenated carbocycle or heterocycle having at least one heteroatom selected from the group consisting of N, O, S and P, which carbocycle or heterocycle is optionally substituted by one or more substituents selected from the group consisting of hydroxy, =O, =N-O-H, =N-O-(C₁-C₆)-alkyl, =N-O-benzyl, =N-O-phenyl, phenyl, phenyl substituted by one or more identical or different halogens, (C₁-C₆)-alkyl, (C₃-C₆)-cycloalkyl and (C₁-C₆)-haloalkyl, and R³ is selected from the group consisting of hydrogen, amino, methyl and trifluoromethyl.

9. Compounds of the general formula (I) according to any of Claims 1 to 8, **characterized in that** the radical R⁴ is selected from the group consisting of hydrogen, CH₃, CH₂CH₂OCH₃, COOCH₃ and CONH₂.

10. Compounds of the general formula (I) according to any of Claims 1 to 9, **characterized in that** the radicals R⁵ und R⁶ each independently of one another are selected from the group consisting of hydrogen, hydroxy, (C₁-C₆)-alkyl, (C₁-C₆)-alkylphenyl and (C₁-C₆)-alkoxy.

11. Compounds of the general formula (I) according to any of Claims 1 to 10, **characterized in that** the radicals R⁷ and R⁸ each independently of one another are selected from the group consisting of hydrogen, (C₁-C₆)-alkyl and (C₆-C₁₄)-aryl.

12. Compounds of the general formula (I) according to any of Claims 1 to 11, **characterized in that** X represents a bond (if n=1 or 2) or is selected from the group consisting of O, S, CH₂, C=O, NH, CHCH₃, NCH₃, C(CH₃)₂, OCH₂ and SCH₂, where in the two last-mentioned groups the carbon atom is attached to the aromatic moiety and the heteroatom O or S is attached to the partially hydrogenated moiety of the amine.

13. Compounds of the general formula (I) according to any of Claims 1 to 12, **characterized in that** the running number n represents 1 or 2.

14. Compounds of the general formula (I) according to Claim 1, **characterized in that**
R¹ and R² and independently of one another are selected from the group consisting of amino, trifluoromethyl (C₁-C₃)-alkyl, (C₁-C₆)-alkylsulfonyl;
R³ is selected from the group consisting of hydrogen, amino, trifluoromethyl, (C₁-C₆)-alkyl, (C₁-C₆)-alkylsulfonyl; or
R¹ is attached to R² via a bond, resulting in a 5- or 6-membered partially hydrogenated carbocycle or heterocycle having at least one heteroatom selected from the group consisting of N, O, S and P, which carbocycle or heterocycle is optionally substituted by one or more substituents selected from the group consisting of hydroxy, =O, =N-O-H, =N-O-(C₁-C₆)-alkyl, =N-O-benzyl, =N-O-phenyl, phenyl, phenyl substituted by one or more halogen atoms, (C₁-C₆)-alkyl, (C₃-C₆)-cycloalkyl and (C₁-C₆)-haloalkyl, and
R³ is selected from the group consisting of hydrogen, methyl, amino and trifluoromethyl;
R⁴ is selected from the group consisting of hydrogen, CH₃,
CH₂CH₂OCH₃, COOCH₃ and CONH₂;
R⁵ and R⁶ each independently of one another are selected from the group
consisting of hydrogen, (C₁-C₃) -alkyl and (C₁-C₆)-alkoxy;
R⁷ and R⁸ each independently of one another are selected from the group
consisting of hydrogen, (C₁-C₆)-alkyl and (C₆-C₁₄)-aryl;
A¹, A² and A³ each independently of one another are selected from the group consisting of S, CR⁹, CR¹⁰, CR¹¹, where exactly one atom of A¹, A² and A³ represents S;
R⁹, R¹⁰ and R¹¹ each independently of one another are selected from the group
consisting of hydrogen, halogen and (C₁-C₆)-alkyl; X represents a bond (if n=1 or 2) or is selected from the
group consisting of O, S, CH₂, =O, NH, CHCH₃, NCH₃, C(CH₃)₂, OCH₂ and SCH₂, where in the two last-mentioned groups the carbon atom is attached to the aromatic moiety and the heteroatom O or S is attached to the partially hydrogenated moiety of the amine; and
n represents the running number 1 or 2.

15. Compounds of the general formula (I) 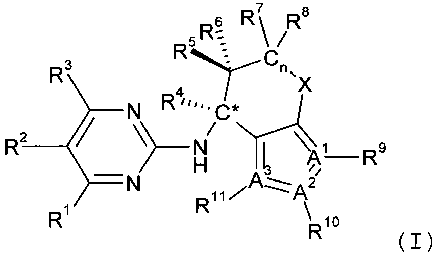 according to any of Claims 1 to 14, **characterized in that** the chiral carbon atom indicated by (*) has (R) configuration.

16. Compounds of the general formula (I) 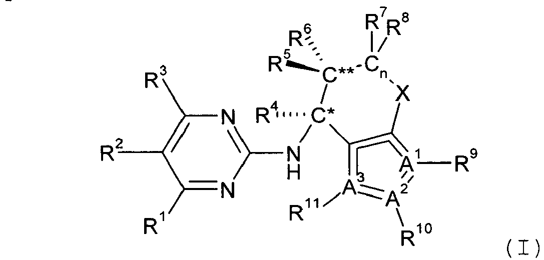 according to Claim 15, **characterized in that** the chiral carbon atom indicated by (*) has (R) configuration and the chiral carbon atom indicated by (**) has (S) configuration.

17. Use of compounds of the general formula (I) 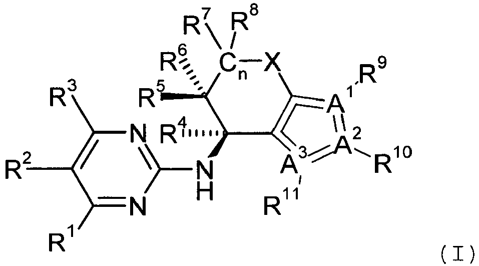 and the agrochemically acceptable salts thereof, where
A¹, A² and A³ each independently of one another are selected from the group consisting of O, S, CR⁹, CR¹⁰ and CR¹¹, where exactly one atom of A¹, A² and A³ represents O or S;
R⁹, R¹⁰ and R¹¹ each independently of one another are selected from the group consisting of hydrogen, halogen, cyano, C(O)OH, C(O)NH₂, (C₁-C₆)-alkyl, (C₁-C₆) -alkylcarbonyl, (C₁-C₆)-alkyloxycarbonyl, (C₁-C₆)-alkylaminocarbonyl, (C₁-C₆)-dialkylaminocarbonyl, (C₁-C₆)-haloalkyl, (C₁-C₆)-alkoxy, (C₁-C₆)-haloalkoxy, (C₂-C₆)-alkenyl, (C₂-C₆)-alkynyl, (C₂-C₆)-haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl, (C₂-C₆)-haloalkynyloxycarbonyl and nitro;
R¹, R² and R³ each independently of one another are selected from the group consisting of
- hydrogen, halogen, hydroxy, nitro, amino, cyano, C(O)OH, C(O)NH₂;
- (C₁-C₆)-alkyl, (C₁-C₆)-haloalkyl, (C₁-C₆)-alkylcarbonyl, (C₁-C₆)-haloalkylcarbonyl, (C₁-C₆)-alkylcarbonyloxy, (C₁-C₆)-haloalkylcarbonyloxy, (C₁-C₆)-alkylcarbonyl-(C₁-C₄)-alkyl;
- (C₁-C₆)-alkoxy, (C₁-C₆)-haloalkoxy, (C₁-C₆)-alkoxycarbonyl, (C₁-C₆)-haloalkoxycarbonyl, (C₁-C₆)-alkoxycarbonyl-(C₁-C₆)-alkyl, (C₁-C₆)-haloalkoxycarbonyl-(C₁-C₆)-alkyl, (C₁-C₆)-alkoxycarbonyl-(C₁-C₆)-haloalkyl, (C₁-C₆)-haloalkoxycarbonyl-(C₁-C₆)-halogenalkyl;
- (C₂-C₆)-alkenyl, (C₂-C₆)-haloalkenyl, (C₂-C₆)-alkenylcarbonyl, (C₂-C₆)-haloalkenylcarbonyl, (C₂-C₆)-alkenyloxy, (C₂-C₆)-haloalkenyloxy, (C₂-C₆)-alkenyloxycarbonyl, (C₂-C₆)-haloalkenyloxycarbonyl;
- (C₂-C₆)-alkynyl, (C₂-C₆)-haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl, (C₂-C₆)-haloalkynyloxycarbonyl;
- tri-(C₁-C₆)-alkylsilyl-(C₂-C₆)-alkynyl, di-(C₁-C₆)-alkylsilyl-(C₂-C₆)-alkynyl, mono-(C₁-C₆)-alkylsilyl-(C₂-C₆)-alkynyl; phenylsilyl-(C₂-C₆)-alkynyl;
- (C₆-C₁₄)-aryl, (C₆-C₁₄)-aryloxy, (C₆-C₁₄)-arylcarbonyl and (C₆-C₁₄)-aryloxycarbonyl which may each be substituted at the aryl moiety by halogen, (C₁-C₆) -alkyl and/or (C₁-C₆)-haloalkyl;
- (C₆-C₁₄)-aryl- (C₁-C₆) -alkyl, (C₆-C₁₄)-aryl- (C₁-C₆)-alkoxy, (C₆-C₁₄)-aryl- (C₁-C₆) -alkylcarbonyl, (C₆-C₁₄)-aryl- (C₁-C₆) -alkylcarbonyloxy, (C₆-C₁₄)-aryl- (C₁-C₆)-alkoxycarbonyl, (C₆-C₁₄)-aryl- (C₁-C₆)-alkoxycarbonyloxy;
- aminocarbonyl-(C₁-C₆)-alkyl, di-(C₁-C₆)-alkylaminocarbonyl-(C₁-C₆)-alkyl;
- N-((C₁-C₆)-haloalkanoyl)-amino-carbonyl, mono-((C₆-C₁₄)-aryl) -amino-carbonyl, di-((C₆-C₁₄)-aryl)-amino-carbonyl;
- (C₁-C₆)-alkoxy-(C₁-C₆) -alkyl, (C₁-C₆)-alkoxy-(C₁-C₆)-alkoxy, (C₁-C₆) -alkoxycarbonyl- (C₁-C₆)-alkoxy;
- (C₃-C₈)-cycloalkyl, which may optionally be substituted at the cycloalkyl radical by (C₁-C₆)-alkyl and/or halogen; (C₃-C₈)-cycloalkoxy, (C₃-C₈)-cycloalkyl- (C₁-C₆)-alkyl, (C₃-C₈)-cycloalkyl-(C₁-C₆)-haloalkyl, (C₃-C₈)-cycloalkyl-(C₁-C₆)-alkoxy, (C₃-C₈)-cycloalkyl-(C₁-C₆)-haloalkoxy, (C₃-C₈)-cycloalkylcarbonyl, (C₃-C₈)-cycloalkoxycarbonyl, (C₃-C₈)-cycloalkyl-(C₁-C₆)-alkylcarbonyl, (C₃-C₈)-cycloalkyl-(C₁-C₆)-haloalkylcarbonyl, (C₃-C₈)-cycloalkyl-(C₁-C₆)-alkoxycarbonyl, (C₃-C₈)-cycloalkyl-(C₁-C₆)-haloalkoxycarbonyl, (C₃-C₈)-cycloalkylcarbonyloxy, (C₃-C₈)-cycloalkoxycarbonyloxy, (C₃-C₈)-cycloalkyl-(C₁-C₆) -alkylcarbonyloxy, (C₃-C₈)-cycloalkyl-(C₁-C₆) -haloalkylcarbonyloxy, (C₃-C₈)-cycloalkyl-(C₁-C₆) -alkoxycarbonyloxy, (C₃-C₈)-cycloalkyl-(C₁-C₆)-haloalkoxycarbonyloxy;
- (C₃-C₈)-cycloalkenyl, (C₃-C₈)-cycloalkenyloxy, (C₃-C₈)-cycloalkenyl-(C₁-C₆)-alkyl, (C₃-C₈)-cycloalkenyl-(C₁-C₆) -haloalkyl, (C₃-C₈)-cycloalkenyl-(C₁-C₆) -alkoxy, (C₃-C₈)-cycloalkenyl-(C₁-C₆) -haloalkoxy, (C₃-C₈)-cycloalkenylcarbonyl, (C₃-C₈)-cycloalkenyloxycarbonyl, (C₃-C₈)-cycloalkenyl-(C₁-C₆) -alkylcarbonyl, (C₃-C₈)-cycloalkenyl-(C₁-C₆) -haloalkylcarbonyl, (C₃-C₈)-cycloalkenyl-(C₁-C₆) -alkoxycarbonyl, (C₃-C₈)-cycloalkenyl-(C₁-C₆) -haloalkoxycarbonyl, (C₃-C₈)-cycloalkenylcarbonyloxy, (C₃-C₈)-cycloalkenyloxycarbonyloxy, (C₃-C₈)-cycloalkenyl-(C₁-C₆)-alkylcarbonyloxy, (C₃-C₈)-cycloalkenyl-(C₁-C₆)-haloalkylcarbonyloxy, (C₃-C₈)-cycloalkenyl- (C₁-C₆)-alkoxycarbonyloxy, (C₃-C₈)-cycloalkenyl- (C₁-C₆)-haloalkoxycarbonyloxy;
- hydroxy-(C₁-C₆)-alkyl, hydroxy- (C₁-C₆) -alkoxy, cyano-(C₁-C₆)-alkoxy, cyano-(C₁-C₆)-alkyl; and
- (C₁-C₆)-alkylsulfonyl, (C₁-C₆) -alkylthio, (C₁-C₆)-alkylsulfinyl, (C₁-C₆)-haloalkylsulfonyl, (C₁-C₆)-haloalkylthio, (C₁-C₆)-haloalkylsulfinyl, (C₁-C₆)-alkylsulfonyl-(C₁-C₆)-alkyl, (C₁-C₆)-alkylthio-(C₁-C₆) -alkyl, (C₁-C₆) -alkylsulfinyl-(C₁-C₆)-alkyl, (C₁-C₆)-haloalkylsulfonyl-(C₁-C₆)-alkyl, (C₁-C₆)-haloalkylthio-(C₁-C₆) -alkyl, (C₁-C₆)-haloalkylsulfinyl-(C₁-C₆)-alkyl, (C₁-C₆)-alkylsulfonyl-(C₁-C₆)-haloalkyl, (C₁-C₆)-alkylthio-(C₁-C₆)-haloalkyl, (C₁-C₆)-alkylsulfinyl-(C₁-C₆) -haloalkyl, (C₁-C₆)-haloalkylsulfonyl-(C₁-C₆) -haloalkyl, (C₁-C₆)-haloalkylthio-(C₁-C₆) -haloalkyl, (C₁-C₆)-haloalkylsulfinyl-(C₁-C₆) -haloalkyl, (C₁-C₆)-alkylsulfonyloxy, (C₁-C₆)-haloalkylsulfonyloxy, (C₁-C₆)-alkylthiocarbonyl, (C₁-C₆)-haloalkylthiocarbonyl, (C₁-C₆)-alkylthiocarbonyloxy, (C₁-C₆)-haloalkylthiocarbonyloxy, (C₁-C₆)-alkylthio-(C₁-C₆) -alkyl, (C₁-C₆) -alkylthio-(C₁-C₆)-alkoxy, (C₁-C₆)-alkylthio-(C₁-C₆)-alkylcarbonyl, (C₁-C₆) -alkylthio-(C₁-C₆)-alkylcarbonyloxy; (C₄-C₁₄)-arylsulfonyl, (C₆-C₁₄)-arylthio, (C₆-C₁₄)-arylsulfinyl, (C₃-C₈)-cycloalkylthio, (C₃-C₈)-alkenylthio, (C₃-C₈)-cycloalkenylthio, (C₃-C₆)-alkynylthio; or
R¹ may be attached to R² via a bond, resulting in a 5- to 7-membered partially hydrogenated carbocycle or heterocycle having at least one heteroatom selected from the group consisting of N, O, S and P, which carbocycle or heterocycle is optionally substituted by one or more substituents selected from the group consisting of hydroxy, =O, =N-O-H, =N-O-(C₁-C₆)-alkyl, =N-O-benzyl, =N-O-phenyl, phenyl, phenyl substituted by one or more identical or different halogen atoms, (C₁-C₆)-alkyl, (C₃-C₆)-cycloalkyl and (C₁-C₆)-haloalkyl, and
R³ is as defined above, but preferably represents hydrogen or amino;
R⁴ is selected from the group consisting of hydrogen, (C₁-C₆)-alkyl, (C₁-C₆)-alkoxy, (C₁-C₆)-alkoxy-(C₁-C₆) -alkyl, (C₁-C₆)-alkoxycarbonyl and aminocarbonyl;
R⁵ and R⁶ are each independently of one another selected from the group consisting of hydrogen, hydroxy, (C₁-C₆)-alkyl, (C₁-C₆)-alkylphenyl, (C₁-C₆) -haloalkyl, (C₁-C₆) -alkoxy and (C₁-C₆)-haloalkoxy; or the radicals R⁵ and R⁶ together with the carbon atom to which they are attached form a 3- to 7-membered ring;
R⁷ and R⁸ are each independently of one another selected from the group consisting of hydrogen, (C₁-C₆) -alkyl, (C₁-C₆) -haloalkyl, (C₁-C₆)-alkoxy, (C₁-C₆)-haloalkoxy, (C₆-C₁₄)-aryl, (C₆-C₁₄)-aryloxy, (C₆-C₁₄)-arylcarbonyl and (C₆-C₁₄)-aryloxycarbonyl; or the radicals R⁷ and R⁸ together form a (C₁-C₇)-alkylene group which may contain one or more oxygen and/or sulfur atoms, where the (C₁-C₇)-alkylene group may be mono- or polysubstituted by halogen and the respective halogen substituents may be identical or different;
X represents a bond (if n=1 or 2) or is selected from the group consisting of O, S, CH₂, C=O, NH, CR¹²R¹³ and NR¹⁴, CH₂O and CH₂S, where in the two last-mentioned groups the carbon atom is attached to the aromatic moiety and the heteroatom O or S is attached to the partially hydrogenated moiety of the amine;
R¹² and R¹³ are each independently of one another selected from the group consisting of hydrogen, (C₁-C₆)-alkyl and (C₁-C₆) -haloalkyl;
R¹⁴ is selected from the group consisting of hydrogen, (C₁-C₆)-alkyl and (C₁-C₆) -haloalkyl; and
n represents the running number 0, 1 or 2.

18. Process for preparing compounds of the general formula (I) and/or agrochemically acceptable salts thereof and/or agrochemically acceptable quaternized nitrogen derivatives thereof 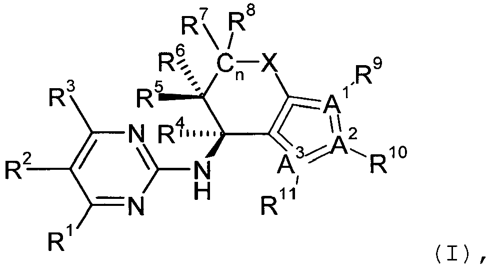 in which the radicals R¹ to R¹⁴ and X are as defined in any of Claims 1 to 14, and where
- a compound of the general formula (II) 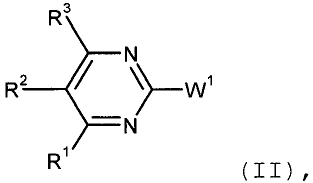
in which R¹ to R³ are as defined in any of Claims 1 to 14 and W¹ represents an exchangeable radical or a leaving group
is reacted with an amine of the general formula (III) or with an acid addition salt of the amine of the general formula (III) 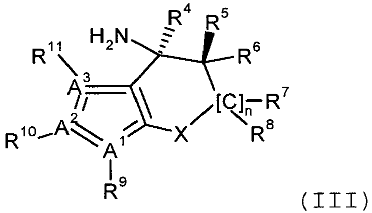
where the radicals R⁴ to R¹¹, A¹ bis A³, n and X are as defined in any of claims 1 to 14.

19. Process according to Claim 18, wherein the exchangeable radical or the leaving group Z¹ represents fluorine, chlorine, bromine, iodine, (C₁-C₄)-alkylsulfanyl or (C₁-C₄)-alkylsulfinyl or (C₁-C₄)-alkylsulfonyl, unsubstituted or substituted phenyl- (C₁-C₄) -alkylsulfonyl or (C₁-C₄)-alkylphenylsulfonyl.

20. Process according to Claim 18, where a compound of the general formula II-a 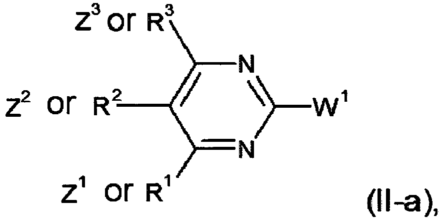
in which Z¹ to Z³ represent COOH, COO(C₁-C₆)-alkyl, nitrile, C₂-C₆-alkynyl, halogen, acetyl, carbonyl and (C₁-C₆)-alkylmercapto and W¹ represents an exchangeable radical or a leaving group according to Claim 19,
is reacted witn an amine or an acid addition salt of the general formula (III) 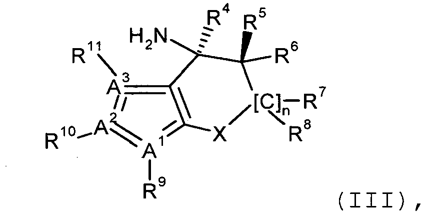
giving initially an intermediate of the formula (I-a) 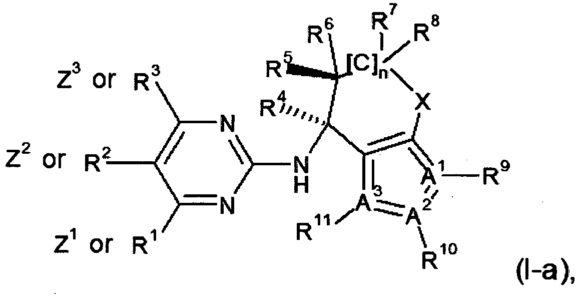 and the intermediate of the formula (I-a) obtained is then converted by known processes into the compound (I).

21. Process for preparing compounds of the general formula (I) and/or agrochemically acceptable salts thereof and/or agrochemically acceptable quaternized nitrogen derivatives thereof 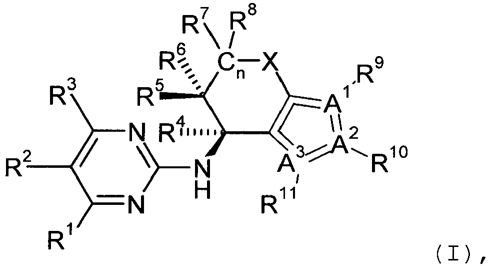
in which the R¹ to R¹⁴ and X radicals are as defined in any of Claims 1 to 16, and wherein
a compound of the general formula (IV) or an acid addition salt thereof 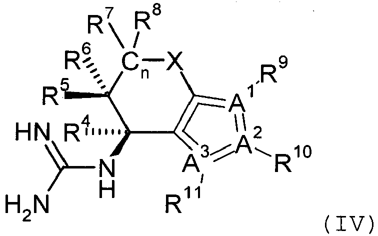
is condensed with a compound of the general formula (V) 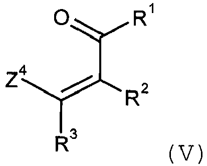 in which the radical Z⁴ represents (C₁-C₆) -alkoxy or di-(C₁-C₆)-alkylamino and R¹ to R³ are defined according to Claims 1 to 14.

22. Process according to Claim 21, where the compound of the formula (IV) or the acid addition salt thereof 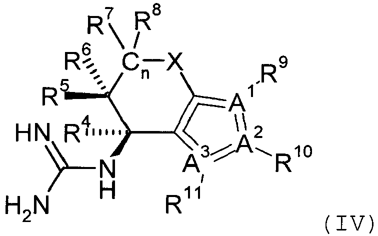 is condensed with a compound of the formula (VI) 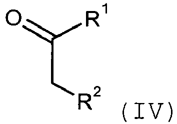 in which R¹ and R² are as defined in Claims 1 to 14, and a compound of the formula (VII) 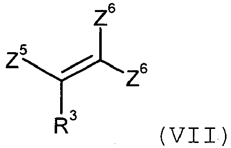 in which Z⁵ represents (C₁-C₆) -alkoxy or di-(C₁-C₆)-alkylamino and Z⁶ represents (C₁-C₆)-alkoxy and R³ is as defined in Claims 1 to 16.

23. Herbicidal composition or plant growth-regulating composition, **characterized in that** it comprises one or more compounds of the general formula (I) or salts thereof according to any of Claims 1 to 16.

24. Method of controlling harmful plants or of regulating the growth of plants, **characterized in that** an effective amount of one or more compounds of the general formula (I) or salts thereof according to any of Claims 1 to 16 is applied to plants, plant parts, plant seeds or an area under cultivation.

25. Use of compounds of the general formula (I) or salts thereof according to any of Claims 1 to 16 as herbicides or as plant growth regulators.

26. Use according to Claim 25, **characterized in that** the compounds of the general formula (I) or salts thereof are used to control harmful plants or to regulate the growth of plants in crops of useful plants or ornamental plants.

27. Use according to Claim 26, **characterized in that** the crop plants are transgenic crop plants.

## Revendications

1. Composés de formule générale (I) 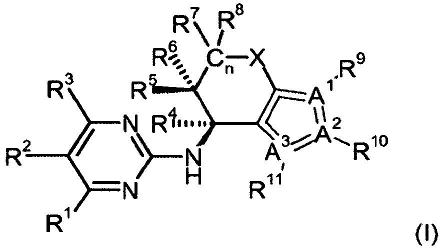 et leurs sels agrochimiquement compatibles, dans laquelle
A¹, A² et A³ sont chacun choisis indépendamment les uns des autres parmi O, S, CR⁹, CR¹⁰ ou CR¹¹, exactement un atome parmi A¹, A² et A³ représentant O ou S ;
R⁹, R¹⁰ et R¹¹ sont chacun choisis indépendamment les uns des autres dans le groupe constitué par hydrogène, halogène, cyano, C(O)OH, C(O)NH₂, alkyle en (C₁-C₆), alkylcarbonyle en (C₁-C₆), alkyloxycarbonyle en (C₁-C₆), alkylaminocarbonyle en (C₁-C₆), di-alkylaminocarbonyle en (C₁-C₆), haloalkyle en (C₁-C₆), alcoxy en (C₁-C₆), halogénoalcoxy en (C₁-C₆), alcényle en (C₂-C₆), alcynyle en (C₂-C₆), halogénoalcynyle en (C₂-C₆), alcynylcarbonyle en (C₂-C₆), haloalcynylcarbonyle en (C₂-C₆), alcynyloxy en (C₂-C₆), haloalcynyloxy en (C₂-C₆), alcynyloxycarbonyle en (C₂-C₆), halogénoalcynyloxycarbonyle en (C₂-C₆) et nitro ;
R¹ et R² sont chacun choisis indépendamment l'un de l'autre dans le groupe constitué par :
- halogène, hydroxy, nitro, amino, cyano, C(O)NH₂ ;
- alkyle en (C₁-C₆), halogénoalkyle en (C₁-C₆), alkylcarbonyle en (C₁-C₆), haloalkylcarbonyle en (C₁-C₆), alkylcarbonyloxy en (C₁-C₆), halogénoalkylcarbonyloxy en (C₁-C₆), alkylcarbonyle en (C₁-C₆) -alkyle en (C₁-C₄) ;
- alcoxy en (C₁-C₆), halogénoalcoxy en (C₁-C₆), alcoxycarbonyle en (C₁-C₆), haloalcoxycarbonyle en (C₁-C₆), alcoxycarbonyle en (C₁-C₆) -alkyle en (C₁-C₆), haloalcoxycarbonyle en (C₁-C₆)-alkyle en (C₁-C₆), alcoxycarbonyle en (C₁-C₆)-halogénoalkyle en (C₁-C₆), halogénoalcoxycarbonyle en (C₁-C₆)-halogénoalkyle en (C₁-C₆) ;
- alcényle en (C₂-C₆), halogénoalcényle en (C₂-C₆), alcénylcarbonyle en (C₂-C₆), haloalcénylcarbonyle en (C₂-C₆), alcényloxy en (C₂-C₆), haloalcényloxy en (C₂-C₆), alcényloxycarbonyle en (C₂-C₆), haloalcényloxycarbonyle en (C₂-C₆) ;
- alcynyle en (C₂-C₆), halogénoalcynyle en (C₂-C₆), alcynylcarbonyle en (C₂-C₆), haloalcynylcarbonyle en (C₂-C₆), alcynyloxy en (C₂-C₆), haloalcynyloxy en (C₂-C₆), alcynyloxycarbonyle en (C₂-C₆), halogénoalcynyloxycarbonyle en (C₂-C₆) ;
- tri-alkylsilyle en (C₁-C₆) -alcynyle en (C₂-C₆), di-alkylsilyle en (C₁-C₆)-alcynyle en (C₂-C₆), mono-alkylsilyle en (C₁-C₆)-alcynyle en (C₂-C₆) ; phénylsilyl-alcynyle en (C₂-C₆) ;
- aryle en (C₆-C₁₄), aryloxy en (C₆-C₁₄), arylcarbonyle en (C₆-C₁₄) et aryloxycarbonyle en (C₆-C₁₄), qui peuvent chacun être substitués dans la partie aryle avec halogène, alkyle en (C₁-C₆) et/ou haloalkyle en (C₁-C₆) ;
- aryle en (C₆-C₁₄)-alkyle en (C₁-C₆), aryle en (C₆-C₁₄)-alcoxy en (C₁-C₆), aryle en (C₆-C₁₄)-alkylcarbonyle en (C₁-C₆), aryle en (C₆-C₁₄)-alkylcarbonyloxy en (C₁-C₆), aryle en (C₆-C₁₄)-alcoxycarbonyle en (C₁-C₆), aryle en (C₆-C₁₄)-alcoxycarbonyloxy en (C₁-C₆) ;
- aminocarbonyle-alkyle en (C₁-C₆), di-alkylaminocarbonyle en (C₁-C₆)-alkyle en (C₁-C₆) ;
- N-(haloalcanoyle en (C₁-C₆))-aminocarbonyle, mono-(aryle en (C₆-C₁₄))-aminocarbonyle, di-(aryle en (C₆-C₁₄))aminocarbonyle ;
- alcoxy en (C₁-C₆)-alkyle en (C₁-C₆), alcoxy en (C₁-C₆)-alcoxy en (C₁-C₆), alcoxycarbonyle en (C₁-C₆)-alcoxy en (C₁-C₆) ;
- cycloalkyle en (C₃-C₈), qui peut éventuellement être substitué sur le radical cycloalkyle par alkyle en (C₁-C₆) et/ou halogène ; cycloalcoxy en (C₃-C₈), cycloalkyle en (C₃-C₈)-alkyle en (C₁-C₆), cycloalkyle en (C₃-C₈)-haloalkyle en (C₁-C₆), cycloalkyle en (C₃-C₈)-alcoxy en (C₁-C₆), cycloalkyle en (C₃-C₈)-haloalcoxy en (C₁-C₆), cycloalkylcarbonyle en (C₃-C₈), cycloalcoxycarbonyle en (C₃-C₈), cycloalkyle en (C₃-C₈)-alkylcarbonyle en (C₁-C₆), cycloalkyle en (C₃-C₈)-haloalkylcarbonyle en (C₁-C₆), cycloalkyle en (C₃-C₈)-alcoxycarbonyle en (C₁-C₆), cycloalkyle en (C₃-C₈)-haloalcoxycarbonyle en (C₁-C₆), cycloalkylcarbonyloxy en (C₃-C₈), cycloalcoxycarbonyloxy en (C₃-C₈), cycloalkyle en (C₃-C₈)-alkylcarbonyloxy en (C₁-C₆), cycloalkyle en (C₃-C₈)-halogénoalkylcarbonyloxy en (C₁-C₆), cycloalkyle en (C₃-C₈)-alcoxycarbonyloxy en (C₁-C₆), cycloalkyle en (C₃-C₈)-haloalcoxycarbonyloxy en (C₁-C₆) ;
- cycloalcényle en (C₃-C₈), cycloalcényloxy en (C₃-C₈), cycloalcényle en (C₃-C₈)-alkyle en (C₁-C₆), cycloalcényle en (C₃-C₈)-halogénoalkyle en (C₁-C₆), cycloalcényle en (C₃-C₈)-alcoxy en (C₁-C₆), cycloalcényle en (C₃-C₈)-halogénoalcoxy en (C₁-C₆), cycloalcénylcarbonyle en (C₃-C₈), cycloalcényloxycarbonyle en (C₃-C₈), cycloalcényle en (C₃-C₈)-alkylcarbonyle en (C₁-C₆), cycloalcényle en (C₃-C₈)-halogénoalkylcarbonyle en (C₁-C₆), cycloalcényle en (C₃-C₈)-alcoxycarbonyle en (C₁-C₆), cycloalcényle en (C₃-C₈)-haloalcoxycarbonyle en (C₁-C₆), cycloalcénylcarbonyloxy en (C₃-C₈), cycloalcényloxycarbonyloxy en (C₃-C₈), cycloalcényle en (C₃-C₈)-alkylcarbonyloxy en (C₁-C₆), cycloalcényle en (C₃-C₈)-halogénoalkylcarbonyloxy en (C₁-C₆), cycloalcényle en (C₃-C₈)-alcoxycarbonyloxy en (C₁-C₆), cycloalcényle en (C₃-C₈)-haloalcoxycarbonyloxy en (C₁-C₆) ;
- hydroxy-alkyle en (C₁-C₆), hydroxy-alcoxy en (C₁-C₆), cyano-alcoxy en (C₁-C₆), cyano-alkyle en (C₁-C₆) ; et
- alkylsulfonyle en (C₁-C₆), alkylthio en (C₁-C₆), alkylsulfinyle en (C₁-C₆), haloalkylsulfonyle en (C₁-C₆), halogénoalkylthio en (C₁-C₆), halogénoalkylsulfinyle en (C₁-C₆), alkylsulfonyle en (C₁-C₆)-alkyle en (C₁-C₆), alkylthio en (C₁-C₆)-alkyle en (C₁-C₆), alkylsulfinyle en (C₁-C₆)-alkyle en (C₁-C₆), haloalkylsulfonyle en (C₁-C₆)-alkyle en (C₁-C₆), haloalkylthio en (C₁-C₆)-alkyle en (C₁-C₆), haloalkylsulfinyle en (C₁-C₆)-alkyle en (C₁-C₆), alkylsulfonyle en (C₁-C₆)-haloalkyle en (C₁-C₆), alkylthio en (C₁-C₆)-haloalkyle en (C₁-C₆), alkylsulfinyle en (C₁-C₆)-haloalkyle en (C₁-C₆), haloalkylsulfonyle en (C₁-C₆)-haloalkyle en (C₁-C₆), haloalkylthio en (C₁-C₆)-haloalkyle en (C₁-C₆), haloalkylsulfinyle en (C₁-C₆)-haloalkyle en (C₁-C₆), alkylsulfonyloxy en (C₁-C₆), halogénoalkylsulfonyloxy en (C₁-C₆), alkylthiocarbonyle en (C₁-C₆), haloalkylthiocarbonyle en (C₁-C₆), alkylthiocarbonyloxy en (C₁-C₆), haloalkylthiocarbonyloxy en (C₁-C₆), alkylthio en (C₁-C₆)-alkyle en (C₁-C₆), alkylthio en (C₁-C₆)-alcoxy en (C₁-C₆), alkylthio en (C₁-C₆)-alkylcarbonyle en (C₁-C₆), alkylthio en (C₁-C₆)-alkylcarbonyloxy en (C₁-C₆); arylsulfonyle en (C₄-C₁₄), arylthio en (C₆-C₁₄), arylsulfinyle en (C₆-C₁₄), cycloalkylthio en (C₃-C₈), alcénylthio en (C₃-C₈), cycloalcénylthio en (C₃-C₈) et alcynylthio en (C₃-C₆) ; R³ est choisi dans le groupe constitué par :
- hydrogène, halogène, hydroxy, nitro, amino, cyano, C(O)OH, C(O)NH₂ ;
- alkyle en (C₁-C₆), halogénoalkyle en (C₁-C₆), alkylcarbonyle en (C₁-C₆), haloalkylcarbonyle en (C₁-C₆), alkylcarbonyloxy en (C₁-C₆), halogénoalkylcarbonyloxy en (C₁-C₆), alkylcarbonyle en (C₁-C₆)-alkyle en (C₁-C₄) ;
- alcoxy en (C₁-C₆), halogénoalcoxy en (C₁-C₆), alcoxycarbonyle en (C₁-C₆), haloalcoxycarbonyle en (C₁-C₆), alcoxycarbonyle en (C₁-C₆)-alkyle en (C₁-C₆), haloalcoxycarbonyle en (C₁-C₆)-alkyle en (C₁-C₆), alcoxycarbonyle en (C₁-C₆)-halogénoalkyle en (C₁-C₆), halogénoalcoxycarbonyle en (C₁-C₆)-halogénoalkyle en (C₁-C₆);
- alcényle en (C₂-C₆), halogénoalcényle en (C₂-C₆), alcénylcarbonyle en (C₂-C₆), haloalcénylcarbonyle en (C₂-C₆), alcényloxy en (C₂-C₆), haloalcényloxy en (C₂-C₆), alcényloxycarbonyle en (C₂-C₆), haloalcényloxycarbonyle en (C₂-C₆);
- alcynyle en (C₂-C₆), halogénoalcynyle en (C₂-C₆), alcynylcarbonyle en (C₂-C₆), haloalcynylcarbonyle en (C₂-C₆), alcynyloxy en (C₂-C₆), haloalcynyloxy en (C₂-C₆), alcynyloxycarbonyle en (C₂-C₆), halogénoalcynyloxycarbonyle en (C₂-C₆) ;
- tri-alkylsilyle en (C₁-C₆)-alcynyle en (C₂-C₆), di-alkylsilyle en (C₁-C₆)-alcynyle en (C₂-C₆), mono-alkylsilyle en (C₁-C₆) -alcynyle en (C₂-C₆) ; phénylsilyle-alcynyle en (C₂-C₆) ;
- aryle en (C₆-C₁₄), aryloxy en (C₆-C₁₄), arylcarbonyle en (C₆-C₁₄) et aryloxycarbonyle en (C₆-C₁₄), qui peuvent chacun être substitués dans la partie aryle avec halogène, alkyle en (C₁-C₆) et/ou haloalkyle en (C₁-C₆) ;
- aryle en (C₆-C₁₄)-alkyle en (C₁-C₆), aryle en (C₆-C₁₄)-alcoxy en (C₁-C₆), aryle en (C₆-C₁₄)-alkylcarbonyle en (C₁-C₆), aryle en (C₆-C₁₄)-alkylcarbonyloxy en (C₁-C₆), aryle en (C₆-C₁₄)-alcoxycarbonyle en (C₁-C₆), aryle en (C₆-C₁₄)-alcoxycarbonyloxy en (C₁-C₆) ;
- aminocarbonyle-alkyle en (C₁-C₆), di-alkylaminocarbonyle en (C₁-C₆)-alkyle en (C₁-C₆) ;
- N-(haloalcanoyle en (C₁-C₆))-aminocarbonyle, mono-(aryle en (C₆-C₁₄))-aminocarbonyle, di-(aryle en (C₆-C₁₄))aminocarbonyle ;
- alcoxy en (C₁-C₆)-alkyle en (C₁-C₆), alcoxy en (C₁-C₆)-alcoxy en (C₁-C₆), alcoxycarbonyle en (C₁-C₆)-alcoxy en (C₁-C₆);
- cycloalkyle en (C₃-C₈), qui peut éventuellement être substitué sur le radical cycloalkyle par alkyle en (C₁-C₆) et/ou halogène ; cycloalcoxy en (C₃-C₈), cycloalkyle en (C₃-C₈)-alkyle en (C₁-C₆), cycloalkyle en (C₃-C₈)-haloalkyle en (C₁-C₆), cycloalkyle en (C₃-C₈)-alcoxy en (C₁-C₆), cycloalkyle en (C₃-C₈)-haloalcoxy en (C₁-C₆), cycloalkylcarbonyle en (C₃-C₈), cycloalcoxycarbonyle en (C₃-C₈), cycloalkyle en (C₃-C₈)-alkylcarbonyle en (C₁-C₆), cycloalkyle en (C₃-C₈)-haloalkylcarbonyle en (C₁-C₆), cycloalkyle en (C₃-C₈)-alcoxycarbonyle en (C₁-C₆), cycloalkyle en (C₃-C₈)-haloalcoxycarbonyle en (C₁-C₆), cycloalkylcarbonyloxy en (C₃-C₈), cycloalcoxycarbonyloxy en (C₃-C₈), cycloalkyle en (C₃-C₈)-alkylcarbonyloxy en (C₁-C₆), cycloalkyle en (C₃-C₈)-halogénoalkylcarbonyloxy en (C₁-C₆), cycloalkyle en (C₃-C₈)-alcoxycarbonyloxy en (C₁-C₆), cycloalkyle en (C₃-C₈)-haloalcoxycarbonyloxy en (C₁-C₆) ;
- cycloalcényle en (C₃-C₈), cycloalcényloxy en (C₃-C₈), cycloalcényle en (C₃-C₈)-alkyle en (C₁-C₆), cycloalcényle en (C₃-C₈)-halogénoalkyle en (C₁-C₆), cycloalcényle en (C₃-C₈)-alcoxy en (C₁-C₆), cycloalcényle en (C₃-C₈)-halogénoalcoxy en (C₁-C₆), cycloalcénylcarbonyle en (C₃-C₈), cycloalcényloxycarbonyle en (C₃-C₈), cycloalcényle en (C₃-C₈)-alkylcarbonyle en (C₁-C₆), cycloalcényle en (C₃-C₈)-halogénoalkylcarbonyle en (C₁-C₆), cycloalcényle en (C₃-C₈)-alcoxycarbonyle en (C₁-C₆), cycloalcényle en (C₃-C₈)-haloalcoxycarbonyle en (C₁-C₆), cycloalcénylcarbonyloxy en (C₃-C₈), cycloalcényloxycarbonyloxy en (C₃-C₈), cycloalcényle en (C₃-C₈)-alkylcarbonyloxy en (C₁-C₆), cycloalcényle en (C₃-C₈)-halogénoalkylcarbonyloxy en (C₁-C₆), cycloalcényle en (C₃-C₈)-alcoxycarbonyloxy en (C₁-C₆), cycloalcényle en (C₃-C₈)-haloalcoxycarbonyloxy en (C₁-C₆) ;
- hydroxy-alkyle en (C₁-C₆), hydroxy-alcoxy en (C₁-C₆), cyano-alcoxy en (C₁-C₆), cyano-alkyle en (C₁-C₆) ; et
- alkylsulfonyle en (C₁-C₆), alkylthio en (C₁-C₆), alkylsulfinyle en (C₁-C₆), haloalkylsulfonyle en (C₁-C₆), halogénoalkylthio en (C₁-C₆), halogénoalkylsulfinyle en (C₁-C₆), alkylsulfonyle en (C₁-C₆)-alkyle en (C₁-C₆), alkylthio en (C₁-C₆)-alkyle en (C₁-C₆), alkylsulfinyle en (C₁-C₆)-alkyle en (C₁-C₆), haloalkylsulfonyle en (C₁-C₆)-alkyle en (C₁-C₆), haloalkylthio en (C₁-C₆)-alkyle en (C₁-C₆), haloalkylsulfinyle en (C₁-C₆)-alkyle en (C₁-C₆), alkylsulfonyle en (C₁-C₆)-haloalkyle en (C₁-C₆), alkylthio en (C₁-C₆)-haloalkyle en (C₁-C₆), alkylsulfinyle en (C₁-C₆)-haloalkyle en (C₁-C₆), haloalkylsulfonyle en (C₁-C₆)-haloalkyle en (C₁-C₆), haloalkylthio en (C₁-C₆)-haloalkyle en (C₁-C₆), haloalkylsulfinyle en (C₁-C₆)-haloalkyle en (C₁-C₆), alkylsulfonyloxy en (C₁-C₆), halogénoalkylsulfonyloxy en (C₁-C₆), alkylthiocarbonyle en (C₁-C₆), haloalkylthiocarbonyle en (C₁-C₆), alkylthiocarbonyloxy en (C₁-C₆), haloalkylthiocarbonyloxy en (C₁-C₆), alkylthio en (C₁-C₆)-alkyle en (C₁-C₆), alkylthio en (C₁-C₆)-alcoxy en (C₁-C₆), alkylthio en (C₁-C₆)-alkylcarbonyle en (C₁-C₆), alkylthio en (C₁-C₆)-alkylcarbonyloxy en (C₁-C₆) ; arylsulfonyle en (C₄-C₁₄), arylthio en (C₆-C₁₄), arylsulfinyle en (C₆-C₁₄), cycloalkylthio en (C₃-C₈), alcénylthio en (C₃-C₈), cycloalcénylthio en (C₃-C₈) et alcynylthio en (C₃-C₆), ou R¹ et R² peuvent être reliés l'un avec l'autre par une liaison, de telle sorte qu'ils forment un carbocycle ou hétérocycle partiellement hydrogéné de 5 à 7 chaînons contenant au moins un hétéroatome choisi parmi N, O, S et P, qui est éventuellement substitué avec un ou plusieurs substituants choisis dans le groupe constitué par hydroxy, =O, =N-O-H, =N-O-alkyle en (C₁-C₆), =N-O-benzyle, =N-O-phényle, phényle, phényle substitué par un ou plusieurs atomes d'halogène identiques ou différents, alkyle en (C₁-C₆), cycloalkyle en (C₃-C₆) et haloalkyle en (C₁-C₆), et
R³ est tel que défini précédemment, mais représente toutefois de préférence hydrogène, méthyle ou amino ;
R⁴ est choisi dans le groupe constitué par hydrogène, alkyle en (C₁-C₆), alcoxy en (C₁-C₆), alcoxy en (C₁-C₆)-alkyle en (C₁-C₆), alcoxycarbonyle en (C₁-C₆) et aminocarbonyle ;
R⁵ et R⁶ sont chacun choisis indépendamment l'un de l'autre dans le groupe constitué par hydrogène, hydroxy, alkyle en (C₁-C₆), alkylphényle en (C₁-C₆), haloalkyle en (C₁-C₆), alcoxy en (C₁-C₆) et halogénoalcoxy en (C₁-C₆) ; ou les radicaux R⁵ et R⁶ forment ensemble avec l'atome de carbone auquel ils sont reliés un cycle de 3 à 7 chaînons ;
R⁷ et R⁸ sont chacun choisis indépendamment l'un de l'autre dans le groupe constitué par hydrogène, alkyle en (C₁-C₆), haloalkyle en (C₁-C₆), alcoxy en (C₁-C₆), halogénoalcoxy en (C₁-C₆), aryle en (C₆-C₁₄), aryloxy en (C₆-C₁₄), arylcarbonyle en (C₆-C₁₄) et aryloxycarbonyle en (C₆-C₁₄); ou les radicaux R⁷ et R⁸ forment ensemble un groupe alkylène en (C₁-C₇), qui peut contenir un ou plusieurs atomes d'oxygène et/ou de soufre, le groupe alkylène en (C₁-C₇) pouvant être substitué une fois ou plusieurs fois par halogène, et les substituants halogène respectifs pouvant être identiques ou différents ;
X représente une liaison (si n = 1 ou 2), ou est choisi dans le groupe constitué par O, S, CH₂, C=O, NH, CR¹²R¹³ et NR¹⁴, CH₂O et CH₂S ; dans les deux derniers groupes cités, l'atome de carbone étant relié à la partie aromatique et l'hétéroatome O ou S à la partie partiellement hydrogénée de l'amine ;
R¹² et R¹³ sont chacun choisis indépendamment l'un de l'autre dans le groupe constitué par hydrogène, alkyle en (C₁-C₆) et haloalkyle en (C₁-C₆) ;
R¹⁴ est choisi dans le groupe constitué par hydrogène, alkyle en (C₁-C₆) et haloalkyle en (C₁-C₆) ; et
n représente le nombre 0, 1 ou 2.

2. Composés de formule générale (I) selon la revendication 1, **caractérisés en ce qu'**A¹, A² et A³ sont chacun choisis indépendamment les uns des autres parmi S, CR⁹, CR¹⁰ ou CR¹¹, exactement un atome parmi A¹, A² et A³ représentant S.

3. Composés de formule générale (I) selon la revendication 1 ou 2, **caractérisés en ce que** les radicaux R⁹, R¹⁰ et R¹¹ sont chacun choisis indépendamment les uns des autres dans le groupe constitué par hydrogène, halogène et alkyle en (C₁-C₆).

4. Composés de formule générale (I) selon l'une quelconque des revendications 1 à 3, **caractérisés en ce que**
A¹, A² et A³ sont chacun choisis indépendamment les uns des autres parmi S, CR⁹, CR¹⁰ ou CR¹¹, exactement un atome parmi A¹, A² et A³ représentant S, et
R⁹, R¹⁰ et R¹¹ sont chacun choisis indépendamment les uns des autres dans le groupe constitué par hydrogène, chlore et alkyle en (C₁-C₃).

5. Composés de formule générale (I) selon les revendications 1 à 4, **caractérisés en ce qu'**exactement un radical R⁹, R¹⁰ ou R¹¹ représente méthyle, et les autres radicaux représentent hydrogène.

6. Composés de formule générale (I) selon l'une quelconque des revendications 1 à 5, **caractérisés en ce que** les radicaux R¹ et R² sont chacun choisis indépendamment l'un de l'autre dans le groupe constitué par halogène, hydroxy, nitro, amino, cyano, alkyle en (C₁-C₆), halogénoalkyle en (C₁-C₆), alkylcarbonyle en (C₁-C₆), alkylsulfonyle en (C₁-C₆), alkylthio en (C₁-C₆), alkylsulfinyle en (C₁-C₆) et haloalkylsulfonyle en (C₁-C₆), et R³ est choisi dans le groupe constitué par hydrogène, halogène, hydroxy, nitro, amino, cyano, alkyle en (C₁-C₆), halogénoalkyle en (C₁-C₆), alkylcarbonyle en (C₁-C₆), alkylsulfonyle en (C₁-C₆), alkylthio en (C₁-C₆), alkylsulfinyle en (C₁-C₆) et haloalkylsulfonyle en (C₁-C₆).

7. Composés de formule générale (I) selon l'une quelconque des revendications 1 à 6, **caractérisés en ce que** les radicaux R¹, R² et R³ sont chacun différents les uns des autres, et R¹ = amino, R² = trifluorométhyle ou méthylsulfonyle, et R³ = hydrogène ou méthyle.

8. Composés de formule générale (I) selon l'une quelconque des revendications 1 à 7, **caractérisés en ce que** les radicaux R¹ et R² sont reliés l'un avec l'autre par une liaison, de telle sorte qu'ils forment un carbocycle ou hétérocycle partiellement hydrogéné à 5 ou 6 chaînons contenant au moins un hétéroatome choisi parmi N, O, S et P, qui est éventuellement substitué avec un ou plusieurs substituants choisis dans le groupe constitué par hydroxy, =O, =N-O-H, =N-O-alkyle en (C₁-C₆), =N-O-benzyle, =N-O-phényle, phényle, phényle substitué par un ou plusieurs halogènes identiques ou différents, alkyle en (C₁-C₆), cycloalkyle en (C₃-C₆) et haloalkyle en (C₁-C₆), et
R³ est choisi dans le groupe constitué par hydrogène, amino, méthyle et trifluorométhyle.

9. Composés de formule générale (I) selon l'une quelconque des revendications 1 à 8, **caractérisés en ce que** le radical R⁴ est choisi dans le groupe constitué par hydrogène, CH₃, CH₂CH₂OCH₃, COOCH₃ et CONH₂.

10. Composés de formule générale (I) selon l'une quelconque des revendications 1 à 9, **caractérisés en ce que** les radicaux R⁵ et R⁶ sont chacun choisis indépendamment l'un de l'autre dans le groupe constitué par hydrogène, hydroxy, alkyle en (C₁-C₆), alkylphényle en (C₁-C₆) et alcoxy en (C₁-C₆).

11. Composés de formule générale (I) selon l'une quelconque des revendications 1 à 10, **caractérisés en ce que** les radicaux R⁷ et R⁸ sont chacun choisis indépendamment l'un de l'autre dans le groupe constitué par hydrogène, alkyle en (C₁-C₆) et aryle en (C₆-C₁₄).

12. Composés de formule générale (I) selon l'une quelconque des revendications 1 à 11, **caractérisés en ce que** X représente une liaison (si n = 1 ou 2), ou est choisi dans le groupe constitué par O, S, CH₂, C=O, NH, CHCH₃, NCH₃, C(CH₃)₂, OCH₂ et SCH₂ ; dans les deux derniers groupes cités, l'atome de carbone étant relié à la partie aromatique et l'hétéroatome O ou S à la partie partiellement hydrogénée de l'amine.

13. Composés de formule générale (I) selon l'une quelconque des revendications 1 à 12, **caractérisés en ce que** le nombre n représente 1 ou 2.

14. Composés de formule générale (I) selon la revendication 1, **caractérisés en ce que**
R¹ et R² sont chacun choisis indépendamment l'un de l'autre dans le groupe constitué par amino, trifluorométhyle, alkyle en (C₁-C₃), alkylsulfonyle en (C₁-C₆) ;
R³ est choisi dans le groupe constitué par hydrogène, amino, trifluorométhyle, alkyle en (C₁-C₆), alkylsulfonyle en (C₁-C₆) ; ou
R¹ et R² sont reliés l'un avec l'autre par une liaison, de telle sorte qu'ils forment un carbocycle ou hétérocycle partiellement hydrogéné à 5 ou 6 chaînons contenant au moins un hétéroatome choisi parmi N, O, S et P, qui est éventuellement substitué avec un ou plusieurs substituants choisis dans le groupe constitué par hydroxy, =O, =N-O-H, =N-O-alkyle en (C₁-C₆), =N-O-benzyle, =N-O-phényle, phényle, phényle substitué par un ou plusieurs atomes d'halogène, alkyle en (C₁-C₆), cycloalkyle en (C₃-C₆) et haloalkyle en (C₁-C₆), et
R³ est choisi parmi hydrogène, méthyle, amino et trifluorométhyle ;
R⁴ est choisi dans le groupe constitué par hydrogène, CH₃, CH₂CH₂OCH₃, COOCH₃ et CONH₂ ;
R⁵ et R⁶ sont chacun choisis indépendamment l'un de l'autre dans le groupe constitué par hydrogène, alkyle en (C1-C₃) et alcoxy en (C₁-C₆) ;
R⁷ et R⁸ sont chacun choisis indépendamment l'un de l'autre dans le groupe constitué par hydrogène, alkyle en (C₁-C₆) et aryle en (C₆-C₁₄) ;
A¹, A² et A³ sont chacun choisis indépendamment les uns des autres parmi S, CR⁹, CR¹⁰, CR¹¹, exactement un atome parmi A¹, A² et A³ représentant S ;
R⁹, R¹⁰ et R¹¹ sont chacun choisis indépendamment les uns des autres dans le groupe constitué par hydrogène, halogène et alkyle en (C₁-C₆) ;
X représente une liaison (si n = 1 ou 2), ou est choisi dans le groupe constitué par O, S, CH₂, =O, NH, CHCH₃, NCH₃, C(CH₃)₂, OCH₂ et SCH₂ ; dans les deux derniers groupes cités, l'atome de carbone étant relié à la partie aromatique et l'hétéroatome O ou S à la partie partiellement hydrogénée de l'amine ; et
n représente le nombre 1 ou 2.

15. Composés de formule générale (I) 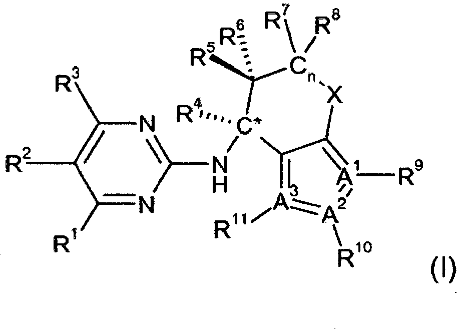 selon l'une quelconque des revendications 1 à 14, **caractérisés en ce que** l'atome de carbone chiral **caractérisé par** (*) présente une configuration (R).

16. Composés de formule générale (I) 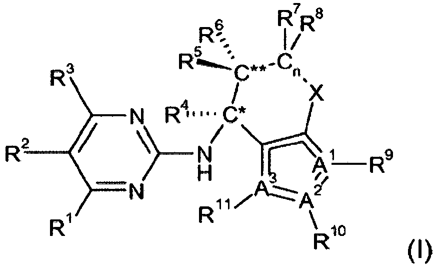 selon la revendication 15, **caractérisés en ce que** l'atome de carbone chiral **caractérisé par** (*) présente une configuration (R) et l'atome de carbone chiral **caractérisé par** (**) présente une configuration (S).

17. Utilisation de composés de formule générale (I) 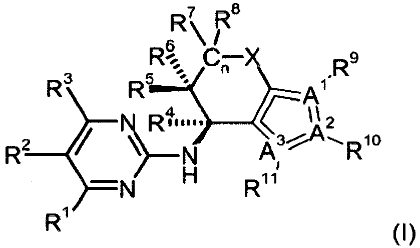 et leurs sels agrochimiquement compatibles, dans laquelle
A¹, A² et A³ sont chacun choisis indépendamment les uns des autres parmi O, S, CR⁹, CR¹⁰ ou CR¹¹, exactement un atome parmi A¹, A² et A³ représentant O ou S ;
R⁹, R¹⁰ et R¹¹ sont chacun choisis indépendamment les uns des autres dans le groupe constitué par hydrogène, halogène, cyano, C(O)OH, C(O)NH₂, alkyle en (C₁-C₆), alkylcarbonyle en (C₁-C₆), alkyloxycarbonyle en (C₁-C₆), alkylaminocarbonyle en (C₁-C₆), di-alkylaminocarbonyle en (C₁-C₆), haloalkyle en (C₁-C₆), alcoxy en (C₁-C₆), halogénoalcoxy en (C₁-C₆), alcényle en (C₂-C₆), alcynyle en (C₂-C₆), halogénoalcynyle en (C₂-C₆), alcynylcarbonyle en (C₂-C₆), haloalcynylcarbonyle en (C₂-C₆), alcynyloxy en (C₂-C₆), haloalcynyloxy en (C₂-C₆), alcynyloxycarbonyle en (C₂-C₆), halogénoalcynyloxycarbonyle en (C₂-C₆) et nitro ;
R¹, R² et R³ sont chacun choisis indépendamment les uns des autres dans le groupe constitué par :
- hydrogène, halogène, hydroxy, nitro, amino, cyano, C(O)OH, C(O)NH₂;
- alkyle en (C₁-C₆), halogénoalkyle en (C₁-C₆), alkylcarbonyle en (C₁-C₆), haloalkylcarbonyle en (C₁-C₆), alkylcarbonyloxy en (C₁-C₆), halogénoalkylcarbonyloxy en (C₁-C₆), alkylcarbonyle en (C₁-C₆)-alkyle en (C₁-C₄);
- alcoxy en (C₁-C₆), halogénoalcoxy en (C₁-C₆), alcoxycarbonyle en (C₁-C₆), haloalcoxycarbonyle en (C₁-C₆), alcoxycarbonyle en (C₁-C₆)-alkyle en (C₁-C₆), haloalcoxycarbonyle en (C₁-C₆)-alkyle en (C₁-C₆), alcoxycarbonyle en (C₁-C₆)-halogénoalkyle en (C₁-C₆), halogénoalcoxycarbonyle en (C₁-C₆)-halogénoalkyle en (C₁-C₆);
- alcényle en (C₂-C₆), halogénoalcényle en (C₂-C₆), alcénylcarbonyle en (C₂-C₆), haloalcénylcarbonyle en (C₂-C₆), alcényloxy en (C₂-C₆), haloalcényloxy en (C₂-C₆), alcényloxycarbonyle en (C₂-C₆), haloalcényloxycarbonyle en (C₂-C₆);
- alcynyle en (C₂-C₆), halogénoalcynyle en (C₂-C₆), alcynylcarbonyle en (C₂-C₆), haloalcynylcarbonyle en (C₂-C₆), alcynyloxy en (C₂-C₆), haloalcynyloxy en (C₂-C₆), alcynyloxycarbonyle en (C₂-C₆), halogénoalcynyloxycarbonyle en (C₂-C₆);
- tri-alkylsilyle en (C₁-C₆)-alcynyle en (C₂-C₆), di-alkylsilyle en (C₁-C₆)-alcynyle en (C₂-C₆), mono-alkylsilyle en (C₁-C₆)-alcynyle en (C₂-C₆); phénylsilyl-alcynyle en (C₂-C₆);
- aryle en (C₆-C₁₄), aryloxy en (C₆-C₁₄), arylcarbonyle en (C₆-C₁₄) et aryloxycarbonyle en (C₆-C₁₄), qui peuvent chacun être substitués dans la partie aryle avec halogène, alkyle en (C₁-C₆) et/ou haloalkyle en (C₁-C₆);
- aryle en (C₆-C₁₄)-alkyle en (C₁-C₆), aryle en (C₆-C₁₄)-alcoxy en (C₁-C₆), aryle en (C₆-C₁₄)-alkylcarbonyle en (C₁-C₆), aryle en (C₆-C₁₄)-alkylcarbonyloxy en (C₁-C₆), aryle en (C₆-C₁₄)-alcoxycarbonyle en (C₁-C₆), aryle en (C₆-C₁₄)-alcoxycarbonyloxy en (C₁-C₆);
- aminocarbonyle-alkyle en (C₁-C₆), di-alkylaminocarbonyle en (C₁-C₆)-alkyle en (C₁-C₆);
- N-(haloalcanoyle en (C₁-C₆))-aminocarbonyle, mono-(aryle en (C₆-C₁₄))-aminocarbonyle, di-(aryle en (C₆-C₁₄)) aminocarbonyle ;
- alcoxy en (C₁-C₆)-alkyle en (C₁-C₆), alcoxy en (C₁-C₆)-alcoxy en (C₁-C₆), alcoxycarbonyle en (C₁-C₆)-alcoxy en (C₁-C₆);
- cycloalkyle en (C₃-C₈) qui peut éventuellement être substitué sur le radical cycloalkyle par alkyle en (C₁-C₆)et/ou halogène ; cycloalcoxy en (C₃-C₈), cycloalkyle en (C₃-C₈)-alkyle en (C₁-C₆), cycloalkyle en (C₃-C₈)-haloalkyle en (C₁-C₆), cycloalkyle en (C₃-C₈)-alcoxy en (C₁-C₆), cycloalkyle en (C₃-C₈)-haloalcoxy en (C₁-C₆), cycloalkylcarbonyle en (C₃-C₈), cycloalcoxycarbonyle en (C₃-C₈), cycloalkyle en (C₃-C₈)-alkylcarbonyle en (C₁-C₆), cycloalkyle en (C₃-C₈)-haloalkylcarbonyle en (C₁-C₆), cycloalkyle en (C₃-C₈)-alcoxycarbonyle en (C₁-C₆), cycloalkyle en (C₃-C₈)-haloalcoxycarbonyle en (C₁-C₆), cycloalkylcarbonyloxy en (C₃-C₈), cycloalcoxycarbonyloxy en (C₃-C₈), cycloalkyle en (C₃-C₈)-alkylcarbonyloxy en (C₁-C₆), cycloalkyle en (C₃-C₈)-halogénoalkylcarbonyloxy en (C₁-C₆), cycloalkyle en (C₃-C₈)-alcoxycarbonyloxy en (C₁-C₆), cycloalkyle en (C₃-C₈)-haloalcoxycarbonyloxy en (C₁-C₆);
- cycloalcényle en (C₃-C₈), cycloalcényloxy en (C₃-C₈), cycloalcényle en (C₃-C₈)-alkyle en (C₁-C₆), cycloalcényle en (C₃-C₈)-halogénoalkyle en (C₁-C₆), cycloalcényle en (C₃-C₈)-alcoxy en (C₁-C₆), cycloalcényle en (C₃-C₈)-halogénoalcoxy en (C₁-C₆), cycloalcénylcarbonyle en (C₃-C₈), cycloalcényloxycarbonyle en (C₃-C₈), cycloalcényle en (C₃-C₈)-alkylcarbonyle en (C₁-C₆), cycloalcényle en (C₃-C₈)-halogénoalkylcarbonyle en (C₁-C₆), cycloalcényle en (C₃-C₈)-alcoxycarbonyle en (C₁-C₆), cycloalcényle en (C₃-C₈)-haloalcoxycarbonyle en (C₁-C₆), cycloalcénylcarbonyloxy en (C₃-C₈), cycloalcényloxycarbonyloxy en (C₃-C₈), cycloalcényle en (C₃-C₈)-alkylcarbonyloxy en (C₁-C₆), cycloalcényle en (C₃-C₈)-halogénoalkylcarbonyloxy en (C₁-C₆), cycloalcényle en (C₃-C₈)-alcoxycarbonyloxy en (C₁-C₆), cycloalcényle en (C₃-C₈)-haloalcoxycarbonyloxy en (C₁-C₆) ;
- hydroxy-alkyle en (C₁-C₆), hydroxy-alcoxy en (C₁-C₆), cyano-alcoxy en (C₁-C₆), cyano-alkyle en (C₁-C₆); et
- alkylsulfonyle en (C₁-C₆), alkylthio en (C₁-C₆), alkylsulfinyle en (C₁-C₆), haloalkylsulfonyle en (C₁-C₆), halogénoalkylthio en (C₁-C₆), halogénoalkylsulfinyle en (C₁-C₆), alkylsulfonyle en (C₁-C₆)-alkyle en (C₁-C₆), alkylthio en (C₁-C₆)-alkyle en (C₁-C₆), alkylsulfinyle en (C₁-C₆)-alkyle en (C₁-C₆), haloalkylsulfonyle en (C₁-C₆)-alkyle en (C₁-C₆), haloalkylthio en (C₁-C₆)-alkyle en (C₁-C₆), haloalkylsulfinyle en (C₁-C₆)-alkyle en (C₁-C₆), alkylsulfonyle en (C₁-C₆)-haloalkyle en (C₁-C₆), alkylthio en (C₁-C₆)-haloalkyle en (C₁-C₆), alkylsulfinyle en (C₁-C₆)-haloalkyle en (C₁-C₆), haloalkylsulfonyle en (C₁-C₆)-haloalkyle en (C₁-C₆), haloalkylthio en (C₁-C₆)-haloalkyle en (C₁-C₆), haloalkylsulfinyle en (C₁-C₆)-haloalkyle en (C₁-C₆), alkylsulfonyloxy en (C₁-C₆), halogénoalkylsulfonyloxy en (C₁-C₆), alkylthiocarbonyle en (C₁-C₆), haloalkylthiocarbonyle en (C₁-C₆), alkylthiocarbonyloxy en (C₁-C₆), haloalkylthiocarbonyloxy en (C₁-C₆), alkylthio en (C₁-C₆)-alkyle en (C₁-C₆), alkylthio en (C₁-C₆)-alcoxy en (C₁-C₆), alkylthio en (C₁-C₆)-alkylcarbonyle en (C₁-C₆), alkylthio en (C₁-C₆)-alkylcarbonyloxy en (C₁-C₆); arylsulfonyle en (C₄-C₁₄), arylthio en (C₆-C₁₄), arylsulfinyle en (C₆-C₁₄), cycloalkylthio en (C₃-C₈), alcénylthio en (C₃-C₈), cycloalcénylthio en (C₃-C₈) et alcynylthio en (C₃-C₆), ou R¹ et R² peuvent être reliés l'un avec l'autre par une liaison, de telle sorte qu'ils forment un carbocycle ou hétérocycle partiellement hydrogéné de 5 à 7 chaînons contenant au moins un hétéroatome choisi parmi N, O, S et P, qui est éventuellement substitué avec un ou plusieurs substituants choisis dans le groupe constitué par hydroxy, =O, =N-O-H, =N-O-alkyle en (C₁-C₆), =N-O-benzyle, =N-O-phényle, phényle, phényle substitué par un ou plusieurs atomes d'halogène identiques ou différents, alkyle en (C₁-C₆), cycloalkyle en (C₃-C₆) et haloalkyle en (C₁-C₆), et
R³ est tel que défini précédemment, mais représente toutefois de préférence hydrogène ou amino ;
R⁴ est choisi dans le groupe constitué par hydrogène, alkyle en (C₁-C₆), alcoxy en (C₁-C₆), alcoxy en (C₁-C₆)-alkyle en (C₁-C₆), alcoxycarbonyle en (C₁-C₆) et aminocarbonyle ;
R⁵ et R⁶ sont chacun choisis indépendamment l'un de l'autre dans le groupe constitué par hydrogène, hydroxy, alkyle en (C₁-C₆), alkylphényle en (C₁-C₆), haloalkyle en (C₁-C₆), alcoxy en (C₁-C₆) et halogénoalcoxy en (C₁-C₆); ou les radicaux R⁵ et R⁶ forment ensemble avec l'atome de carbone auquel ils sont reliés un cycle de 3 à 7 chaînons ;
R⁷ et R⁸ sont chacun choisis indépendamment l'un de l'autre dans le groupe constitué par hydrogène, alkyle en (C₁-C₆), haloalkyle en (C₁-C₆), alcoxy en (C₁-C₆), halogénoalcoxy en (C₁-C₆), aryle en (C₆-C₁₄), aryloxy en (C₆-C₁₄), arylcarbonyle en (C₆-C₁₄) et aryloxycarbonyle en (C₆-C₁₄); ou les radicaux R⁷ et R⁸ forment ensemble un groupe alkylène en (C₁-C₇), qui peut contenir un ou plusieurs atomes d'oxygène et/ou de soufre, le groupe alkylène en (C₁-C₇) pouvant être substitué une fois ou plusieurs fois par halogène, et les substituants halogène respectifs pouvant être identiques ou différents ;
X représente une liaison (si n = 1 ou 2), ou est choisi dans le groupe constitué par O, S, CH₂, C=O, NH, CR¹²R¹³ et NR¹⁴, CH₂O et CH₂S ; dans les deux derniers groupes cités, l'atome de carbone étant relié à la partie aromatique et l'hétéroatome O ou S à la partie partiellement hydrogénée de l'amine ;
R¹² et R¹³ sont chacun choisis indépendamment l'un de l'autre dans le groupe constitué par hydrogène, alkyle en (C₁-C₆) et haloalkyle en (C₁-C₆);
R¹⁴ est choisi dans le groupe constitué par hydrogène, alkyle en (C₁-C₆) et haloalkyle en (C₁-C₆); et
n représente le nombre 0, 1 ou 2.

18. Procédé de fabrication de composés de formule générale (I) et/ou leurs sels agrochimiquement compatibles et/ou leurs dérivés d'azote quaternisés agrochimiquement compatibles 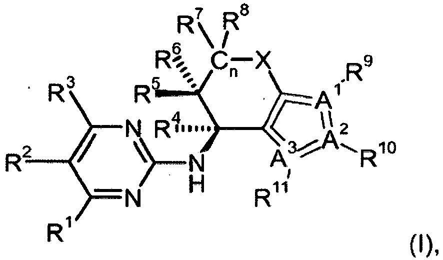 dans laquelle les radicaux R¹ à R¹⁴ et X sont tels que définis dans l'une quelconque des revendications 1 à 14, selon lequel
- un composé de formule générale (II) 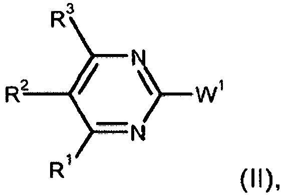
dans laquelle R¹ à R³ sont tels que définis dans l'une quelconque des revendications 1 à 14, et W¹ représente un radical échangeable ou un groupe partant,
est mis en réaction avec une amine de formule générale (III) ou avec un sel d'addition acide de l'aminé de formule générale (III) 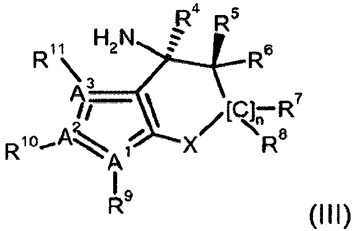
dans laquelle les radicaux R⁴ à R¹¹, A¹ à A³, n et X sont tels que définis dans l'une quelconque des revendications 1 à 14.

19. Procédé selon la revendication 18, dans lequel le radical échangeable ou le groupe partant Z¹ représente fluor, chlore, brome, iode, un alkylsulfanyle en (C₁-C₄) ou un alkylsulfinyle en (C₁-C₄) ou un alkylsulfonyle en (C₁-C₄), un phényl-alkylsulfonyle en (C₁-C₄) non substitué ou substitué, ou un alkylphényl-sulfonyle en (C₁-C₄).

20. Procédé selon la revendication 18, dans lequel un composé de formule générale (II-a) 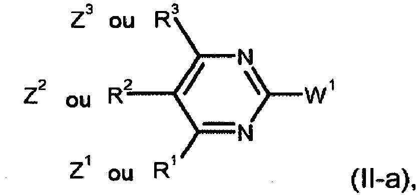
dans laquelle Z¹ à Z³ représentent COOH, COO-alkyle en (C₁-C₆), nitrile, alcynyle en C₂-C₆, halogène, acétyle, carbonyle et alkylmercapto en (C₁-C₆), et W¹ représente un radical échangeable ou un groupe partant selon la revendication 19,
est mis en réaction avec une amine ou un sel d'addition acide de formule générale (III) 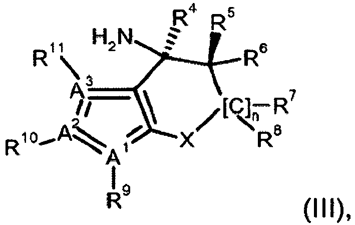
et un intermédiaire de formule (I-a) est tout d'abord obtenu 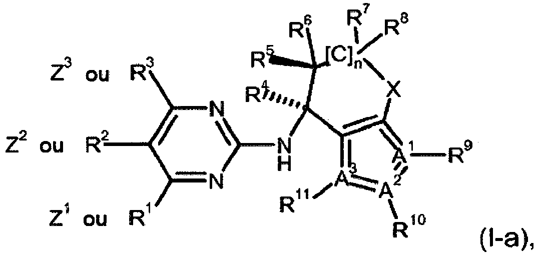 puis l'intermédiaire de formule (I-a) obtenu est transformé par des procédés connus en le composé (I).

21. Procédé de fabrication de composés de formule générale (I) et/ou leurs sels agrochimiquement compatibles et/ou leurs dérivés d'azote quaternisés agrochimiquement compatibles 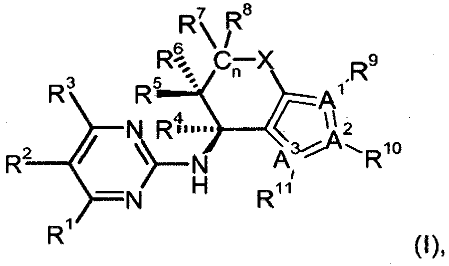
dans laquelle les radicaux R¹ à R¹⁴ et X sont tels que définis dans l'une quelconque des revendications 1 à 16, selon lequel
un composé de formule générale (IV) ou son sel d'addition acide 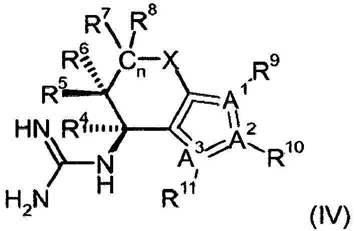
est condensé avec un composé de formule générale (V) 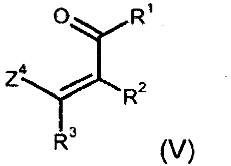 dans laquelle le radical Z⁴ représente un alcoxy en (C₁-C₆) ou di-alkylamino en (C₁-C₆), et R¹ à R³ sont définis selon les revendications 1 à 14.

22. Procédé selon la revendication 21, dans lequel le composé de formule (IV) ou son sel d'addition acide 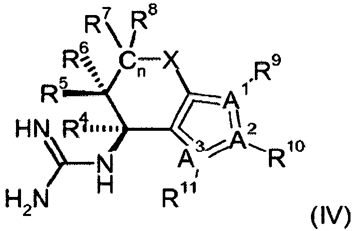 est condensé avec un composé de formule (VI) 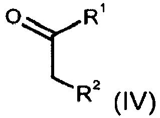
dans laquelle R¹ et R² sont tels que définis dans les revendications 1 à 14,
et un composé de formule (VII) 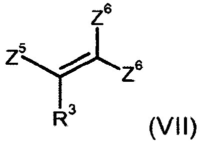
dans laquelle Z⁵ représente un alcoxy en (C₁-C₆) ou di-alkylamino en (C₁-C₆), et Z⁶ représente un alcoxy en (C₁-C₆), et R³ est tel que défini dans les revendications 1 à 16.

23. Agent herbicide ou agent de régulation de la croissance de plantes, **caractérisé en ce qu'**il contient un ou plusieurs composés de formule générale (I) ou leurs sels selon l'une quelconque des revendications 1 à 16.

24. Procédé de lutte contre des plantes nocives ou de régulation de la croissance de plantes, **caractérisé en ce qu'**une quantité efficace d'un ou de plusieurs composés de formule générale (I) ou leurs sels selon l'une quelconque des revendications 1 à 16 est appliquée sur des plantes, des parties de plantes, des graines de plantes ou sur une surface de culture.

25. Utilisation de composés de formule générale (I) ou leurs sels selon l'une quelconque des revendications 1 à 16 en tant qu'herbicides ou en tant que régulateurs de la croissance de plantes.

26. Utilisation selon la revendication 25, **caractérisée en ce que** les composés de formule générale (I) ou leurs sels sont utilisés pour lutter contre des plantes nocives ou pour réguler la croissance de plantes dans des cultures de plantes utiles ou ornementales.

27. Utilisation selon la revendication 26, **caractérisée en ce que** les plantes cultivées sont des plantes cultivées transgéniques.
